(19) Europäisches Patentamt

European Patent Office

Office européen des brevets

(11) **EP 3 762 333 B1**

(12) **EUROPEAN PATENT SPECIFICATION**

(45) Date of publication and mention
of the grant of the patent:
**01.03.2023 Bulletin 2023/09**

(21) Application number: **19730774.7**

(22) Date of filing: **14.06.2019**

(51) International Patent Classification (IPC):
***B82Y 5/00*** *(2011.01)* ***A61K 47/69*** *(2017.01)*

(52) Cooperative Patent Classification (CPC):
**A61K 47/6923; B82Y 5/00**

(86) International application number:
**PCT/EP2019/065653**

(87) International publication number:
**WO 2020/007590 (09.01.2020 Gazette 2020/02)**

(54) **COMPOSITE MATERIAL COMPRISING DNA HYDROGEL AND SILICA NANOPARTICLES**

VERBUNDMATERIAL MIT DNA-HYDROGEL UND SILICA-NANOPARTIKEL

MATÉRIAU COMPOSITE COMPRENANT DE L'HYDROGEL À BASE D'ADN ET DES NANOPARTICULES DE SILICE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **05.07.2018 EP 18181821**

(43) Date of publication of application:
**13.01.2021 Bulletin 2021/02**

(73) Proprietor: **Karlsruher Institut für Technologie**
**76131 Karlsruhe (DE)**

(72) Inventors:
• **NIEMEYER, Christof**
**28213 Bremen (DE)**
• **GRÖSCHE, Maximilian**
**79219 Staufen (DE)**
• **RABE, Kersten**
**76137 Karlsruhe (DE)**
• **LEIDNER, Arnold**
**67466 Lambrecht (DE)**
• **HU, Yong**
**76344 Eggenstein-Leopoldshafen (DE)**
• **BAUER, Jens**
**76889 Vorderweidenthal (DE)**

(74) Representative: **Müller-Boré & Partner**
**Patentanwälte PartG mbB**
**Friedenheimer Brücke 21**
**80639 München (DE)**

(56) References cited:
**US-A1- 2005 019 369**

• **BAOFEN YE ET AL: "Colorimetric logic response based on aptamer functionalized colloidal crystal hydrogels", NANOSCALE, vol. 7, no. 17, 1 January 2015 (2015-01-01), pages 7565-7568, XP055512439, United Kingdom ISSN: 2040-3364, DOI: 10.1039/C5NR00586H**
• **LI ZHOU ET AL: "Towards intelligent bioreactor systems: triggering the release and mixing of compounds based on DNA-functionalized hybrid hydrogel", CHEMICAL COMMUNICATIONS, vol. 50, no. 71, 1 January 2014 (2014-01-01), pages 10255-10257, XP055512295, ISSN: 1359-7345, DOI: 10.1039/C4CC04791E**
• **ANATOLY ZINCHENKO ET AL: "DNA-Assisted Solubilization of Carbon Nanotubes and Construction of DNA-MWCNT Cross-Linked Hybrid Hydrogels", NANOMATERIALS, vol. 5, no. 1, 3 March 2015 (2015-03-03), pages 270-283, XP055512187, DOI: 10.3390/nano5010270**
• **NIKHITA D. MANSUKHANI ET AL: "Optothermally Reversible Carbon Nanotube-DNA Supramolecular Hybrid Hydrogels", MACROMOLECULAR RAPID COMMUNICATIONS, vol. 39, no. 2, 24 October 2017 (2017-10-24), page 1700587, XP055512190, DE ISSN: 1022-1336, DOI: 10.1002/marc.201700587**

- **ARGHYA PAUL ET AL: "The attenuation of restenosis following arterial gene transfer using carbon nanotube coated stent incorporating TAT/DNAAng1+Vegf nanoparticles", BIOMATERIALS., vol. 33, no. 30, 1 October 2012 (2012-10-01), pages 7655-7664, XP055293796, GB ISSN: 0142-9612, DOI: 10.1016/j.biomaterials.2012.06.096**
- **ARNOLD LEIDNER ET AL: "Biopebbles: DNA-Functionalized Core-Shell Silica Nanospheres for Cellular Uptake and Cell Guidance Studies", ADVANCED FUNCTIONAL MATERIALS, vol. 28, no. 18, 1 May 2018 (2018-05-01), page 1707572, XP055511830, DE ISSN: 1616-301X, DOI: 10.1002/adfm.201707572**

Remarks:

The complete document including Reference Table(s) and the Sequence Listing(s) can be downloaded from the EPO website

**Description**

The present invention relates to a composite material comprising DNA hydrogel and silica nanoparticles, a hollow spherical container, a microreactor and microgel employing the composite material and uses thereof.

The design of novel materials to steer the interaction of eukaryotic cells with technical surfaces is of paramount interest for biomedical applications, such as cancer and stem cell therapies, tissue engineering or drug delivery. Synthetic hydrogels hold great promise as scaffolding materials in biosciences because their 3D porous structure reveals biocompatibility, high water content, and tissue-like elastic properties that allow for effective permeation of oxygen and nutrients, which is crucial for cellular colonization. Hydrogels with defined bulk and surface characteristics can be manufactured with controllable composition from various prepolymers and they are amenable to post synthetic modifications with molecular and colloidal compounds. DNA hydrogels are currently attracting much attention because the inherent properties of the genetic material allow for rational programming of structural and mechanical properties as well as of molecular recognition capabilities of the polymer backbone. This approach has led to an impressive collection of novel biocompatible materials that can display shape memory persistence, stimuli responsiveness and, by integration of genetic elements or proteins, even biochemical activity. However, there is a great demand for novel strategies to create DNA polymer materials with designed properties, such as adhesiveness, stiffness, plasticity, or tensile strength.

US 2005/019369 A1 describes polymers that are biocompatible and can be used for various medical purposes such as tissue repair, reconstruction and wound healing, as well as respective methods and compositions for producing the same.

Baofen Ye et al., Nanoscale, vol. 7, no. 17, 2015, pages 7565 to 7568, discloses a colorimetric logic response based on aptamer functionalized colloidal crystal hydrogels.

Li Zhou et al., Chemical Communications, vol. 50, no. 71, 2014, pages 10255 to 10257, discloses a mesoporous silica nanoparticle-DNA hydrogel bioreactor system. Anatoly Zinchenko et al., Nanomaterials, vol. 5, no. 1, 2015, pages 270 to 283, discloses DNA-assisted solubilization of carbon nanotubes and construction of DNA-MWCNT cross-linked hybrid hydrogels.

Nikhita D. Mansukhani et al., Macromolecular Rapid Communications, vol. 39, no. 2, 2017, page 1700587, discloses optothermally reversible carbon nanotube-DNA supramolecular hybrid hydrogels.

Arghya Paul et al., Biomaterials, vol. 33, no. 30, 2012, pages 7655 to 7664, discloses the attenuation of restenosis following arterial gene transfer using carbon nanotube coated stent incorporating TAT/DNA$_{Ang1+Vegf}$ nanoparticles.

Thus, the problem underlying the present invention is to provide a DNA polymer material with designed properties, such as adhesiveness, stiffness, plasticity, or tensile strength.

The solution to the above technical problem is achieved by the embodiments characterized in the claims.

(1) Composite material

The present invention relates to a composite material comprising DNA hydrogel, and silica nanoparticles, wherein the silica nanoparticles are connected with the DNA hydrogel through DNA strands. By incorporating silica nanoparticles into the DNA hydrogel, the mechanical stiffness and elastic properties can be improved.

In the present invention, the content of the silica nanoparticles based on the total amount of the composite material is from 10 to 80 wt%. Preferably, the lower limit of the content of the silica nanoparticles is more than 50 wt%.

Further, the content of the DNA hydrogel based on the total amount of the composite material is from 20 to 90 wt%.

By adjusting the contents of the silica nanoparticles and the DNA hydrogel to the above ranges, the composite material is mechanically stable while having a porous structure such that the composite material can be used for building three-dimensional structures which allow the diffusion of chemical and/or biological substances through the walls of said structures.

In the present invention, the pore size in the structure is more than 100 nm and less than 700 nm. The pore size can be measured by using two kinds of tracer particles (200 nm tracer particles and 500 nm tracer particles) in multiple particle tracking (MPT) microrheology (see below). Specifically, when the mesh size is estimated to be bigger than 100 nm and smaller than 500 nm, the 200 nm tracer particles freely move inside the hydrogel mesh structure, while 500 nm tracer particles are restrained inside the hydrogel mesh structure.

According to an embodiment, the composite material further comprises carbon nanotubes embedded in the DNA hydrogel. The carbon nanotubes can add an improved mechanical stiffness to the composite material such that a structure being more dimensionally stable can be achieved. The improvement in elastic modulus (storage modulus $G_0$, which is proportional to the number of entanglements in a polymer) by using both silica nanoparticles and carbon nanotubes in the DNA hydrogel is higher than expected from totalling the $G_0$ values of the binary composites silica nanoparticles-DNA hydrogel and carbon nanotubes-DNA hydrogel. Therefore, the ternary composite silica nanoparticles/carbon nanotubes-DNA hydrogel shows a synergistic effect in the mechanical properties, such as elasticity and stiffness.

The carbon nanotubes (CNT) can be mixed with the silica nanoparticles (SiNP) in different weight ratios in the ternary composite material. Preferably, the mass ratio of SiNP:CNT in the ternary composite material ranges from 100:1 to 1:1, preferably from 50:1 to 2.5:1. In an embodiment, the maximum concentration of SiNP in the binary SiNP-DNA hydrogel composite material and in the ternary SiNP/CNT-DNA hydrogel composite material is 20 mg/mL. According to a further embodiment, the maximum concentration of CNT in the ternary SiNP/CNT-DNA hydrogel composite material is 1000 μg/mL.

In an embodiment, the content of the carbon nanotubes based on the total amount of the composite material ranges from 1 to 10 wt%.

The composite material according to the present invention does not only provide mechanical stability while allowing diffusion of chemical and/or biological substances, but does also not show cytotoxicity such that it can be advantageously used for investigations of cells. These properties allow the use of the composite material in various fields of chemical, biological and biomedical applications. For example, the composite material according to the present invention may be advantageously used in cell biology, such as surface coatings for cell culture.

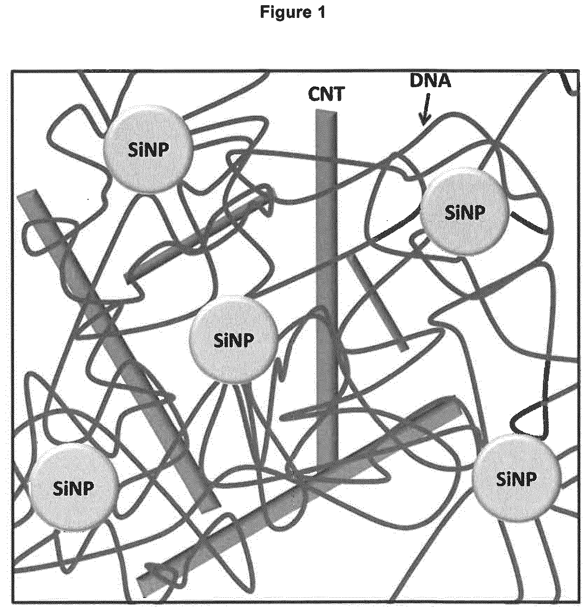

Figure 1 shows an example of the composite material of the present invention comprising DNA hydrogel, silica nanoparticles (SiNP) and carbon nanotubes (CNT).

The expression "embedded in the DNA hydrogel" used herein means that the respective embedded component is fixed in the surrounding DNA hydrogel.

In particular, in the composite material comprising DNA hydrogel, and silica nanoparticles, the DNA strand can be bonded to silica nanoparticles by at least one of its ends. Further, a DNA strand may also be bonded to a silica nanoparticle at other points along its strand. The DNA hydrogel is attached to the silica nanoparticles *via* a covalent bond.

In addition, in the embodiment further comprising carbon nanotubes, the DNA hydrogel contacts a carbon nanotube at least at one position along the outer wall of the carbon nanotube. The carbon nanotube and the DNA hydrogel are initially bound by hydrophobic π-π interaction. In the composite material, the CNT are interwoven with the DNA strands such that they are restrained inside the material.

In an embodiment, the composite material can further comprise at least one of the components selected from gold nanoparticles, metal oxide nanoparticles, such as quantum dots, agarose, alginate, polyacrylamide, polyethylene glycol, proteins (e.g. membrane proteins), peptides, graphene, DNA-protein conjugates, DNA-ligand conjugates and MOSAIC systems (i.e. nanoscaled DNA systems with proteins presented in defined spatial arrangements and stoichiometry).

According to an embodiment of the present invention, individual SiNP/CNT-DNA hydrogel composite materials may be encoded with a distinctive DNA sequence that is introduced to serve as identifier code which allows decoding or deconvolution to identify the material and the substances or compounds being located inside the material after performing cell adhesion or cellular uptake experiments.

The composite material of the present invention is an electrically conductive material. The conductivity of the composite material increases when it is in a dry (dehydrated) state. Further, increasing the amount of CNT in the composite material leads to an improved electrical conductivity. Electrical conductivity enables new applications in the fields of electronics, energy management, data processing and, in particular, electrochemical or electrobiochemical syntheses, and biochemical fuel cells. Since the SiNP/CNT-DNA composite material can be modified by biochemical reactions and is also biodegradable, potential applications arise in the fields of implantable biointerfaces and sensors as well as in the fabrication of biohybrid material systems that can be engineered and evolved under non-equilibrium, energy dissipative process conditions.

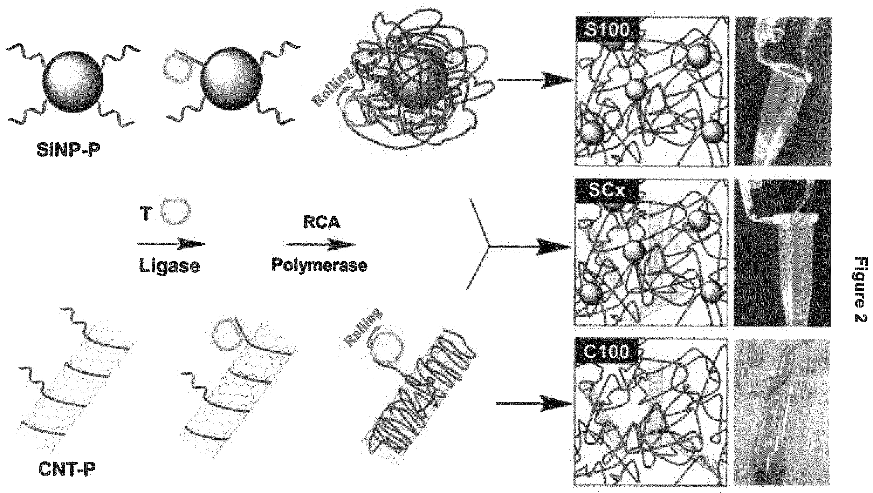

The method for producing the composite material according to the present invention is not particularly limited. For example, rolling circle amplification (RCA) or hybridization chain reaction (HCR) may be used. An embodiment of the manufacture of the composite material by using RCA is shown in Figure 2. According to an embodiment of the present invention, the composite material is produced by *in-situ* synthesis/polymerization of DNA polymers starting from primers bound to the SiNP and/or CNT using enzymatic elongation or hybridization.

Further, the present invention relates to the use of the composite material for cargo delivery to a cell. Cargo delivery includes delivery to the outer membrane of the cell, to the transmembrane compartment of the cell, and the cytosolic compartment of the cell. The cargo to be delivered is not particularly limited and can be selected from pharmacologically active compounds, drugs, metals, metal nanoparticles, metal chelates, proteins, peptides, and DNA molecules that encode genes for RNA and protein production. In an embodiment of the present invention, the composite material is used for drug delivery to a cell. Specifically, the DNA backbone may have an aptamer which specifically binds to a receptor of a cell and, in addition, a drug association site, which can be used for enrichment of intercalating drugs. When incubating a cell with the composite material and the drug which binds to the drug association site, the cell will uptake the composite material bearing the drug at the drug association site. This drug delivery to a cell may be tracked for example by using fluorescent composite material by employing e.g. silica nanoparticles having a fluorescence marker and/or chemically modified deoxynucleotides bearing fluorescent dyes, such as fluorescein (FITC)-modified dUTP.

The present invention further relates to the use of the composite material as surface coating for *in vitro* cell culture. According to an embodiment, the composite material is dried. In another embodiment, the dried composite material has a film form having a thickness of from 0.01 $\mu$m to 10 m. In a preferred embodiment, the film is a thin film having a thickness of from 0.1 $\mu$m to 1000 $\mu$m. In a more preferred embodiment, the film is a thin film having a thickness of from 1 $\mu$m to 100 $\mu$m. The drying process may be carried out for 2 to 6 h under vacuum at room temperature. Cells can be seeded on top of the composite material and cultured for 10 to 30 h. The composite material enables proliferation of cells higher than standard tissue culture (STC) surfaces. This effect can be emphasized by using the composite material comprising DNA hydrogel, silica nanoparticles and carbon nanotubes. The composite material holds the potential as tailorable coating for steering of cell adhesion, proliferation and motility, which is of substantial relevance for biomedical applications, such as medical implants.

According to an embodiment of the present invention, the composite material may be used as surface coating for *in vitro* cell culture of embryonic stem cells. The composite material of the present invention not only provides an advantageous proliferation of the cells, as mentioned above, but also enables the embryonic stem cells to maintain their pluripotency. In an embodiment, the embryonic stem cells can be grown in the shape of spheroids. The composite materials of the present invention can open the door to simpler and more robust stem cell culture techniques. Such methods are urgently needed for fundamental research in stem cell pluripotency and differentiation mechanisms as well as for applications in regenerative medicine and stem cell-based therapies. In this context, it should be noted that the stem cells are not obtained by destroying a human embryo.

In another embodiment, the composite material of the present invention may be used as surface coating for *in vitro* cultivation of bacteria. The bacteria that can be grown on the composite material are not particularly limited. Non-limiting examples of suitable prokaryotic cells include gram-negative or gram-positive bacteria (e.g. *E. coli, Burkholderia spec.* Shewanella, Geobacter, *Bacillus spec.*) and Archaea. Given that the composite material of the present invention is electrically conductive and can be designed as a growth matrix for the stabilization and standardization of bacterial biofilms, the composite material is particularly suitable for applications of electrochemically active bacteria as anodic or cathodic biocatalyst. Applications include the field of microbial electrosynthesis (also known as microbial bioelectrocatalysis, where electric energy is used to drive the bacterial metabolism to produce carbon compounds), bioelectrogenesis (the generation of electricity by bacteria) and microbial fuel cells (where chemical energy is converted to electrical energy). In a preferred embodiment, the bacteria are exoelectrogens like Shewanella oneidensis MR-1, or other strains where exoelectrogenic activity has been observed (e.g., Shewanella putrefaciens IR-1, Clostridium butyricum, Desulfuromonas acetoxidans, Geobacter metallireducens, Geobacter sulfurreducens, Rhodoferax ferrireducens, Aeromonas hydrophilia (A3), Pseudomonas aeruginosa, Desulfobulbus propionicus, Geopsychrobacter electrodiphilus, Geothrix fermentans, Shewanella oneidensis DSP10, Escherichia coli, Rhodopseudomonas palustris, Ochrobactrum anthropic YZ-1, Desulfovibrio desulfuricans, Acidiphilium sp.3.2Sup5, Klebsiella pneumonia L17, Thermincola sp.strain JR, Pichia anomala).

Further, the present invention relates to the use of the composite material for enzyme-triggered cell release. In an embodiment, the composite material has a film form having a thickness of from 0.01 $\mu$m to 10 m. In a preferred embodiment, the film is a thin film having a thickness of from 0.1 $\mu$m to 1000 $\mu$m. In a more preferred embodiment, the film is a thin film having a thickness of from 1 $\mu$m to 100 $\mu$m. For the use of the composite material for enzyme-triggered cell release, the composite material can be used directly after synthesis. Specifically, no drying step before application is required. Cells seeded on top of the composite material strongly adhere to the surface thereof. In particular, the cells are entrapped by said composite material in this embodiment. Treatment of the entrapped cells with a restriction enzyme according to an embodiment leads to a reduction of the thickness of the composite material along with the release of the adhered cells. Thus, the composite material allows the selective capture and enzyme-triggered release of surface-bound cells. This effect can be emphasized by using the composite material comprising DNA hydrogel, silica nanoparticles and carbon nanotubes.

According to another embodiment, the composite material of the present invention can be used for cell adhesion and transmigration of cells into the hydrogel layers, thereby the cells being protected from potentially harmful components in the cell medium.

In an embodiment of the present invention, the composite material may be used for flow-assisted capture and release of cells in a microchannel coated with the composite material. According to an embodiment, the composite material is coated onto the microchannel and dried. The coating of the microchannel with the composite material can be carried out by transfusing the composite material through the microchannel. In another embodiment, the dried composite material has a film form having a thickness of from 0.01 $\mu$m to 10 m. In a preferred embodiment, the film is a thin film having a thickness of from 0.1 $\mu$m to 1000 $\mu$m. In a more preferred embodiment, the film is a thin film having a thickness of from 1 $\mu$m to 100 $\mu$m. The drying process may be carried out for 2 to 12 h under normal pressure at room temperature. After coating the microchannel with the composite material, a cell suspension can be transfused through the microchannel. Then, the flow is stopped to allow the cells to settle and adhere to the composite material. Afterwards, the cells can be released by transfusing the microchannel with a medium containing a restriction enzyme as described above. The released cells can be collected and cultured.

According to another embodiment of the present invention, the composite material can be used for *in vitro* protein production. A gene fragment for expressing a protein can be introduced into the composite material during the production thereof. Following to synthesis, the gene-containing composite material can be used as template for *in vitro* transcription/translation by employment of conventional cell lysates obtained by various manufacturers using established methodologies.

The present invention is not limited to a particular type of cell. The cell used in the present invention is preferably a cell derived from a cell culture or a cell derived from a sample of an individual. The cell culture preferably comprises at least one type of eukaryotic cells or microorganisms. Non-limiting examples of cells are prokaryotic cells including gram-negative or gram-positive bacteria (e.g. *E. coli, Burkholderia spec., Bacillus spec.*) and Archaea as well as eukaryotic cells including yeast cells (such as *Saccharomyces*, e.g. *Saccharomyces cerevisiae*), insect cells (e.g. *Drosophila melanogaster*), plant cells and mammalian cells (e.g. HEK 293, HeLa). Furthermore isolates from natural or technical biofilm communities (e.g. from waste-treatment facilities, biogas producing plants, sewage plants, extreme habitats, sites of chemical spillage) or mixtures of different origin including combinations of a non-limited number of species are suitable for the present invention.

The term "individual" as used herein is not limited to any particular individual and may be a fungus, an animal, a human being or a plant.

The sample taken from an individual may be any sample. In one embodiment of the present invention, the sample may be derived from a naturally occurring system, preferably a sample containing a body fluid or components derived from a body fluid. In one embodiment of the present invention, the sample may be a naturally occurring system such as a solution selected from the group consisting of serum, saliva, urine, bile, lymph, tissue, like e.g. bladder or kidney, cerebrospinal fluid and/or other body fluids in the case of an animal or human individual.

In another embodiment, the sample may be derived from a naturally occurring system, preferably a sample containing a tissue or components derived from a tissue. A tissue or a component derived therefrom may be of various origins, like for example leafs, roots, petals, germs or stems in the case of plant individual, or muscle tissue, heart tissue, liver tissue, kidney tissue, epithelial tissue, connective tissue, or nervous tissue.

The term "cell culture" in its various grammatical forms, refers to cells grown in suspension, roller bottles, flasks and the like. Large scale approaches, such as bioreactors, including adherent cells growing attached to microcarriers in stirred fermenters, also are included. According to an embodiment of the present invention, cells derived from a cell culture may be derived from animals, plants or fungus. Animal cells may be derived for example from insects, fish, birds, or mammals. In a preferred embodiment, cells derived form a cell culture are mammalian cells including those of human origin, which may be primary cells derived from a tissue sample, diploid cell strains, transformed cells or established cell lines. Mammalian cells can include human and non-human cells alike. Mammalian cells of non-human origin can be monkey kidney cells, bovine kidney cells, dog kidney cells, pig kidney cells, rabbit kidney cells, mouse kidney cells, rat kidney cells, sheep kidney cells, hamster kidney cells, Chinese hamster ovarian cells or an animal cell derived from any tissue. In particular, mammalian cells can be BSC-1 cells, LLC-MK cells, CV-1 cells, COS-cells, COS-1 cells, COS-3 cells, COS-7 cells, VERO cells, MDBK cells, MDCK cells, CRFK cells, RAF cells, RK-cells, TCMK-1 cells, LLC-PK cells, PK15 cells, LLC-RK cells, MDOK cells, BHK-21 cells, CHO cells, NS-1 cells MRC-5 cells, WI-38 cells, BHK cells, 293 cells, HeLa, MCF7 and RK-cells.

The sample may be derived from a mammal, preferably a mammal selected from the group consisting of human, mouse, rat, pig, cat, dog, horse, goat, cattle, cow, and monkey and/or others. In a preferred embodiment of the present invention, the sample is derived from a human. In another embodiment of the present invention, the sample contains isolated body fluid compounds or processed body fluids derived from a mammal, preferably a mammal selected from the group consisting of human, mouse, rat, pig, cat, dog, horse, goat, cattle, cow, and monkey and/or others. In a preferred embodiment of the present invention, the sample contains isolated body fluid compounds or processed body fluids derived from a human.

In a preferred embodiment, the sample is pre-purified. In a more preferred embodiment of the present invention, the pre-purification comprises the step of removing impurities that impair significantly or prevent the growth of the cell.

In the following, the DNA hydrogel, the silica nanoparticles and the carbon nanotubes will be described.

(a) DNA hydrogel

A DNA hydrogel is a three-dimensional network of DNA strands held together by association bonds. The DNA hydrogel may contain aqueous components which are added during the gelling process of the DNA strands or added after the DNA hydrogel has been synthesized. DNA hydrogel is able to absorb a large quantity of water. Further, DNA hydrogel may be used as a scaffold material for forming three-dimensional structures having excellent elasticity.

Several methods for synthesizing DNA hydrogel are known in the art.

For example, Liu and co-workers reported the preparation of a fast forming, pH-responsive DNA hydrogel with three-armed DNA nanostructures (Y units) assembled together through the formation of intermolecular i-motif structures (Cheng, E.; Xing, Y.; Chen, P.; Yang, Y.; Sun, Y.; Zhou, D.; Xu, L.; Fan, Q.; Liu, D. A pH-triggered, fast-responding DNA hydrogel. Angew. Chem., Int. Ed. Engl. 2009, 121 (41), 7796-7799).

They also reported that DNA hydrogels can be prepared by crosslinking DNA building blocks by sequence-directed hybridization (Xing, Y.; Cheng, E.; Yang, Y.; Chen, P.; Zhang, T.; Sun, Y.; Yang, Z.; Liu, D. Self-assembled DNA hydrogels with designable thermal and enzymatic responsiveness. Adv. Mater. 2011, 23 (9), 1117-1121). Briefly, the Y-scaffold containing three sticky ends and complementary linker DNA (double-stranded DNA, dsDNA) with the two sticky ends were designed. Accumulative hybridization between those two blocks would lead to hydrogel formation.

Nöll et al. reported that three-dimensional DNA hydrogel was generated by self-assembly of short linear double-stranded DNA (dsDNA) building blocks equipped with sticky ends (Nöll, T.; Schönherr, H.; Wesner, D.; Schopferer, M.; Paululat, T.; Nöll, G. Construction of Three-Dimensional DNA Hydrogels from Linear Building Blocks. Angew. Chem., Int. Ed. Engl. 2014, 53 (32), 8328-8332). The resulting DNA hydrogel is thermoresponsive and the length of the supramolecular dsDNA structures varies with temperature.

In 2006, Luo and coworkers first reported the construction of a hydrogel entirely made from branched DNA *via* an enzymatic ligation reaction (Um, S. H.; Lee, J. B.; Park, N.; Kwon, S. Y.; Umbach, C. C.; Luo, D. Enzyme-catalysed assembly of DNA hydrogel. Nat. Mater. 2006, 5 (10), 797-801). In this process, DNA served as both a linker and a substrate. To form the DNA hydrogel, branched DNA motifs, such as X-, T-, or Y-shaped DNA (termed as X-DNA, Y-DNA, and T-DNA), were first prepared through the self-assembly of rationally designed ssDNA strands. They also reported an enzyme-catalyzed assembly of DNA hydrogels through 3D-entanglement of DNA products by combination of the processes of rolling circle amplification (RCA) and multi-primed chain amplification (MCA) (Lee, J. B.; Peng, S.; Yang, D.; Roh, Y. H.; Funabashi, H.; Park, N.; Rice, E. J.; Chen, L.; Long, R.; Wu, M.; Dan, L. A mechanical metamaterial made from a DNA hydrogel. Nat. Nanotechnol. 2012, 7 (12), 816-820). After running RCA and subsequent MCA, a continuous chain reaction was established, thereby creating extremely long DNA molecules which resulted in a physically linked DNA hydrogel.

By introducing G-quadruplex inside the RCA-generated DNA hydrogel, the structure and function of hemin/G-quadruplex moieties were implemented in the hydrogel, which showed a high capability in catalysing the $ABTS^{2-}$ colorimetric reaction (Huang, Y.; Xu, W.; Liu, G.; Tian, L. A pure DNA hydrogel with stable catalytic ability produced by one-step rolling circle amplification. Chem. Commun. 2017, 53 (21), 3038-3041).

Based on RCA, multifunctional DNA nanostructures termed nanoflowers (NFs) were self-assembled from long DNA building blocks for multiplexed cellular imaging/trackable targeted drug delivery (Hu, R.; Zhang, X.; Zhao, Z.; Zhu, G.; Chen, T.; Fu, T.; Tan, W. DNA nanoflowers for multiplexed cellular imaging and traceable targeted drug delivery. Angew. Chem., Int. Ed. Engl. 2014, 126 (23), 5931-5936; Zhu, G.; Hu, R.; Zhao, Z.; Chen, Z.; Zhang, X.; Tan, W. Noncanonical self-assembly of multifunctional DNA nanoflowers for biomedical applications. J. Am. Chem. Soc. 2013, 135 (44), 16438-16445; Lv, Y.; Hu, R.; Zhu, G.; Zhang, X.; Mei, L.; Liu, Q.; Qiu, L.; Wu, C.; Tan, W. Preparation and biomedical applications of programmable and multifunctional DNA nanoflowers. Nat. Protoc. 2015, 10 (10), 1508-1524; Sun, W.; Jiang, T.; Lu, Y.; Reiff, M.; Mo, R.; Gu, Z. Cocoon-like self-degradable DNA nanoclew for anticancer drug delivery. J. Am. Chem. Soc. 2014, 136 (42), 14722-14725) and immobilization of proteins (Kim, E.; Zwi-Dantsis, L.; Reznikov, N.; Hansel, C. S.; Agarwal, S.; Stevens, M. M. One-Pot Synthesis of Multiple Protein - Encapsulated DNA Flowers and Their Application in Intracellular Protein Delivery. Adv. Mater. 2017, 29, 1701086).

Wang *et al.* developed a clamped hybridization chain reaction (HCR)-based strategy, including crosslinked self-assembly and clamped hybridization of hairpin strands and their initiator strand, to guide DNA self-assembly to form hydrogel (Wang, J.; Chao, J.; Liu, H.; Su, S.; Wang, L.; Huang, W.; Willner, I.; Fan, C. Clamped Hybridization Chain Reactions for the Self - Assembly of Patterned DNA Hydrogels. Angew. Chem., Int. Ed. Engl. 2017, 56 (8), 2171-2175).

Preferably, the DNA hydrogel in the composite material of the present invention can be produced by an enzymatic reaction-mediated polymerization or by hybridization chain reaction starting with DNA strands which are covalently coupled to the surface of the silica nanoparticles. Both methods can also be combined to generate the DNA hydrogel by two complementary mechanisms.

According to an embodiment, variations of the DNA backbone in the hydrogel can be implemented. For example, the introduction of restriction sites in conventional DNA polymers enables the design of responsive materials that can be post-synthetically modified with enzymes. To introduce restriction sites into the single-stranded backbone of the composite material according to an embodiment, stem-loop structures of appropriate sequence can be encoded in a rolling circle amplification (RCA) template, thereby enabling efficient enzymatic degradation and concomitant gel-to-sol transition of the composite material. This feature is of great utility, for instance, to achieve controllable release of polymers or polymer-embedded cells, which is required for therapeutic applications and dynamic cell manipulations.

In a further embodiment, the DNA backbone may have an aptamer which specifically binds to a receptor of a cell. According to another embodiment, the DNA backbone may have a drug association site which can be used for enrichment of intercalating drugs.

According to another embodiment, fluorescent properties can be generated by rolling circle amplification (RCA)-based incorporation of chemically modified deoxynucleotides bearing fluorescent dyes, such as fluorescein (FITC)-modified dUTP.

(b) Silica nanoparticles

In the present invention, the average particle size of the silica nanoparticles (SiNP) is in the range of from 20 nm to 2 $\mu$m. According to a further embodiment, the average particle size is in the range of from 50 nm to 1 $\mu$m. In another embodiment, the average particle size is in the range of from 60 nm to 100 nm. The average particle size can be measured by a transmission electron microscope (TEM).

By providing silica nanoparticles having an average particle size in the range of from 20 nm to 2 $\mu$m, a composite material having an advantageous porosity and stability can be achieved.

In an embodiment, the silica nanoparticles are functionalized silica nanoparticles. The functionalization is not particularly limited as long as the chemical group allows the binding to a different molecule. Preferably, the functional group is at least one group selected from amino, thiol, phosphonate, carboxy, alkene, alkyne, halo-alkane, maleimide, succinimide and azide.

According to another embodiment, the silica nanoparticles have a fluorescence marker. This enables fluorescence tracking of the composite material and may be used for e.g. studies of cell uptake and degradation of hydrogels. For example, dye-encoded SiNP can be used. Dye-encoded SiNP can for example be obtained through co-hydrolysis of TEOS and Cy5 dye-conjugated silane as described in the literature (Leidner, A., Weigel, S., Bauer, J., Reiber, J., Angelin, A., Grösche, M., Scharnweber, T., Niemeyer Christof, M. (2018) Biopebbles: DNA-Functionalized Core-Shell Silica Nanospheres for Cellular Uptake and Cell Guidance Studies. Adv. Funct. Mater. DOI 10.1002/adfm.201707572 and references cited therein).

(c) Carbon nanotubes

In an embodiment of the present invention, the carbon nanotubes are single-walled carbon nanotubes. According to another embodiment, the carbon nanotubes are multi-walled carbon nanotubes. Other morphologies of carbon nanotubes, such as peapod and nanobud carbon nanotubes as well as nanotube derivatives containing junctions, metal-doped or metal-coated sites and other allotropes of carbon, such as fullerenes, also represent embodiments.

Preferably, the average diameter of single-walled carbon nanotube is from 0.5 to 1 nm, more preferred from 0.7 to 0.9 nm.

In an embodiment of the present invention, the median length of a carbon nanotube is from 500 nm to 1.5 $\mu$m, preferably from 750 nm to 1.25 $\mu$m, most preferred from 900 nm to 1.1 $\mu$m.

The average diameter and the median length of the carbon nanotube can either be retrieved from the manufacturer (e.g. in the Sigma website) or measured by commonly known techniques. The average diameter can be evaluated by using a Raman microscope and the median length can be measured by an atomic force microscope (AFM).

The carbon nanotubes used in the present invention may be commercially available carbon nanotubes provided by e.g. Sigma.

(2) Hollow spherical container

Further, the present invention relates to a hollow spherical container, wherein the wall of the hollow spherical container comprises at least one layer containing the previously defined composite material.

In an embodiment, the wall of the hollow spherical container further comprises at least one additional layer comprising at least one component selected from DNA-protein conjugate, DNA-ligand conjugate, a DNA origami nanostructure-based ligand assembly system, as well as proteins and ligands such as carbohydrates, hormones, peptides and small-molecule organic ligands. When at least one of such an additional layer is used, the at least one layer containing the previously defined composite material forms the outermost layer of the wall of the hollow spherical container while the at least one additional layer forms an inner layer of the wall of the hollow spherical container.

In another embodiment, additional functional components, such as proteins (e.g. membrane proteins), may be incorporated into the at least one layer of the hollow spherical container comprising the composite material.

The word "spherical" used herewith means that the ratio between the minor axis and the major axis of the container is not less than 0.8, preferably not less than 0.9, most preferably not less than 0.95.

Preferably, the wall of the hollow spherical container is porous. In an embodiment, the pore size of the wall is more than 200 nm and less than 500 nm. The preferred pore size can be measured as outlined above using MPT microrheology. By providing pores in the wall, for example small molecules may enter the inside of the hollow spherical container.

In an embodiment of the present invention, the hollow spherical container has a diameter of 30 $\mu$m or more and less than 1 mm. According to a further embodiment, the hollow spherical container has a diameter in the range of

from 50 $\mu$m to 200 $\mu$m, preferably from 80 $\mu$m to 160 $\mu$m. By setting the diameter in the above range, a container can be provided which allows performing applications on a small scale while being dimensionally stable. The smaller the diameter of the hollow spherical container is, the higher the dimensional stability.

According to an embodiment of the hollow spherical container, each layer has a thickness in the range of from 5 nm to 10 $\mu$m. In another embodiment, the thickness is in the range of from 50 nm to 8 $\mu$m, preferably from 100 nm to 5 $\mu$m. When having a thickness of less than 5 nm, the layer may not provide sufficient dimensional stability.

In an embodiment of the hollow spherical container, the thickness of all layers is in the range of from 5 nm to 100 $\mu$m. In an embodiment, the thickness is in the range of from 50 nm to 50 $\mu$m, preferably from 100 nm to 10 $\mu$m. When having a thickness of less than 5 nm, the total thickness of the layers may not provide sufficient dimensional stability.

The diameter of the hollow spherical container, the thickness of each layer, and the thickness of all layers comprised in the hollow spherical container can be measured by using confocal laser scanning fluorescence microscopy. The thickness of all layers refers to the length of the hydrogel fluorescence shell measured by confocal laser scanning fluorescence microscopy. The thickness (T) of all layers can be calculated according to the formula $T = T2 - T1$, where T2 and T1 are Cy5 fluorescence (included in the shell of the container) starting point and ending point in shell of the image, respectively. The positions of T1 and T2 are determined from microscopic images by the commercial software Zen Blue and the connecting line between T1 and T2 is perpendicular to the tangent of T1,

According to an embodiment, the wall of the hollow spherical container comprises at least two layers. Further, the wall of the hollow spherical container may comprise at least three layers.

In a preferred embodiment, the at least two layers in the wall of the hollow spherical container differ in their composition. By combining at least two layers, the dimensional stability of the hollow spherical container can be advantageously adjusted. For example, each of the two layers may comprise the composite material, wherein the content of the silica nanoparticles, the carbon nanotubes and the DNA hydrogel are adjusted. In another embodiment, the previously defined additional layer may be used in combination with at least one layer comprising the composite material.

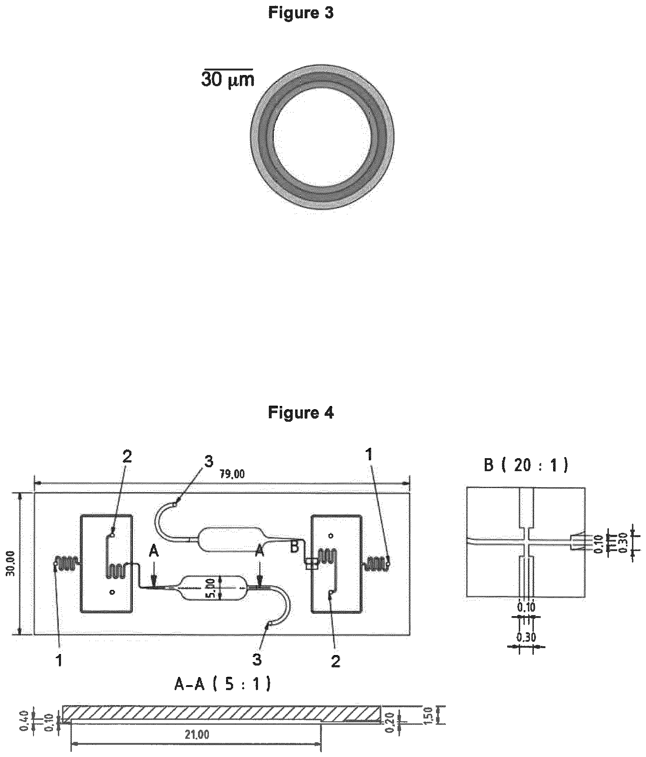

Figure 3 exhibits an example of a hollow spherical container according to the present invention, wherein the container comprises three layers.

In a preferred embodiment, the hollow spherical container is obtained by a process comprising the following steps in the order:

(i) providing a reaction solution comprising the components of the composite material;
(ii) providing a continuous phase which is immiscible with the reaction solution; and
(iii) combining the reaction solution and the continuous phase thereby forming droplets comprising the reaction solution, wherein the droplets are dispersed in the continuous phase and the components of the composite material contained in the reaction solution are absorbed to the inner surface of the droplets.

In an embodiment of the present invention, the components of the composite material provided in the above step (i) comprise the previously defined silica nanoparticles and DNA strands which form the DNA hydrogel during the above process. Preferably, the previously defined carbon nanotubes are also contained in the components of the composite material in step (i). In a further embodiment, the components of the composite material comprise the additional previously defined compounds which may be present in the composite material.

The reaction solution and the continuous phase are not specifically limited as long as they are immiscible, and the reaction solution comprises the components of the composite material.

According to an embodiment of the present invention, the continuous phase is a liquid phase or a gas phase.

In an embodiment, one of the reaction solution and the continuous phase is a hydrophobic phase while the other is a water-based phase. According to a preferred embodiment, the reaction solution is a water-based phase and the continuous phase is a hydrophobic phase.

An oil is preferably used in the hydrophobic phase. The oil may be selected from a mineral oil or a fluorocarbon oil. An example for a mineral oil is bioreagent for molecular biology from Roth (Mineralöl für die Molekularbiologie, Art. HP50.3). Further, as the fluorocarbon oil, at least one of QX200 Droplet Generation Oil for EvaGreen (BioRad), HFE 7500 (3M), Fluoridrop 7500 (Dolomite Microfluidics), Fluoridrop 40 (Dolomite Microfluidics), FC40 (3M) and Novec 7500 (3M) may be used.

In an embodiment of the present invention, at least one of the reaction solution and the continuous phase comprises a surfactant. The surfactant is not particularly limited as long as it changes the interfacial tension between the reaction solution and the continuous phase. In specific, the surfactant can be an ionic or a non-ionic surfactant. Examples of surfactants are PicoSurf 1 (Dolomite Microfluidics), Span 40/80, Tween20/80, SDS, ABIL EM90 (Baret, J.-C. Surfactants in droplet-based microfluidics. Lab Chip 2012, 12 (3), 422-433), 1,2-dioleoyl-3-trimethylammonium-propane (DOTAP), triton X-100, ABIL EM90, oleic acid, monolein, Krytox FSH, 1,2-dioleoyl-sn-glycero-3-phosphocholine (DOPC), 2-oleoyl-1-palmitoyl-sn-glycero-3-phosphocholine (POPC), 1,2-dipalmitoyl-sn-glycero-3-phosphoethanolamine (DPPE), 1,2-dimyristoyl-sn-glycero-3-phosphocholine (DMPC), 1,2-dipalmitoyl-sn-glycero-3-phosphocholine (DPPC), and hydrophobic particles.

According to a preferred embodiment, the continuous phase comprises an anionic or a cationic surfactant. By using an anionic or a cationic surfactant in the continuous phase, the droplet can be advantageously stabilized. Further, provided that the components of the composite material are oppositely charged compared to the surfactant, said components of the composite material can be easily absorbed to the inner surface of the droplets by electrostatic attraction.

In a preferred embodiment of the present invention, a positively charged surfactant, such as DOTAP, is used as a surfactant in the continuous phase. By using a combination of a positively charged surfactant in combination with negatively charged DNA molecules, carbon nanotubes or silica nanoparticles or negatively charged DNA strand-modified silica nanoparticles or negatively charged DNA strand-modified carbon nanotubes, the components of the composite material can be easily absorbed to the inner surface of the droplet.

According to an embodiment, in the process for obtaining the hollow spherical container, the combining in step (iii) is carried out with a microfluidic droplet generator. In another embodiment, all of the steps of the process for obtaining the hollow spherical container are carried out with a microfluidic droplet generator.

Preferably, in step (iii), the droplets are generated in flow, i.e. the reaction phase passes a narrow point while being combined with the continuous phase upon which the reaction phase forms droplets being dispersed in the continuous phase, since the form of a droplet is energetically favorable. The optionally added surfactant increases the tendency of forming droplets.

The microfluidic droplet generator used in the embodiment of the present invention is not particularly limited as long as it allows the generation of droplets in the sub-millimeter scale.

The use of a microfluidic droplet generator in contrast to commonly used droplet techniques is favorable, since it can provide the generation of highly monodisperse droplets with the advantage of low sample consumption. The droplets are formed, if two immiscible liquids are combined for a defined time.

In an embodiment of the present invention, the volume of a droplet is in a range of from 0.2 nL to 20 nL. Preferably, the volume of a droplet is in the range of from 0.3 nL to 2 nL. The volume of a droplet can be calculated according to the volume formula $4/3*\pi*r^3$, where r is the droplet radius which can be calculated from microscopic images. The monodispersity depends on the specific combination of the components.

In order to generate droplets by using a microfluidic droplet generator, various methods can be used such as the T-junction, Head-on, Double T-junction, Flow-Focusing and Step-Emulsification. These methods are advantageous, since they can be precisely tuned by varying a number of different parameters. According to a preferred embodiment, the Flow-Focusing method is used, since said method can provide a higher monodispersity and a higher amount of droplets being generated in the same amount of time compared to other methods.

In a microfluidic droplet generator, the generation of droplets comprising different compounds and the addition of new compounds at defined time points can be achieved with various unit operations. The simplest approach is to premix everything outside the generator resulting in the encapsulation of all compounds. If the compounds should not react with each other before encapsulation, they can be encapsulated by two laminar flows meeting at the encapsulation junction. A second option is to generate two droplets in different junctions and fuse them in either passive structures or active structures. Passive structures only use geometrical confinements in order to change the droplet stability regarding unwanted coalescences, whereas active structures use electrodes to change the interfacial tension of the droplets and the surrounding oil. Another possible method is the so-called picoinjection. With this procedure, a second fluid is directly injected into the droplet via a side channel. Since microfluidic flows are total laminar, a meander like structure is advantageous to support combining the different compounds.

In an embodiment of the combining step (iii) in the process for obtaining the hollow spherical container, each of the reaction solution and the continuous phase has a flow rate in the range of from 0.1 μL/min to 1000 μL/min. By adjusting the flow rates of each of the reaction solution and the continuous phase, the volume of the droplets can be precisely varied. Preferably, the flow rates of each of the reaction solution and the continuous phase is in the range of from 0.5 μL/min to 100 μL/min. More preferred is a flow rate for the continuous phase in the range of from 10 to 30 μL/min while the reaction solution has a flow rate in the range of from 0.5 μL/min to 5 μL/min. When using said specific combination of the flow rates for the reaction solution and the continuous phase, droplets with a defined volume in the range of from 0.3 nL to 2 nL can be generated.

In addition, the volume of the droplets can be precisely varied by using different channel geometries, flow rates and components in the reaction solution and the continuous phase.

According to an embodiment, the droplets are collected after step (iii) and incubated. For collecting the droplets, a well plate may be used. In an embodiment of the present invention, the droplets are incubated at a temperature in the range from 20 °C to 40 °C, preferably from 25 °C to 37 °C. In another embodiment, incubation is carried out for 1 to 60 h, preferably from 40 to 50 h.

In the following, an example of a microfluidic droplet generator, which can be used in the present invention, will be described.

The material which can be used for the structure of the microfluidic droplet generator is not particularly limited.

For example, one of polydimethylsiloxane (PDMS) and poly(methyl methacrylate) (PMMA) can be used. In order to close the structure to enable fluidic application, the structure can be exposed to thermal plasma oxidation and be bonded onto glass (e.g. microscope slides) or a sealing foil (e.g. polyolefin) can be applied.

In Figure 4, an example of a microfluidic droplet generator which can be used in the present invention is shown which comprises five elements/functional units.

The first unit is the inlet for the continuous phase 1, which comprises two identical arms with any desired profile. In the specific example shown here it is 300 $\mu$m $\times$ 100 $\mu$m (width $\times$ height).

The second unit is the inlet for the reaction solution 2, which intersects the two arms of the first unit, resulting in the third unit, i.e. the junction for droplet generation (see detail B in Figure 4).

The junction (i.e. the third unit) has a rectangular canal profile of 100 $\mu$m $\times$ 100 $\mu$m in the specific example shown here.

The fourth canal making up this junction leads to the droplet collection chamber (i.e. the fourth unit). In the specific example shown here, the dimensions of the droplet collection chamber are 21 $\times$ 5 mm (length $\times$ width) with a depth of 400 $\mu$m in the main part of the canal structure. Coming from the entry canal (in the example: 100 $\mu$m $\times$ 100 $\mu$m), the profile is expanded stepwise until the full chamber dimensions are met.

The fifth element is the outlet 3 with a profile of 800 $\mu$m $\times$ 200 $\mu$m (width $\times$ height) in the specific example shown here. The inlets/outlets can be realized by using drills with a diameter of 800 $\mu$m. The inlets/outlets can be sealed to a connector plate with o-rings made from NBR 70. The connector plate is preferably in turn connected to precision pumps utilizing special fittings and tubings.

In an embodiment, the channels of the microfluidic droplet generator can be treated with substances which make the channels more hydrophobic and/or fluorophobic and/or hydrophilic. Preferably, the channels can be treated with Pico-Glide (Dolomite Microfluidics) or via layer-by-layer deposition of poly(allylamine hydrochloride) and poly-(sodium 4-styrensulfonate) (also see Bauer, W.-A. C.; Fischlechner, M.; Abell, C.; Huck, W. T. Hydrophilic PDMS microchannels for high-throughput formation of oil-in-water microdroplets and water-in-oil-in-water double emulsions. Lab Chip 2010, 10 (14), 1814-1819).

(3) Microreactor

Further, the present invention relates to a microreactor comprising the above-defined hollow spherical container and at least one cell located in the space enclosed by the hollow spherical container. The microreactor according to the present invention can be advantageously used for investigations of cells, since the composite material according to the present invention does not show cytotoxicity.

The at least one cell located in the space enclosed by the hollow spherical container is not particularly limited and corresponds to the cell type defined in the context of the composite material.

The term "microreactor" according to the present invention means a device with a size of less than 1 mm in which chemical or biological reactions can be carried out.

Preferably, the microreactor can be used for the cultivation of cells inside the microreactor. The hollow spherical container provides mechanical stability for the manipulation, but at the same time cells can grow inside. Preferably, small molecules can enter the hollow spherical container due to the walls being porous. The at least one cell located in the space enclosed by the hollow spherical container can be supplied with nutrients and/or treated with pharmaceuticals to analyze the cellular response.

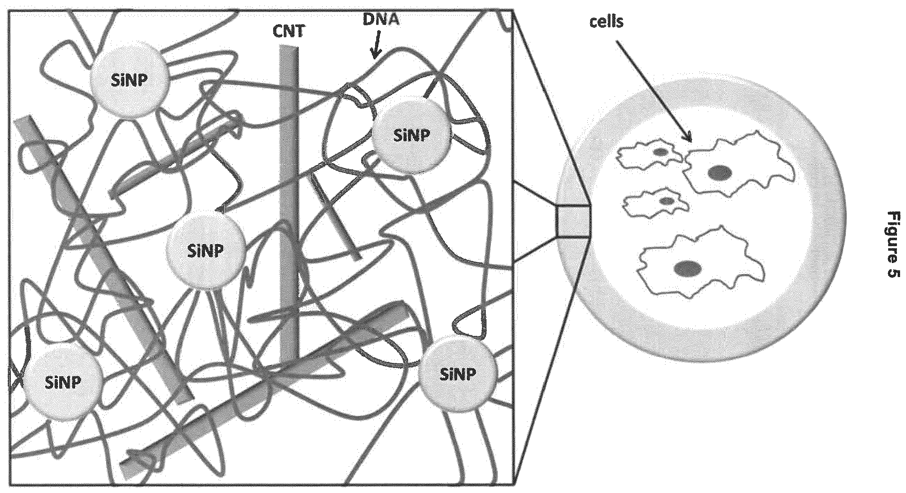

In Figure 5, an example of a microreactor according to the present invention is shown on the right side which comprises several cells in the space enclosed by the hollow spherical container.

Based on the variation of the components contained in the composite material, the wall thickness and the diameter, the hollow spherical container may be easily moved mechanically. Further, the hollow spherical container is compatible with the at least one cell which is enclosed by said hollow spherical container. Due to the encapsulation of the at least one cell in the microreactor, individual experiments can be carried out at a small scale.

In an embodiment of the present invention, the microreactor is semipermeable. The term "semipermeable" used herein means that certain molecules and/or ions can pass through the wall of the microreactor while other substances cannot pass the wall. According to an embodiment, substances that can pass the wall of the microreactor may be chosen from substances that are necessary for the cell viability and/or therapeutic products. The degree of permeability of the microreactor can be adjusted by the porosity of the wall of the hollow spherical container.

Preferably, the microreactor is optically transparent and thereby enables the application of high-throughput screening. Due to the size of the microreactors, pharmacological screens can be carried out at a small scale.

According to an embodiment of the present invention, individual DNA oligomers may be introduced as codes into the wall of the hollow spherical container which allows decoding or deconvolution to identify the microreactor and the substances being located inside the microreactor after performing experiments.

The present invention further relates to the use of the above-defined microreactor for high-throughput screening.

(4) Microgels

Further, the present invention relates to a microgel comprising the composite material according to the present invention.

The term "microgel" according to the present invention refers to a hydrogel of microscale size, wherein the average particle size of the microgel is less than 1 mm.

In an embodiment of the present invention, the microgel has an average particle size of from 0.05 to 50 $\mu$m, preferably from 0.1 to 20 $\mu$m, more preferably, from 1 to 10 $\mu$m, and, even more preferably, from 1 to 5 $\mu$m. The average particle size is measured by using fluorescence microscopy.

In the microgel comprising the composite material, the carbon nanotubes (CNT) can be mixed with the silica nanoparticles (SiNP) in different weight ratios when the ternary composite material is employed. Preferably, the mass ratio of SiNP:CNT in the ternary composite material is from 100:1 to 1:1, preferably from 50:1 to 2.5:1. In an embodiment, the maximum concentration of SiNP in the binary SiNP-DNA hydrogel composite material and in the ternary SiNP/CNT-DNA hydrogel composite material is 20 mg/mL, preferably 4000 $\mu$g/mL. According to a further embodiment, the maximum concentration of CNT in the ternary SiNP/CNT-DNA hydrogel composite material is 1000 $\mu$g/mL, preferably 320 $\mu$g/mL.

The method for manufacturing the microgel is not particularly limited. For example, micellar techniques, using, for instance, "inverse micelles" of water-in-oil droplets can be used. Then, after incubation, the particles of the microgel can be collected.

Further, the present invention relates to the use of the microgel for the delivery of cargo to the cytosolic compartment of cells. Owing to the favorable characteristics and biocompatibility, the microgel is readily ingested by cells whereupon it might be transported within the cell. In a preferred embodiment, during this transport, the microgel remains intact and, more preferably, resides in close proximity to the nucleus. Thus, the microgel of the present invention can be used to realize delivery of cargo to cells. The cargo to be delivered is not particularly limited and can be selected from pharmacologically active compounds, drugs, metals, metal and semiconductor nanoparticles, metal chelates, proteins, peptides, and DNA molecules that encode genes for RNA and protein production.

In an embodiment, the microgel of the present invention can be used for gene transfection. Preferably, the microgel can be used as a "synthetic cellular compartment" for the production of endogeneous components, such as messengerRNA or small-interfering RNAs, which has many implications for diagnostics and therapy *in vitro* and *in vivo.*

The cell employed in the above uses of the microgel is not particularly limited and corresponds to the cell type defined in the context of the composite material.

The composite material according to the present invention not only displays cell attractiveness and stiffness, but can also be conveniently designed with inherent functionality of the connecting DNA backbone by incorporation of enzymatic restriction sites and aptamer moieties for specific binding of cellular components, such as proteins, peptides, nucleic acids or glycans located on the inner or outer side of the cell surface or intracellular structures, such as the nucleus, chromosomes, mitochondria, endoplasmatic reticulum or cytosolic vesicles.

Adapting the relative amount of SiNP:CNT leads to gradual changes in the mechanical properties. This feature can be used for controlling cellular adhesion on and transmigration through the hydrogel matrix as well as for controlling cellular uptake and intracellular stability and degradation.

The composite material of the present invention can be employed as a novel substratum for cell adhesion that outperforms standard tissue culture surfaces.

Further, the modularity of the synthesis allows convenient incorporation of fluorescent nanoparticles which renders the composite material of the invention trackable by optical means, which is of utmost importance for biomedical studies, such as materials uptake, cellular degradation or other cell-surface interactions based on adhesion, motility and propagation.

The figures show:

Figure 1 shows an embodiment of the composite material of the present invention comprising DNA hydrogel, silica nanoparticles (SiNP) and carbon nanotubes (CNT).

Figure 2 exemplifies RCA-based synthesis for obtaining binary and ternary SiNP/CNT-DNA hydrogel composite materials. SiNP and/or CNT modified with ssDNA primers are subjected to RCA-based primer extension, which leads to formation of the corresponding pure (S100, binary SiNP-DNA hydrogel or C100, binary CNT-DNA hydrogel) or mixed (SCx (ternary)) composite materials. While S100 and SCx represent embodiments of the present invention, C100 is a general example.

Figure 3 exhibits an example of a hollow spherical container according to the present invention, wherein the container comprises three layers.

Figure 4 shows an example of a microfluidic droplet generator which can be used in the present invention.

Figure 5 displays an embodiment of the microreactor according to the present invention on the right side comprising several cells in the space enclosed by the hollow spherical container.

Figure 6 shows an example of the synthesis of multifunctional oligonucleotide-modified SiNP.

Figure 7 displays SEM images of freeze-dried SiNP-DNA hydrogel synthesized by RCA of P1 primer on SiNP-P at 30 °C for 48 h.

Figure 8 presents DLS measurements of the hydrodynamic size of RCA products produced from RCA of P1 primer on SiNP-P after hybridized with template T in the presence or absence of T4 ligase (insert is the digital photograph of SiNP-DNA hydrogel synthesized by RCA of P1 primer on SiNP-P at 30 °C for 48 h).

Figure 9 shows the variation of dynamic shear-moduli G' (closed symbol) and G" (open symbol) of SiNP-DNA hydrogel synthesized by RCA of P1 primer on SiNP-P at 30 °C for 48 h as a function of frequency.

Figure 10 exhibits the hydrodynamic size of HCR products produced from HCR of H1 and H2 on SiNP-lh or SiNP-I (insert is the digital photograph of SiNP-DNA hydrogel synthesized by HCR of H1 and H2 on SiNP-I under room temperature for 48 h).

Figure 11 displays SEM images of freeze-dried S100 hydrogel products generated from HCR of H1 and H2 on SiNP-I at room temperature for 48 h.

Figure 12 shows the results of a frequency-sweep rheological test (0.05 and 100 rad s$^{-1}$, 1% strain, 25 °C) for SiNP-DNA hydrogel S100 generated *via* HCR of H1 and H2 on SiNP-I at room temperature for 48 h.

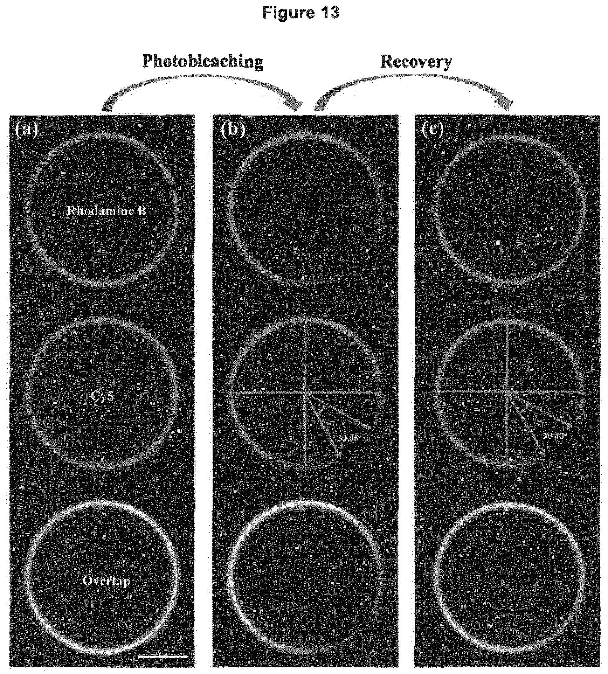

Figure 13 exhibits CLSM images of DOTAP droplets with Cy5-doped SiNP as an interior surface after incubation at 30 °C for 48 h. Three images show before photobleaching (a), after photobleaching (b), and after fluorescence recovery for 2 h (c), respectively. The central angels between two arrows represent the bleaching range of the membrane and the scale bar represents 50 $\mu$m. Note that fluorescence recovery after photobleaching occurs on a fast time scale for the DOTAP lipid molecules (top row) while the (middle row) SiNP show a much lower diffusion rate, thereby indicating high cross-linking rates and successful polymerization of the DNA-SiNP hydrogel.

Figure 14 shows the structural features of an embodiment of the SC50 composite material comprising silica nanoparticles, carbon nanotubes and a DNA hydrogel characterized by SEM. The SCx materials of Table 2 show similar morphological structures.

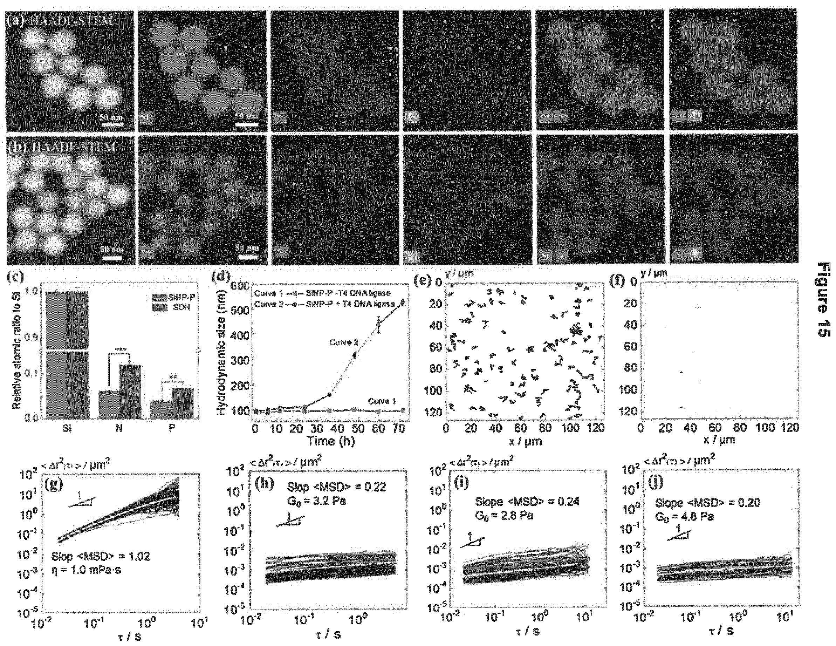

Figure 15 shows the characterization of an embodiment of SiNP/CNT-DNA hydrogel composite materials. Self-assembly of SiNP to S100 during RCA reaction. (a and b) Representative HAADF-STEM imaging (first column), EDS elemental mapping (second to sixth column in Figures a and b) and (c) relative atomic ratios of N and P to Si of SiNP-P and S100$_{48h}$ (shown as "SDH"), respectively. Data represent mean $\pm$ S.D. of the EDS measurements determined over fifty particles in four random areas, (d) Hydrodynamic size of products produced from RCA on SiNP-P constructed in the presence or absence of T4 DNA ligase (inset is the photograph of S100$_{48h}$). (e, f) the trajectory map of tracer particles and (g, h) the slope <MSD> of tracer particles in SiNP-P and S100$_{48h}$, respectively. (i, j) the slope <MSD> of tracer particles in C100 and SC50, respectively. The bright line represents the average MSD.

Figure 16 shows representative TEM images of SiNP-P and S100 obtained after 48 h of RCA reaction.

Figure 17 shows representative SEM images of S100 samples drawn at variable time points during RCA polymerization. Note that the sample drawn at t=0 (S100$_{0h}$) only shows SiNP particles that aggregate to superlattices in the course of dehydration during SEM specimen preparation.

Figure 18 shows examples of the incorporation of fluorescent properties into S100 *via* (a) the use of Cy5 fluorescently-labeled SiNP, (b) enzymatic introduction of chemically modified deoxynucleotides (FITC-dUTP), and (c) through both aforementioned strategies. The resulting hydrogels were analyzed by confocal fluorescence microscopy. The shown images are 3D reconstructions from z-stack analyses (Cy5 dark gray, FITC light gray).

Figure 19 shows representative SEM images of Cy5-encoded S100 materials (Cy5@S100 (Figure 19a), S100-FITC (Figure 19b), and Cy5@S100-FITC (Figure 19c).

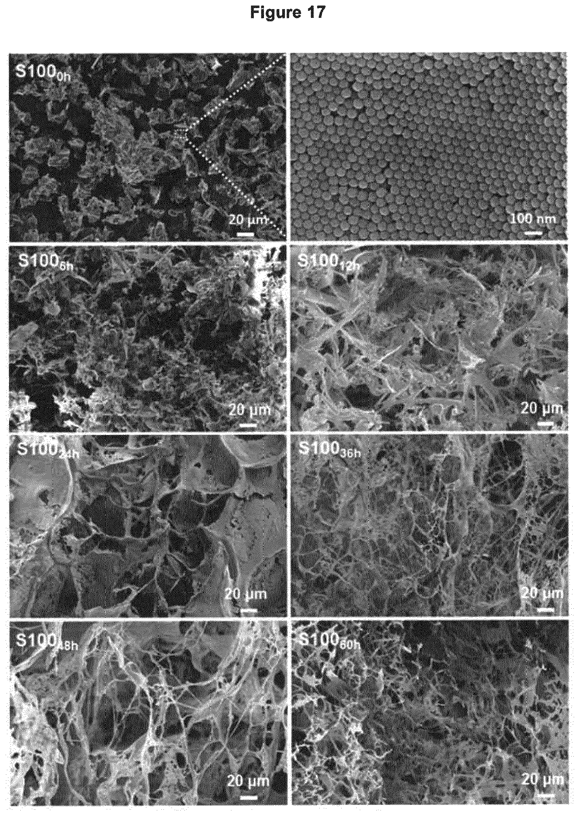

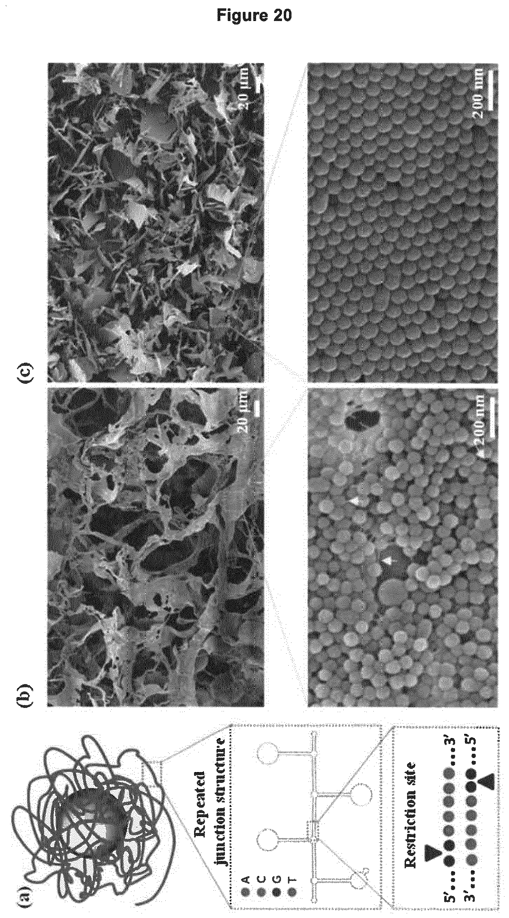

Figure 20 illustrates the engineering of the ssDNA backbone by incorporation of enzymatic restriction sites. (a) Predicted secondary structures of RCA product obtained from template T bearing stem-loop structures and double stranded regions with BstEII restriction sites (5'-GAATTC-3') in a repeated junction structure. SEM images of S100 treated for 2 h (37 °C) with restriction enzymes BstEII-HF (b) or, as a control, with SexAI (c). The arrows point at representative DNA linkers between particles. Note that enzymatic digestion leads to particle assemblies (b) similar as obtained from unpolymerized SiNP (Figure 17), whereas treatment with the non-complementary restriction endonuclease SexAI does not change the gel's morphology (c).

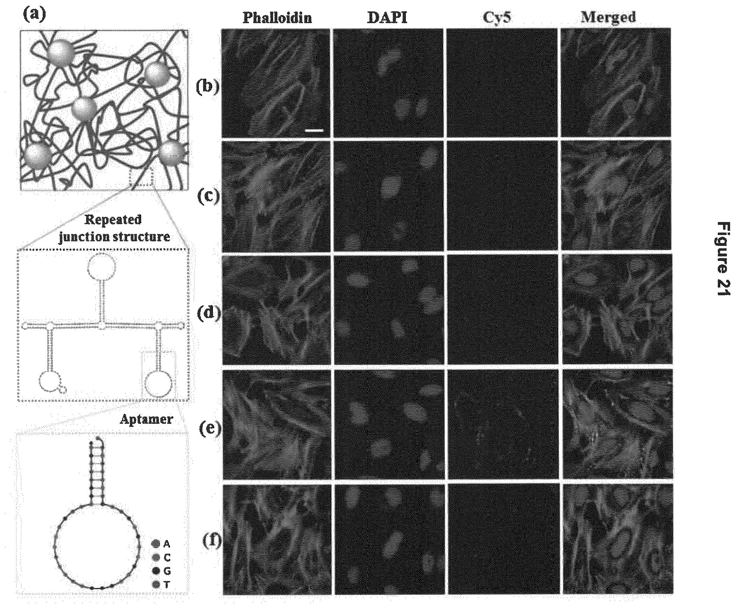

Figure 21 shows an example of fluorescently trackable uptake of SC100 by HeLa cells. (a) Predicted secondary structures of the RCA product obtained from template T. Structures were predicted using the Nupack software (J. N. Zadeh, C. D. Steenberg, J. S. Bois, B. R. Wolfe, M. B. Pierce, A. R. Khan, R. M. Dirks, N. A. Pierce, J. Comput. Chem. 2011, 32, 170). Note the presence branched Sgc8 aptamer structures protruding from the backbone. The Sgc8 aptamer specifically binds to PTK7 receptors overexpressed on many cancer cells (D. Shangguan, Y. Li, Z. Tang, Z. C. Cao, H. W. Chen, P. Mallikaratchy, K. Sefah, C. J. Yang, W. Tan, Proc. Natl. Acad. Sci. U. S. A. 2006, 103, 11838; G. Zhu, R. Hu, Z. Zhao, Z. Chen, X. Zhang, W. Tan, J. Am. Chem. Soc. 2013, 135, 16438). According to an embodiment, RCA polymerization was carried out with Cy5-embedded SiNP to yield nanocomposite $S100_{apt}$. (b-f) Confocal fluorescence microscopy images of PTK7-positive HeLa cells treated with 100 $\mu$M $S100_{apt}$ that was polymerized by RCA for 0 h (b), 2 h (c), 4 h (d), or 6 h (e). For control, cells in (f) were treated with 100 $\mu$M of the free aptamer. Note that increasing polymerization times (and thus increasing amounts of incorporated aptamers) lead to increase in Cy5 fluorescence (third column). Filamentous actin and nuclei were stained with Alexa Fluor 488 Phalloidin (stripe-like structures in the first and fourth column) and DAPI (spots in second and fourth column), respectively. Scale bars are 20 $\mu$m.

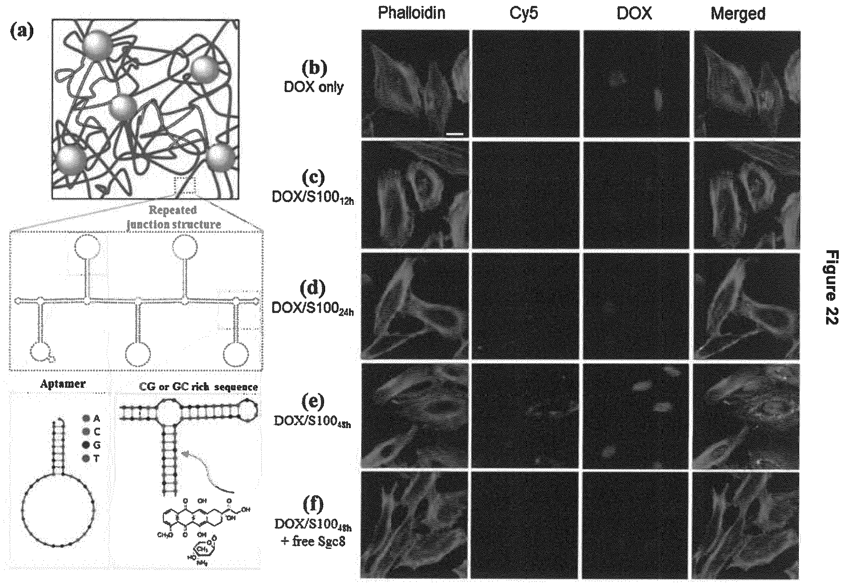

Figure 22 shows an example of trackable drug delivery to PTK7-positive HeLa cells. (a) Predicted secondary structure of the DNA backbone of fluorescent S100 material produced by RCA according to an embodiment. Note the presence of Sgc8 aptamer and GG/GC-rich stem loops, the latter of which serve as binding sites for the intercalating drug Doxorubicin (DOX). Representative confocal microscopy images of HeLa cells treated with (b) 5 $\mu$M free DOX or S100, polymerized for variable times (c, 12 h; d, 24 h; e and f, 48 h) before loading with 5 $\mu$M DOX. The cells in (f) were additionally treated with free Sgc8 aptamer. In all cases, cells were incubated with DOX/S100 for 2 h, washed, fixed and subjected to staining of the filamentous actin with Alexa Fluor 488 Phalloidin (stripe-like structures in the first and fourth column). Scale bars are 20 $\mu$m. Note that the amount of Cy5-labeled S100 (second column) as well as of DOX (spots in third and fourth column) increases with increasing amounts RCA-synthesized DNA backbone.

Figure 23 exhibits an example of a CCK-8 viability assay of MCF-7 cells after treated with SiNP-DNA hydrogel (S100), CNT-DNA hydrogel (C100), SiNP/CNT-DNA composite hydrogel (SCx, where x gives the mass ratio of SiNP-P:CNT-P).

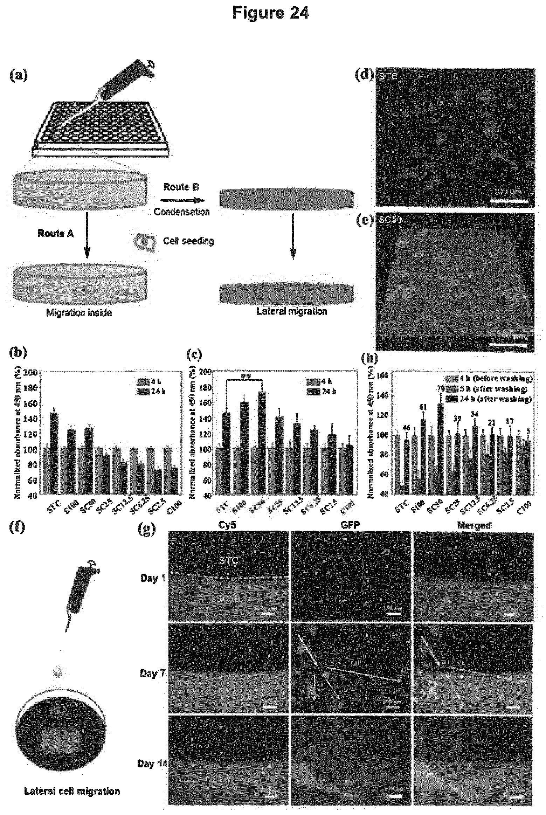

Figure 24 shows viability, proliferation and migration of MCF7$_{eGFP}$ cells adhered on thin films of the various composite materials according to an embodiment, (a) Schematic illustration of the preparation of fresh and dried composite material films for cultivation of cells. (b,c) Quantification of cells grown on fresh (b) or dried (c) thin films using the CCK-8 assay. The bars indicate relative cell numbers normalized to initially seeded cells (4 h, left column). STC data was obtained from polylysine-coated glass surface lacking hydrogels. (d,e) Representative fluorescence microscopy images of MCF7$_{eGFP}$ cells (spots) after adhesion on STC or SC50 surfaces for 24 h. (f, g) Directional lateral migration of MCF7$_{eGFP}$ cells (spots), which were seeded in a petri dish on STC surface and allowed to migrate to a patch of Cy5-labeled dried SC50. The fluorescence microscopy images of the border region between STC and SC50 surfaces (g) illustrate the gradual migration of the cells from STC to SC50 surfaces.

Figure 25 shows an example of transmitted light inverted microscopy (EVOS™ FL Imaging System) analysis of MCF7 cells adhered for 24 h on the various surfaces of dried hydrogels or, for control, on STC surface. Note that cells on S100 and SCx surfaces reveal a more pronounced elongated fusiform and flattened morphology than cells adhered on STC surface.

Figure 26 shows examples of fluorescence microscopy images of MCF7$_{eGFP}$ cells cultivated on dried SC50 surface prepared from Cy5-labeled SiNP. Note that the cells have not transmigrated through the dried gel matrix. The shown images are 3D reconstructions from z-stack analyses (Cy5: surface, GFP: spots on surface).

Figure 27 shows examples of transmitted light inverted microscopy (EVOS™ FL Imaging System) images of REF52 cells adhered onto the various dried hydrogels and STC surfaces for 4 h and 24 h. Note that cells on S100 and SCx surfaces reveal a more pronounced elongated fusiform and flattened morphology than cells adhered on STC surface.

Figure 28 shows quantification of REF52 cell proliferation grown on dried hydrogel films using the CCK-8 assay. The right bars indicate relative cell numbers normalized to initially seeded cells (4 h). STC data was obtained from polylysine-coated glass surface lacking hydrogels.

Figure 29 shows an example of fluorescence microscopy analysis of MCF7$_{eGFP}$ cells cultivated for 24 h on the dried Cy5-labeled SiNP-P (Cy5: stripes in the background and the role in front, GFP: spots).

Figure 30 shows an example of competitive adhesion of MCF7$_{eGFP}$ cells (spots) to either STC or Cy5-labeled dried SC50 (right-hand site). 1 mL medium containing $1.2 \times 10^4$ MCF7$_{eGFP}$ cells was added to a petri dish (Ibidi) containing a patch of dried SC50 (a) Schematic drawing of the experiment, (b) Fluorescence microscopy image of the border region between STC and SC50 surfaces.

Figure 31 shows an example of the relative cell number of MCF7 cells adhered for 1 h (left column), thoroughly washed to remove loosely attached cells (middle column), followed by culturing for additional 24 h (right column). The numbers indicate percentage increase of proliferated cells. Note that increasing amounts of CNT lead to increased adhesion strength but decreased proliferation.

Figure 32 shows an example of cellular transmigration, adhesion and release. Fluorescence images of MCF7$_{eGFP}$ cells (spots) cultivated on freshly prepared Cy5-labeled SC50 and SC25 (layer) for variable times.

Figure 33 shows an example of flow-assisted capture and release of MCF7$_{eGFP}$ in a microchannel coated with SC25 before (i) and after (ii) endonuclease digestion. The released cells are viable (iii). The scale bars are 200 $\mu$m.

Figure 34 shows an example of stem cell cultivation and maintenance of mESC sternness, (a) Quantification of time-dependent mESC growth on various substrata, normalized to data obtained from mESC on PLL for 4 h (dashed line). All data are represented by mean ± S.D. of triplicate samples. (b) Representative fluorescence images of immunostained mESCs grown for 4 days on gelatine-coated PLL in the absence (upper row) or presence (middle) of LIF or on S100 in the absence of LIF (bottom). Scale bars in b are 50 $\mu$m.

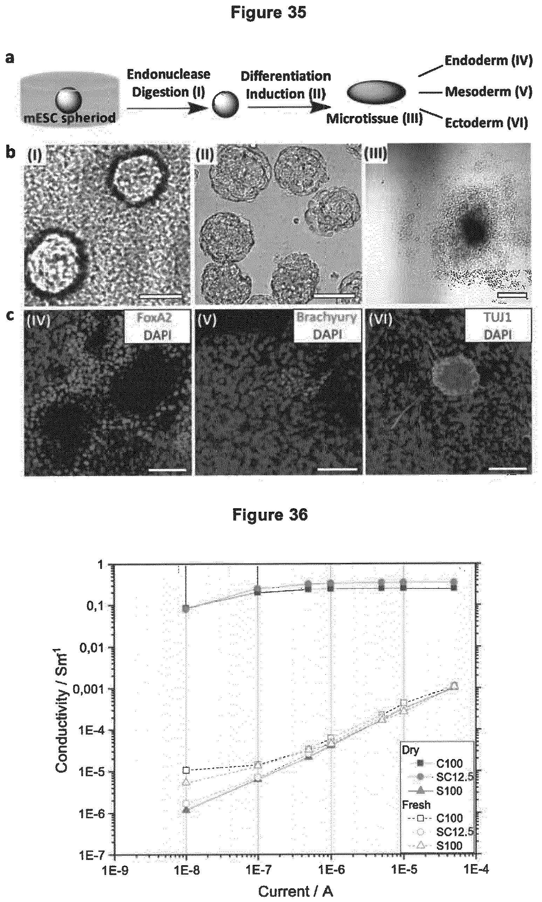

Figure 35 shows an example of release and differentiation of mESC spheroids. (a) Schematics of mESC culturing, release of spheroids and expression of germ layer markers in replated aggregates. (b) Bright field images of mESC spheroids before (I) and after release (II) from S100 culture materials and formation of differentiating colonies (III), observed via the outgrowth of cells in the periphery. (c) Representative fluorescence images of immunostained markers for germ layers in the colonies after 14 days. Endoderm (IV), Mesoderm (V) and Ectoderm (VI). Scale bars 50 $\mu$m (I, II), 500 $\mu$m (III), or 100 $\mu$m (IV, V, VI).

Figure 36 shows an example of the electrical conductivity of composite materials according to the present invention measured on 10 $\mu$m IDA.

Figure 37 shows E. coli cells (dots, on top of the layer) grown on the fresh S100 and SC12.5 (layer at the bottom), respectively, grown for 2 h (top images) or 24 h (bottom images). Scale bars are 50 $\mu$m. Note that, after 24 h, E. coli cells stay on top of the S100 while they transmigrate into the SC12.5 composite material.

Figure 38 shows Shewanella cells (dots, on top of the layer) grown on the fresh S100 and SC12.5 (layer at the bottom), respectively, grown for 2 h (top images) or 24 h (bottom images). Scale bars are 50 $\mu$m. Note that, after 24 h, Shewanella cells transmigrate into both materials but propagate to a much greater extent in the SC12.5 composite material.

Figure 39 shows the time course of eGFP expression observed during *in vitro* transcription/translation using S100 P-gel, SC12.5 P-gel, pure DNA P-gel or pure plasmid as well as the commercial coupled transcription and translation kit (Rapid Translation System, RTS) employed in the solution-phase-system (SPS).

Figure 40 shows an example of a fluorescence image of the SiNP/CNT-DNA composite microgel SC50 according to an embodiment.

Figure 41 shows an example of the fluorescence image of REF52 cells after incubation with SiNP/CNT-DNA microgel SC50 according to an embodiment. Note that the microgel particle (irregular-shaped spot) remained intact and resides in close proximity to the nucleus (round spot in the middle).

Figure 42 shows an example of fluorescence images of REF52 cells after being incubated with SiNP/CNT-DNA microgel SC50 according to an embodiment encoded with mKate gene fragments for different time periods. Note that the REF52 cells show the typical red fluorescence of expressed mKate protein after 5 days (day 6) of culturing.

The present invention is more specifically explained below by examples. However, the present invention is not limited to those examples.

*Materials*: Tetraethyl orthosilicate (TEOS), 3-(trihydroxysilyl)propyl methylphosphonate (THPMP, monosodium salt solution), (3-aminopropyl)trimethoxysilane (APTMS), polyethylene glycol) methyl ether maleimide (mPEG-mal, molecular weight ~ 2000), N1-(3-trimethoxysilylpropyl)diethylenetriamine (DETAPTMS), 5,5'-dithiobis(2-nitrobenzoic acid) (DTNB, Ellman's reagent), tris(2-carboxyethyl)phosphine hydrochloride (TCEP, 0.5 M in water), sodium cyanoborohydride, single-walled carbon nanotubes (CNT, 0.83 nm average diameter, 1 $\mu$m median length), oligonucleotides, G418 disulfate salt solution (50 mg/mL in water), doxorubicin (DOX), and Cell Counting Kit-8 (CCK-8) were purchased from Sigma-Aldrich. Cyclohexane, 1-hexanol, hydrogen fluoride (HF), and glutaradehyde (50% in water) were from VWR. Fluorescamine and (3-mercaptopropyl) trimethoxysilane (MPTMS) were purchased from Alfa Aesar. T4 DNA ligase (400,000 units/ml), phi29 DNA polymerase (10,000 units/ml), restriction enzymes (BstEll-HF and SexAl), and deoxynucleotide (dNTP) solution mix (10 mM for each nucleotide) were from New England Biolabs. Sulfo-cyanine 5 NHS-ester was from Lumiprobe. Triton X-100, 4',6-diamidino-2-phenylindole (DAPI), and glycine were from AppliChem. Trypsin/EDTA solution (0.25%/0.02%) and fetal calf serum (FCS) were from Biochrom. Eagle's minimum essential medium (EMEM), Dulbecco's modified Eagle's medium (DMEM), and L-glutamine were from Gibco Laboratories. Fluorescein-12-dUTP solution (FITC-dUTP, 1 mM), penicillin/streptomycin, ethidium bromide (EtBr, 10 mg/mL in water), SYBR™ Gold Nucleic Acid Gel Stain (10,000× Concentrate in water), Alexa Fluor 488 phalloidin, and Dulbecco's phosphate buffered saline (DPBS) for cell experiments were from Thermo Fisher Scientific. Paraformaldehyde (PFA, 16% aqueous solution) was from Polysciences. 1,2-dioleoyl-3-trimethylammonium-propane (DOTAP) (chloride salt) and 1,2-dimyristoyl-sn-glycero-3-phosphoethanolamine-N-(lissamine rhodamine B sulfonyl) (Rh-PE) (ammonium salt) were from Avanti Polar Lipids. Mineral oil was from Carl Roth. All chemicals were used as received without further purification.

*Absorbance and fluorescence spectroscopy*: Absorption spectra were measured by an Agilent Cary 100 UV-Vis spectrophotometer or a BioTek Synergy microplate reader. The latter was used for the measurement of fluorescence spectra as well.

*Transmission electron microscopy (TEM), scanning transmission electron microscopy (STEM), energy dispersive X-ray spectroscopy (EDS) analysis*: Particle size and morphology of SiNP were investigated by FEI Titan[3] 80-300 electron microscope (FEI Company) at an accelerating voltage of 200 kV and the obtained images were analyzed by imaged (http://www.rsb.info.nih.gov/ij/download.html). Further, STEM and EDS analysis were performed at a voltage of 200 kV, equipped with a high-angle annular dark-field (HAADF) detector (Fischione Company) and a high solid-angle silicon drift detector system (FEI Company). The samples were prepared by placing a drop of the sample solution on 200-mesh carbon-coated Cu grids (Plano GmbH) and air-dried at room temperature.

*Dynamic light scattering (DLS) measurements*: The kinetic hydrodynamic size of SiNP during RCA reaction was studied by DLS (Malvern Zetasizer Nano ZSP) coupled with a standard 633 nm laser at 30 °C.

*Scanning electron microscopy (SEM) analysis*: After lyophilization, the hydrogels were coated with 4 nm platinum using ion beam deposition and their morphology was characterized by using a QUANTA 650-FEG scanning electron microscope from FEI Company, with an accelerating voltage of 5-10 kV.

*Raman analysis*: Raman analysis was performed using a Senterra Raman microscope (Bruker Optics, Ettlingen, Germany) equipped with a 532 nm laser operated at 5 mW output power. An Olympus MPLAN 100× objective, NA 0.9 (Olympus, Tokyo, Japan) was used for visualization of the samples, focusing of the excitation beam, and collimation of backscattered light as well. The spot size on the sample was 5 $\mu$m Ø. The measurement time was 15 s with three coadditions (3 × 5 s) for each spot. Samples were prepared by transferring a piece of hydrogel (~ 5 $\mu$L) onto a flat gold-coated Si wafer and air-drying before measurements.

*Multiple particle tracking (MPT) method*: The mechanical property of hydrogels was investigated *via* Multiple Particle Tracking (MPT) method. MPT experiments were performed using an inverted fluorescence microscope (Axio Observer D1, Zeiss), equipped with a Fluar 100x, N.A. 1.3, oil-immersion lens combined with a 1× optovar magnification changer. The Brownian motion of green fluorescent polystyrene microspheres of 0.2, 0.5, and 1.0 $\mu$m diameter (Bangs Laboratories) used as tracer particles were tracked. Before measurement, the tracer particles were trapped inside hydrogels by mixing of tracer particles with initial RCA reaction mixture. Images of these fluorescent beads were recorded onto a personal computer *via* a sCMOS camera Zyla X (Andor Technology: 21.8 mm diagonal sCMOS Sensor size, 2160 × 2160 square pixels). Displacements of particle centers were monitored in an 127 × 127 $\mu$m field of view, at a rate of 50 frames/s. Movies of the fluctuating microspheres were analyzed by a custom MPT routine incorporated into the software Image Processing System (Visiometrics iPS) and a self-written Matlab program (A. Kowalczyk, C. Oelschlaeger, N. Willenbacher, Polymer 2015, 58, 170) based on the widely used Crocker and Grier tracking algorithm.

*Atomic force microscopy (AFM) imaging*: The morphology of DNA-dispersed CNT (CNT-P) was observed by atomic force microscopy (AFM). Briefly, the sample was diluted up to 25 fold in TEMg (20 mM Tris base, 1 mM EDTA, 12.5 mM $MgCl_2$, pH 7.4). Then, 5 $\mu$l solution was deposited on a cleaved mica surface (Plano GmbH) and adsorbed for 3 min at room temperature. After addition of 10 $\mu$l TAEMg (40 mM Tris, 20 mM acetic acid, 2 mM EDTA, 12.5 mM Mg acetate, pH 8.0), the sample was scanned with sharpened pyramidal tips (SNL-10 tips 0.35 N/m, Bruker) in Tapping Mode with a MultiModeTM 8 microscope (Bruker) equipped with a Nanoscope V controller.

*Electrophoresis:* For both polyacrylamide gel electrophoresis (PAGE) and agarose gel electrophoresis tests, the samples were prepared at required temperature for certain duration to complete the reaction. The raw products without purification were loaded onto a 6% native polyacrylamide gel (1x TAE-Mg$^{2+}$) and run under a voltage of 120 V for 45 min then stained with SYBR Gold or EtBr. Likewise, the raw products without purification were loaded onto a 2.5% agarose gel (1$\times$ TAE-Mg$^{2+}$) and run under a voltage of 120 V for 45 min then stained with SYBR Gold or 20 min then stained with EtBr.

DNA sequences:

| H1 | 5'-GAT CGC GAT CCT GGC TCC TGT GAT TGT GCT CTA GAC ATC GCT AGA GCA CAA TCA CAG G-3' |
|---|---|
| H2 | 5'-CTA GAG CAC AAT CAC AGG AGC CAG TTT TCC TGT GAT TGT GCT CTA GCG ATG T-3' |
| alh | 5'-[AmC12]-TTT TTT TTT TTT TTT TTT TTC ATC TCA GTC TAG GAT CGC GCG TG-3' |
| h1 | 5'-AGT CTA GGA TTC GGC GTG ACG ACT TTC ACG CCG AAT CCT AGA CTG AGA TG-3' |
| h2-I | 5'-ACA TCG CTA GAG CAC AAT CAC AGG TTA GTC GTC ACG CCG AAT CCT AGA CTT TCA TCT CAG TCT AGG ATT CGG CGT G-3' |
| I | ACA TCG CTA GAG CAC AAT CAC AGG |
| aP1 (primer for SiNP modification) | 5'-[AmC12]-TTT TTT TTT TTT TCT AAC TGC TGC GCC GCC GGG AAA ATA CTG TAC GGT TAG A-3' |
| P2 (primer for CNT modification) | 5'- TTT TTT TTT TTT TTT TTT TTT TTT TTT TTT TTT TTT TTT TTC TAA CTG CTG CGC CGC CGG GAA AAT ACT GTA CGG TTA GA-3' |
| Sgc8 | 5'-ATC TAA CTG CTG CGC CGC CGG GAA AAT ACT GTA CGG TTA GA-3' |
| T (template for RCA) | 5'-[Phos]TTC CCG GCG GCG CAG CAG TTA GAT GCT GCT GCA GCG ATA CGC GTA TCG CTA TGG GTA ACC GTA CGG TTA CCC GCA GCA GCA TCT AAC CGT ACA GTA TT-3' |

(continued)

| Th (template for simultaneous RCA and HCR) | 5'-[Phos]TTCCCGGCGGCGCAGCAGTTAGATGCTGCTGCAGCGAACATCGCTAGAGCACAATCACAGGTACGATATGCCGCAGCAGCATCTAACCGTACAGTATT-3' |
|---|---|
| Forward primer (primer for PCR on mKate-producing plasmid) | 5'-CGT ACG ATA TGC CAT AGC GAT ACG CGG CGG TTT GCG TAT TGG GCG CTC TTC C-3' |
| Reverse primer (primer for PCR on mKate-producing plasmid) | 5'-CTC TCC CCG CGC GTT GGC CG-3' |

(continued)

| pcDNA-EXP40-mKate (plasmid for PCR) | 5'- GACGGATCGGGAGATCTCCCGATCCCCTATGGTGCACTCTC AGTACAATCTGCTCTGATGCCGCATAGTTAAGCCAGTATCTG CTCCCTGCTTGTGTGTTGGAGGTCGCTGAGTAGTGCGCGAG CAAAATTTAAGCTACAACAAGGCAAGGCTTGACCGACAATTG CATGAAGAATCTGCTTAGGGTTAGGCGTTTTGCGCTGCTTCG CGATGTACGGGCCAGATATACGCGTTGACATTGATTATTGAC TAGTTATTAATAGTAATCAATTACGGGGTCATTAGTTCATAGC CCATATATGGAGTTCCGCGTTACATAACTTACGGTAAATGGC CCGCCTGGCTGACCGCCCAACGACCCCCGCCCATTGACGTC AATAATGACGTATGTTCCCATAGTAACGCCAATAGGGACTTT CCATTGACGTCAATGGGTGGAGTATTTACGGTAAACTGCCCA CTTGGCAGTACATCAAGTGTATCATATGCCAAGTACGCCCCC TATTGACGTCAATACGGTAAATGGCCCGCCTGGCATTATGCC CAGTACATGACCTTATGGGACTTTCCTACTTGGCAGTACATC TACGTATTAGTCATCGCTATTACCATGGTGATGCGGTTTTGG CAGTACATCAATGGGCGTGGATAGCGGTTTGACTCACGGGG ATTTCCAAGTCTCCACCCCATTGACGTCAATGGGAGTTTGTT TTGGCACCAAAATCACGGGACTTTCCAAAATGTCGTAACAAC TCCGCCCCATTGACGCAAATGGGCGGTAGGCGTGTACGGTG GGAGGTCTATATAAGCAGAGCTCTCTGGCTAACTAGAGAACC CACTGCTTACTGGCTTATCGAAATTAATACGACTCACTATAGG GAGACCCAAGCTGGCTAGTTAAGCTATCAACAAGTTTGTACA AAAAGCAGGCTCTAAGGAGGATAGAACCATGGTGTCTAAGG GCGAAGAGCTGATTAAGGAGAACATGCACATGAAGCTGTAC ATGGAGGGCACCGTGAACAACCACCACTTCAAGTGCACATC CGAGGGCGAAGGCAAGCCCTACGAGGGCACCCAGACCATG AGAATCAAGGTGGTCGAGGGCGGCCCTCTCCCCTTCGCCTT |

CGACATCCTGGCTACCAGCTTCATGTACGGCAGCAAAACCTT
CATCAACCACACCCAGGCATCCCCGACTTCTTTAAGCAGTCC
TTCCCTGAGGGCTTCACATGGGAGAGAGTCACCACATACGA
AGACGGGGGCGTGCTGACCGCTACCCAGGACACCAGCCTC
CAGGACGGCTGCCTCTCTACAACGTCAAGATCAGAGGGGTG
AACTTCCCATCCAACGGCCCTGTGATGCAGAAGAAAACACTC
GGCTGGGAGGCCTCCACCGAGATGCTGTACCCCGCTGACG
GCGGCCTGGAAGGCAGAAGCGACATGGCCCTGAAGCTCGT
GGGCGGGGGCCACCTGATCTGCAACTTGAAGACCACATACA
GATCCAAGAAACCCGCTAAGAACCTCAAGATGCCCGGCGTC
TACTATGTGGACAGAAGACTGGAAAGAATCAAGGAGGCCGA
CAAAGAGACCTACGTCGAGCAGCACGAGGTGGCTGTGGCCA
GATACTGCGACCTCCCTAGCAAACTGGGGCACAAACTTAATT
ACCCAGCTTTCTTGTACAAAGTGGTTGATCTAGAGGGCCCGC
GGTTCGAAGGTAAGCCTATCCCTAACCCTCTCCTCGGTCTCG
ATTCTACGCGTACCGGTCATCATCACCATCACCATTGAGTTT
AAACCCGCTGATCAGCCTCGACTGTGCCTTCTAGTTGCCAGC
CATCTGTTGTTTGCCCCTCCCCCGTGCCTTCCTTGACCCTGG
AAGGTGCCACTCCCACTGTCCTTCCTAATAAAATGAGGAAAT
TGCATCGCATTGTCTGAGTAGGTGTCATTCTATTCTGGGGGG
TGGGGTGGGGCAGGACAGCAAGGGGGAGGATTGGGAAGAC
AATAGCAGGCATGCTGGGGATGCGGTGGGCTCTATGGCTTC
TGAGGCGGAAAGAACCAGCTGGGGCTCTAGGGGGTATCCC
CACGCGCCCTGTAGCGGCGCATTAAGCGCGGCGGGTGTGG
TGGTTACGCGCAGCGTGACCGCTACACTTGCCAGCGCCCTA
GCGCCCGCTCCTTTCGCTTTCTTCCCTTCCTTTCTCGCCACG
TTCGCCGGCTTTCCCCGTCAAGCTCTAAATCGGGGGCTCCC
TTTAGGGTTCCGATTTAGTGCTTTACGGCACCTCGACCCCAA
AAAACTTGATTAGGGTGATGGTTCACGTAGTGGGCCATCGCC
CTGATAGACGGTTTTTCGCCCTTTGACGTTGGAGTCCACGTT
CTTTAATAGTGGACTCTTGTTCCAAACTGGAACAACACTCAAC
CCTATCTCGGTCTATTCTTTTGATTTATAAGGGATTTTGCCGA
TTTCGGCCTATTGGTTAAAAAATGAGCTGATTTAACAAAAATT
TAACGCGAATTAATTCTGTGGAATGTGTGTCAGTTAGGGTGT
GGAAAGTCCCCAGGCTCCCCAGCAGGCAGAAGTATGCAAAG

(continued)

CATGCATCTCAATTAGTCAGCAACCAGGTGTGGAAAGTCCCC
AGGCTCCCCAGCAGGCAGAAGTATGCAAAGCATGCATCTCA
ATTAGTCAGCAACCATAGTCCCGCCCCTAACTCCGCCCATCC
CGCCCCTAACTCCGCCCAGTTCCGCCCATTCTCCGCCCCAT
GGCTGACTAATTTTTTTTATTTATGCAGAGGCCGAGGCCGCC
TCTGCCTCTGAGCTATTCCAGAAGTAGTGAGGAGGCTTTTTT
GGAGGCCTAGGCTTTTGCAAAAGCTCCCGGGAGCTTGTAT
ATCCATTTTCGGATCTGATCAAGAGACAGGATGAGGATCGTT
TCGCATGATTGAACAAGATGGATTGCACGCAGGTTCTCCGG
CCGCTTGGGTGGAGAGGCTATTCGGCTATGACTGGGCACAA
CAGACAATCGGCTGCTCTGATGCCGCCGTGTTCCGGCTGTC
AGCGCAGGGGCGCCCGGTTCTTTTTGTCAAGACCGACCTGT
CCGGTGCCCTGAATGAACTGCAGGACGAGGCAGCGCGGCT
ATCGTGGCTGGCCACGACGGGCGTTCCTTGCGCAGCTGTGC
TCGACGTTGTCACTGAAGCGGGAAGGGACTGGCTGCTATTG
GGCGAAGTGCCGGGGCAGGATCTCCTGTCATCTCACCTTGC
TCCTGCCGAGAAAGTATCCATCATGGCTGATGCAATGCGGC
GGCTGCATACGCTTGATCCGGCTACCTGCCCATTCGACCAC
CAAGCGAAACATCGCATCGAGCGAGCACGTACTCGGATGGA
AGCCGGTCTTGTCGATCAGGATGATCTGGACGAAGAGCATC
AGGGGCTCGCGCCAGCCGAACTGTTCGCCAGGCTCAAGGC
GCGCATGCCCGACGGCGAGGATCTCGTCGTGACCCATGGC
GATGCCTGCTTGCCGAATATCATGGTGGAAAATGGCCGCTTT
TCTGGATTCATCGACTGTGGCCGGCTGGGTGTGGCGGACCG
CTATCAGGACATAGCGTTGGCTACCCGTGATATTGCTGAAGA
GCTTGGCGGCGAATGGGCTGACCGCTTCCTCGTGCTTTACG
GTATCGCCGCTCCCGATTCGCAGCGCATCGCCTTCTATCGC
CTTCTTGACGAGTTCTTCTGAGCGGGCTCTGGGGTTCGCGA
AATGACCGACCAAGCGACGCCCAACCTGCCATCACGAGATT
TCGATTCCACCGCCGCCTTCTATGAAAGGTTGGGCTTCGGAA
TCGTTTTCCGGGACGCCGGCTGGATGATCCTCCAGCGCGGG
GATCTCATGCTGGAGTTCTTCGCCCACCCCAACTTGTTTATT
GCAGCTTATAATGGTTACAAATAAAGCAATAGCATCACAAATT
TCACAAATAAAGCATTTTTTTCACTGCATTCTAGTTGTGGTTT
GTCCAAACTCATCAATGTATCTTATCATGTCTGTATACCGTCG

(continued)

ACCTCTAGCTAGAGCTTGGCGTAATCATGGTCATAGCTGTTT
CCTGTGTGAAATTGTTATCCGCTCACAATTCCACACAACATAC
GAGCCGGAAGCATAAAGTGTAAAGCCTGGGGTGCCTAATGA
GTGAGCTAACTCACATTAATTGCGTTGCGCTCACTGCCCGCT
TTCCAGTCGGGAAACCTGTCGTGCCAGCTGCATTAATGAATC
GGCCAACGCGCGGGGAGAGGCGGTTTGCGTATTGGGCGCT
CTTCCGCTTCCTCGCTCACTGACTCGCTGCGCTCGGTCGTTC
GGCTGCGGCGAGCGGTATCAGCTCACTCAAAGGCGGTAATA
CGGTTATCCACAGAATCAGGGGATAACGCAGGAAAGAACAT
GTGAGCAAAAGGCCAGCAAAAGGCCAGGAACCGTAAAAAGG
CCGCGTTGCTGGCGTTTTTCCATAGGCTCCGCCCCCCTGAC
GAGCATCACAAAAATCGACGCTCAAGTCAGAGGTGGCGAAA
CCCGACAGGACTATAAAGATACCAGGCGTTTCCCCCTGGAA
GCTCCCTCGTGCGCTCTCCTGTTCCGACCCTGCCGCTTACC
GGATACCTGTCCGCCTTTCTCCCTTCGGGAAGCGTGGCGCT
TTCTCATAGCTCACGCTGTAGGTATCTCAGTTCGGTGTAGGT
CGTTCGCTCCAAGCTGGGCTGTGTGCACGAACCCCCCGTTC
AGCCCGACCGCTGCGCCTTATCCGGTAACTATCGTCTTGAGT
CCAACCCGGTAAGACACGACTTATCGCCACTGGCAGCAGCC
ACTGGTAACAGGATTAGCAGAGCGAGGTATGTAGGCGGTGC
TACAGAGTTCTTGAAGTGGTGGCCTAACTACGGCTACACTAG
AAGAACAGTATTTGGTATCTGCGCTCTGCTGAAGCCAGTTAC
CTTCGGAAAAAGAGTTGGTAGCTCTTGATCCGGCAAACAAAC
CACCGCTGGTAGCGGTGGTTTTTTTGTTTGCAAGCAGCAGAT
TACGCGCAGAAAAAAAGGATCTCAAGAAGATCCTTTGATCTT
TTCTACGGGGTCTGACGCTCAGTGGAACGAAAACTCACGTTA
AGGGATTTTGGTCATGAGATTATCAAAAAGGATCTTCACCTA
GATCCTTTTAAATTAAAAATGAAGTTTTAAATCAATCTAAAGTA
TATATGAGTAAACTTGGTCTGACAGTTACCAATGCTTAATCAG
TGAGGCACCTATCTCAGCGATCTGTCTATTTCGTTCATCCATA
GTTGCCTGACTCCCCGTCGTGTAGATAACTACGATACGGGA
GGGCTTACCATCTGGCCCCAGTGCTGCAATGATACCGCGAG
ACCCACGCTCACCGGCTCCAGATTTATCAGCAATAAACCAGC
CAGCCGGAAGGGCCGAGCGCAGAAGTGGTCCTGCAACTTTA
TCCGCCTCCATCCAGTCTATTAATTGTTGCCGGGAAGCTAGA

(continued)

| | |
|---|---|
| | GTAAGTAGTTCGCCAGTTAATAGTTTGCGCAACGTTGTTGCC<br>ATTGCTACAGGCATCGTGGTGTCACGCTCGTCGTTTGGTATG<br>GCTTCATTCAGCTCCGGTTCCCAACGATCAAGGCGAGTTACA<br>TGATCCCCCATGTTGTGCAAAAAAGCGGTTAGCTCCTTCGGT<br>CCTCCGATCGTTGTCAGAAGTAAGTTGGCCGCAGTGTTATCA<br>CTCATGGTTATGGCAGCACTGCATAATTCTCTTACTGTCATG<br>CCATCCGTAAGATGCTTTTCTGTGACTGGTGAGTACTCAACC<br>AAGTCATTCTGAGAATAGTGTATGCGGCGACCGAGTTGCTCT<br>TGCCCGGCGTCAATACGGGATAATACCGCGCCACATAGCAG<br>AACTTTAAAAGTGCTCATCATTGGAAAACGTTCTTCGGGGCG<br>AAAACTCTCAAGGATCTTACCGCTGTTGAGATCCAGTTCGAT<br>GTAACCCACTCGTGCACCCAACTGATCTTCAGCATCTTTTAC<br>TTTCACCAGCGTTTCTGGGTGAGCAAAAACAGGAAGGCAAAA<br>TGCCGCAAAAAAGGGAATAAGGGCGACACGGAAATGTTGAA<br>TACTCATACTCTTCCTTTTTCAATATTATTGAAGCATTTATCAG<br>GGTTATTGTCTCATGAGCGGATACATATTTGAATGTATTTAGA<br>AAAATAAACAAATAGGGGTTCCGCGCACATTTCCCCGAAAAG<br>TGCCACCTGACGTC-3' |
| Forward primer (primer for PCR for PCR on eGFP-producing plasmid | 5'-CGCGGCGGCCCTTTTATGACGGACAGGTATCCGGTAAGCG-3' |
| Reverse primer (primer for PCR for PCR on eGFP-producing plasmid | 5'-GGCGGTGCTACAGAGTTCTT-3' |
| pET-eGFP (plasmid for PCR) | GCTGAGTTGGCTGCTGCCACCGCTGAGCAATAACTAGC<br>ATAACCCCTTGGGGCCTCTAAACGGGTCTTGAGGGGGTT<br>TTTTGCTGAAAGGAGGAACTATATCCGGATTGGCGAAT<br>GGGACGCGCCCTGTAGCGGCGCATTAAGCGCGGCGG<br>GTGTGGTGGTTACGCGCAGCGTGACCGCTACACTTGC<br>CAGCGCCCTAGCGCCCGCTCCTTTCGCTTTCTTCCCTT<br>CCTTTCTCGCCACGTTCGCCGGCTTTCCCCGTCAAGCT<br>CTAAATCGGGGGCTCCCTTTAGGGTTCCGATTTAGTGC |

(continued)

TTTACGGCACCTCGACCCCAAAAAACTTGATTAGGGTG
ATGGTTCACGTAGTGGGCCATCGCCCTGATAGACGGTT
TTTCGCCCTTTGACGTTGGAGTCCACGTTCTTTAATAGT
GGACTCTTGTTCCAAACTGGAACAACACTCAACCCTATC
TCGGTCTATTCTTTTGATTTATAAGGGATTTTGCCGATTT
CGGCCTATTGGTTAAAAAATGAGCTGATTTAACAAAAT
TTAACGCGAATTTTAACAAATATTAACGTTTACAATTTC
AGGTGGCACTTTCGGGGAAATGTGCGCGGAACCCCTA
TTTGTTTATTTTTCTAAATACATTCAAATATGTATCCGCT
CATGAGACAATAACCCTGATAAATGCTTCAATAATATTG
AAAAAGGAAGAGTATGAGTATTCAACATTTCCGTGTCGC
CCTTATTCCCTTTTTTGCGGCATTTTGCCTTCCTGTTTTT
GCTCACCCAGAAACGCTGGTGAAAGTAAAAGATGCTGA
AGATCAGTTGGGTGCACGAGTGGGTTACATCGAACTGG
ATCTCAACAGCGGTAAGATCCTTGAGAGTTTTCGCCCC
GAAGAACGTTTTCCAATGATGAGCACTTTTAAAGTTCTG
CTATGTGGCGCGGTATTATCCCGTATTGACGCCGGGCA
AGAGCAACTCGGTCGCCGCATACACTATTCTCAGAATG
ACTTGGTTGAGTACTCACCAGTCACAGAAAAGCATCTTA
CGGATGGCATGACAGTAAGAGAATTATGCAGTGCTGCC
ATAACCATGAGTGATAACACTGCGGCCAACTTACTTCTG
ACAACGATCGGAGGACCGAAGGAGCTAACCGCTTTTTT
GCACAACATGGGGGATCATGTAACTCGCCTTGATCGTT
GGGAACCGGAGCTGAATGAAGCCATACCAAACGACGA
GCGTGACACCACGATGCCTGCAGCAATGGCAACAACGT
TGCGCAAACTATTAACTGGCGAACTACTTACTCTAGCTT
CCCGGCAACAATTAATAGACTGGATGGAGGCGGATAAA
GTTGCAGGACCACTTCTGCGCTCGGCCCTTCCGGCTG
GCTGGTTTATTGCTGATAAATCTGGAGCCGGTGAGCGT
GGGTCTCGCGGTATCATTGCAGCACTGGGGCCAGATG
GTAAGCCCTCCCGTATCGTAGTTATCTACACGACGGGG
AGTCAGGCAACTATGGATGAACGAAATAGACAGATCGC

TGAGATAGGTGCCTCACTGATTAAGCATTGGTAACTGTC
AGACCAAGTTTACTCATATATACTTTAGATTGATTTAAAA
CTTCATTTTTAATTTAAAAGGATCTAGGTGAAGATCCTTT
TTGATAATCTCATGACCAAAATCCCTTAACGTGAGTTTT
CGTTCCACTGAGCGTCAGACCCCGTAGAAAAGATCAAA
GGATCTTCTTGAGATCCTTTTTTTCTGCGCGTAATCTGC
TGCTTGCAAACAAAAAAACCACCGCTACCAGCGGTGGT
TTGTTTGCCGGATCAAGAGCTACCAACTCTTTTTCCGAA
GGTAACTGGCTTCAGCAGAGCGCAGATACCAAATACTG
TCCTTCTAGTGTAGCCGTAGTTAGGCCACCACTTCAAG
AACTCTGTAGCACCGCCTACATACCTCGCTCTGCTAATC
CTGTTACCAGTGGCTGCTGCCAGTGGCGATAAGTCGTG
TCTTACCGGGTTGGACTCAAGACGATAGTTACCGGATA
AGGCGCAGCGGTCGGGCTGAACGGGGGGTTCGTGCA
CACAGCCCAGCTTGGAGCGAACGACCTACACCGAACT
GAGATACCTACAGCGTGAGCTATGAGAAAGCGCCACGC
TTCCCGAAGGGAGAAAGGCGGACAGGTATCCGGTAAG
CGGCAGGGTCGGAACAGGAGAGCGCACGAGGGAGCTT
CCAGGGGGAAACGCCTGGTATCTTTATAGTCCTGTCGG
GTTTCGCCACCTCTGACTTGAGCGTCGATTTTTGTGATG
CTCGTCAGGGGGGCGGAGCCTATGGAAAAACGCCAGC
AACGCGGCCTTTTTACGGTTCCTGGCCTTTTGCTGGCC
TTTTGCTCACATGTTCTTTCCTGCGTTATCCCCTGATTCT
GTGGATAACCGTATTACCGCCTTTGAGTGAGCTGATAC
CGCTCGCCGCAGCCGAACGACCGAGCGCAGCGAGTCA
GTGAGCGAGGAAGCGGAAGAGCGCCTGATGCGGTATT
TTCTCCTTACGCATCTGTGCGGTATTTCACACCGCATAT
ATGGTGCACTCTCAGTACAATCTGCTCTGATGCCGCAT
AGTTAAGCCAGTATACACTCCGCTATCGCTACGTGACT
GGGTCATGGCTGCGCCCCGACACCCGCCAACACCCGC
TGACGCGCCCTGACGGGCTTGTCTGCTCCCGGCATCC
GCTTACAGACAAGCTGTGACCGTCTCCGGGAGCTGCAT

(continued)

GTGTCAGAGGTTTTCACCGTCATCACCGAAACGCGCGA
GGCAGCTGCGGTAAAGCTCATCAGCGTGGTCGTGAAG
CGATTCACAGATGTCTGCCTGTTCATCCGCGTCCAGCT
CGTTGAGTTTCTCCAGAAGCGTTAATGTCTGGCTTCTGA
TAAAGCGGGCCATGTTAAGGGCGGTTTTTTCCTGTTTG
GTCACTGATGCCTCCGTGTAAGGGGGATTTCTGTTCAT
GGGGGTAATGATACCGATGAAACGAGAGAGGATGCTCA
CGATACGGGTTACTGATGATGAACATGCCCGGTTACTG
GAACGTTGTGAGGGTAAACAACTGGCGGTATGGATGCG
GCGGGACCAGAGAAAAATCACTCAGGGTCAATGCCAG
CGCTTCGTTAATACAGATGTAGGTGTTCCACAGGGTAG
CCAGCAGCATCCTGCGATGCAGATCCGGAACATAATGG
TGCAGGGCGCTGACTTCCGCGTTTCCAGACTTTACGAA
ACACGGAAACCGAAGACCATTCATGTTGTTGCTCAGGT
CGCAGACGTTTTGCAGCAGCAGTCGCTTCACGTTCGCT
CGCGTATCGGTGATTCATTCTGCTAACCAGTAAGGCAA
CCCCGCCAGCCTAGCCGGGTCCTCAACGACAGGAGCA
CGATCATGCGCACCCGTGGGGCCGCCATGCCGGCGAT
AATGGCCTGCTTCTCGCCGAAACGTTTGGTGGCGGGAC
CAGTGACGAAGGCTTGAGCGAGGGCGTGCAAGATTCC
GAATACCGCAAGCGACAGGCCGATCATCGTCGCGCTC
CAGCGAAAGCGGTCCTCGCCGAAAATGACCCAGAGCG
CTGCCGGCACCTGTCCTACGAGTTGCATGATAAAGAAG
ACAGTCATAAGTGCGGCGACGATAGTCATGCCCCGCG
CCCACCGGAAGGAGCTGACTGGGTTGAAGGCTCTCAA
GGGCATCGGTCGAGATCCCGGTGCCTAATGAGTGAGC
TAACTTACATTAATTGCGTTGCGCTCACTGCCCGCTTTC
CAGTCGGGAAACCTGTCGTGCCAGCTGCATTAATGAAT
CGGCCAACGCGCGGGGAGAGGCGGTTTGCGTATTGGG
CGCCAGGGTGGTTTTTCTTTTCACCAGTGAGACGGGCA
ACAGCTGATTGCCCTTCACCGCCTGGCCCTGAGAGAGT
TGCAGCAAGCGGTCCACGCTGGTTTGCCCCAGCAGGC

(continued)

```
GAAAATCCTGTTTGATGGTGGTTAACGGCGGGATATAA
CATGAGCTGTCTTCGGTATCGTCGTATCCCACTACCGA
GATATCCGCACCAACGCGCAGCCCGGACTCGGTAATG
GCGCGCATTGCGCCCAGCGCCATCTGATCGTTGGCAA
CCAGCATCGCAGTGGGAACGATGCCCTCATTCAGCATT
TGCATGGTTTGTTGAAAACCGGACATGGCACTCCAGTC
GCCTTCCCGTTCCGCTATCGGCTGAATTTGATTGCGAG
TGAGATATTTATGCCAGCCAGCCAGACGCAGACGCGCC
GAGACAGAACTTAATGGGCCCGCTAACAGCGCGATTTG
CTGGTGACCCAATGCGACCAGATGCTCCACGCCCAGTC
GCGTACCGTCTTCATGGGAGAAAATAATACTGTTGATG
GGTGTCTGGTCAGAGACATCAAGAAATAACGCCGGAAC
ATTAGTGCAGGCAGCTTCCACAGCAATGGCATCCTGGT
CATCCAGCGGATAGTTAATGATCAGCCCACTGACGCGT
TGCGCGAGAAGATTGTGCACCGCCGCTTTACAGGCTTC
GACGCCGCTTCGTTCTACCATCGACACCACCACGCTGG
CACCCAGTTGATCGGCGCGAGATTTAATCGCCGCGACA
ATTTGCGACGGCGCGTGCAGGGCCAGACTGGAGGTGG
CAACGCCAATCAGCAACGACTGTTTGCCCGCCAGTTGT
TGTGCCACGCGGTTGGGAATGTAATTCAGCTCCGCCAT
CGCCGCTTCCACTTTTTCCCGCGTTTTCGCAGAAACGT
GGCTGGCCTGGTTCACCACGCGGGAAACGGTCTGATA
AGAGACACCGGCATACTCTGCGACATCGTATAACGTTA
CTGGTTTCACATTCACCACCCTGAATTGACTCTCTTCCG
GGCGCTATCATGCCATACCGCGAAAGGTTTTGCGCCAT
TCGATGGTGTCCGGGATCTCGACGCTCTCCCTTATGCG
ACTCCTGCATTAGGAAGCAGCCCAGTAGTAGGTTGAGG
CCGTTGAGCACCGCCGCCGCAAGGAATGGTGCATGCA
AGGAGATGGCGCCCAACAGTCCCCCGGCCACGGGGCC
TGCCACCATACCCACGCCGAAACAAGCGCTCATGAGCC
CGAAGTGGCGAGCCCGATCTTCCCCATCGGTGATGTC
GGCGATATAGGCGCCAGCAACCGCACCTGTGGCGCCG
```

(continued)

| | GTGATGCCGGCCACGATGCGTCCGGCGTAGAGGATCG<br>AGATCTGATCCCGCGAAATTAATACGACTCACTATAGG<br>GGAATTGTGAGCGGATAACAATTCCCCTCTAGAAATAAT<br>TTTGTTTAACTTTAAGAAGGAGATATACATGGCACATATT<br>GTTATGGTTGATGCCTATAAACCGACCAAAGGTGGTGG<br>TGGCAGCGTTAGCAAAGGTGAAGAACTGTTTACCGGTG<br>TTGTTCCGATTCTGGTTGAACTGGATGGTGATGTTAATG<br>GCCACAAATTTTCAGTTAGCGGTGAAGGTGAGGGTGAT<br>GCAACCTATGGTAAACTGACCCTGAAATTTATCTGCACC<br>ACCGGCAAACTGCCGGTTCCGTGGCCGACCCTGGTTA<br>CCACCCTGACCTATGGTGTTCAGTGTTTTAGCCGTTATC<br>CGGATCATATGAAACAGCACGATTTTTTCAAAAGCGCAA<br>TGCCGGAAGGTTATGTTCAAGAACGTACCATCTTTTTCA<br>AAGACGACGGCAACTATAAACCCGTGCCGAAGTTAAA<br>TTTGAAGGTGATACCCTGGTGAATCGCATTGAACTGAAA<br>GGCATCGATTTTAAAGAAGATGGCAATATCCTGGGCCA<br>CAAACTGGAATATAATTATAATAGCCACAACGTGTACAT<br>CATGGCCGACAAACAGAAAAATGGCATCAAAGTGAACT<br>TCAAAATCCGCCACAATATTGAAGATGGCTCAGTTCAGC<br>TGGCAGATCATTATCAGCAGAATACCCCGATCGGTGAT<br>GGTCCGGTTCTGCTGCCGGATAATCATTATCTGAGCAC<br>CCAGAGCGCACTGAGCAAAGATCCGAATGAAAACGTG<br>ATCACATGGTGCTGCTGGAATTTGTTACCGCAGCAGGT<br>ATTACCCTGGGTATGGATGAACTGTATAAACATCATCAC<br>CACCATCATTAAGATCCGGCTGCTAACAAAGCCCGAAA<br>GGAA |

Note: "[AmC12]" and [Phos] represent "Amino-Modifier C12" and "Phosphate", respectively.

1. Rolling circle amplification (RCA)-mediated synthesis of silica nanoparticles (SiNP)-DNA hydrogel

1.1 Synthesis of ssDNA (P1)-modified SiNP

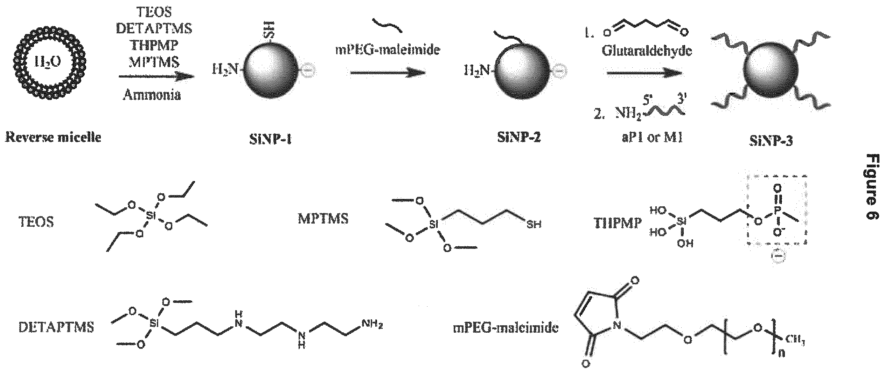

Multifunctional SiNP having an average particle size of 80 nm with amino, thiol and phosphonate groups were firstly synthesized. An embodiment thereof is shown in Figure 6. In particular, cyclohexane (38 mL), 1-hexanol (9 mL) and triton X-100 (9 mL) was mixed rigorously in a 250 mL round-bottom glass bottles. Then, double distilled water (2 mL) was added to the mixture to form stable reverse micelles. TEOS (500 $\mu$L) was introduced in the mixture after 10 min, and the hydrolysis and condensation of TEOS was catalyzed by adding ammonia solution (28-30 %, 500 $\mu$L). The mixture was stirred at room temperature for 24 h. Then an additional TEOS (250 $\mu$L) was added to the mixture and stirred for another 30 min, and subsequently THPMP (200 $\mu$L) and DETAPTMS (50 $\mu$L) were added to modify the surface of the nanoparticles with negatively charged phosphonate and amino groups. The mixture was allowed to react for 24 h, and MPTMS (30 $\mu$L) was added to modify the nanoparticle's surface with thiol groups. The mixture was stirred at room temperature for an additional 3 h. The micelles were broken with acetone, and the obtained nanoparticles were centrifuged and washed at least 5 times with absolute ethanol, and finally dispersed in PBS buffer (pH 7.4, 10 mM) with a concentration of 10 mg/mL. Then, TCEP (0.5 M solution, 8.0 $\mu$L) was added to 1 mL of SiNP PBS solution to cleave any disulfide bonds on the particle surface, followed by reaction with mPEG-mal (50 mg/mL, 10 $\mu$L) to fabricate PEGlyated SiNP. Afterwards, amino-modified ssDNA P1 (aP1) was chemically immobilized on the particle surface *via* glutaraldehyde chemistry. Specifically, PEGlyated SiNP (10 mg/mL, 1.0 mL) in PBS buffer were mixed with glutaraldehyde (50 % in water, 250 $\mu$L), and the mixture was stirred at room temperature for 1 h. The resulting nanoparticles were washed 3 times with PBS buffer to remove unreacted glutaraldehyde. The obtained nanoparticles were dispersed in PBS buffer (1.0 mL), and then aP1 (100 $\mu$M, 50 $\mu$L) was added to the particle suspension and incubated for 2 h at room temperature. Following, glycine (0.4 M, 1.0 mL) was added in the mixture to block unreacted aldehyde groups, followed by adding sodium cyanoborohydride (60 mM, 400 $\mu$L) to reduce the imide bond into a stable C-N bond.

### 1.2 Synthesis particle-primer-template complexes

After obtaining (P1)-modified SiNP (SiNP-P), ssDNA (T) was phosphorylated at its 5' end and used to hybridize with P1 primer on the particle. In brief, the template T (10 $\mu$M, 30 $\mu$L) and 10x T4 DNA ligation buffer (10 $\mu$L) were added into 60 $\mu$L (10 mg/mL) SiNP suspension and the mixture was incubated for 3 h at 25 °C. After adding 2.5 $\mu$L T4 DNA ligase, the mixture was further incubated for more than 3 h at 25 °C to ligate the nicked ends of the template, leading to the formation of particle-primer-template complexes.

### 1.3 Rolling circle amplification of primer on particle

The rolling circular amplification solution was firstly prepared through mixing dNTPs (10 mM, 10 $\mu$L), 10$\times$ BSA (5 $\mu$L), 10$\times$ phi29 DNA polymerase buffer (5 $\mu$L), and phi29 DNA polymerase (10,000 U/ml, 5 $\mu$L), then 51.25 $\mu$L of the above-prepared particle-primer-template complexes was subjected to RCA reaction solution for 48 h at 30 °C. After purification of the raw product by carefully replacing surrounding solution of hydrogel with distilled water for 5-7 times, SiNP-DNA hydrogel was collected and stored at 4 °C.

### 2. Hybridization chain reaction (HCR)-mediated synthesis of SiNP-DNA hydrogel

### 2.1 Synthesis of ssDNA (Ih)-modified SiNP

ssDNA (Ih)-modified SiNP (SiNP-Ih) were synthesized with the similar protocol in part 1.1 using amine-modified Ih (aIh) instead of using aP1.

### 2.2 Annealing procedure for constructing hairpin structures

To prepare DNA hairpins for HCR, H1, H2, h1 and h2-I were purchased, dissolved and annealed. To prepare DNA hairpins for the linear HCR process, the purchased strands (H1, H2, h1 or h2-I) were kept at 95 °C for five minutes and then quickly quenched with ice to 4 °C. A 1X TAE-Mg$^{2+}$ buffer (40 mM Tris, 20 mM acetic acid, 2 mM EDTA, 12.5 mM Mg$^{2+}$, pH=8.0) was used to dissolve H1 and H2 strands. A 5X SSC buffer (750 mM sodium chloride, 75 mM sodium citrate, pH=7.0) was used for h1 and h2-I strands.

### 2.3 HCR on SiNP-Ih

h1 (500 $\mu$M, 20 $\mu$L) and h2-I (500 $\mu$M, 20 $\mu$L) were successively added into 20 $\mu$L SiNP-Ih suspension (10 mg/mL) and the reaction mixture was incubated overnight under room temperature. After purification by centrifugation/resuspension in distilled water for 3-5 times, the excess h1 and h2-I were removed and the obtained particle (SiNP-I) suspension was concentrated to 10 $\mu$L. After the first hybridization chain reaction (HCR) process, the initiator on particle was changed from Ih to I and the number of initiator on particle was amplified. The formed SiNP-I can further trigger the second HCR process of H1 and H2. Thus, 10 $\mu$L SiNP-I suspension was thoroughly mixed with 10 $\mu$L H1 (2.25 mM) and 10 $\mu$L H2 (2.25 mM) solutions in 250-$\mu$L tube and the mixture was exposed to air and evaporated overnight under room temperature. Afterwards, 10 $\mu$L distilled water was replenished and the mixture solution was incubated for at least 36 h to obtain a SiNP-DNA hydrogel.

### 3. Formation of SiNP-DNA hydrogel in DOTAP droplet

#### 3.1 Preparation of RCA reaction solution and DOTAP-containing mineral oil

The protocol for preparation of the RCA reaction solution can be found in part 1.1 as described above. The only difference is that SiNP used here were doped with Cy5 by co-condensation and co-hydrolysis of tetraethoxysilane (TEOS) and Cy5-modified (3-aminopropyl)trimethylsilane (APTMS-Cy5) during particle synthesis. Notably, APTES-Cy5 was synthesized by co-incubation of sulfo-Cy5-NHS ester (1.28 pmol, in 1 mL DMSO) with APTMS at a molar ratio of 1:10 at room temperature overnight.

1,2-dioleoyl-3-trimethylammonium-propane (DOTAP)-containing mineral oil was prepared by ultrasonication of DOTAP in mineral oil in an ice bath for 90 min. For visualization of the droplet membrane, 1,2-dimyristoyl-sn-glycero-3-phosphoethanolamine-N-(lissamine rhodamine B sulfonyl) was also used during the preparation of DOTAP-containing mineral oil. The concentration of DOTAP and Rh-PE in mineral oil are 4 mM and 4 $\mu$M, respectively.

#### 3.2 RCA reaction inside the droplet

Mineral oil containing DOTAP and Rh-PE (continuous phase) and RCA reaction solution were pumped into a microfluidic droplet generator made of poly(methyl methacrylate) (PMMA) which uses a flow-focusing junction at the speeds of 20 $\mu$L/min (continuous phase) and 1 $\mu$L/min (reaction solution), respectively. The formed droplets were collected into 96-well plate and incubated at 30 °C.

To monitor the gelation process in droplets over time, fluorescence recovery after photobleaching (FRAP) experiment was performed using confocal laser scanning fluorescence microscopy (CLSM) following the protocol with slight modification. The excitation wavelengths are 561 nm for rhodamine B and 633 nm for Cy5, respectively. When bleaching both dyes, the parameters were set as: bleach radius 25 $\mu$m to 35 $\mu$m, bleach time 1 min, and laser power 100%. Then, the images of droplets were acquired before and after bleaching at defined time points.

### 4. Hybridization chain reaction (HCR)-reinforced SiNP-DNA hydrogel preparation

First of all, hairpin structures of H1 and H2 were formed by the annealing procedure, described in part 2.2. Herein, a 1X phi29 DNA polymerase buffer (5 mM Tris-HCl, 1 mM MgCl$_2$, 1 mM, (NH$_4$)$_2$SO$_4$, 0.4 mM DTT (pH 7.5 @ 25°C) was used to dissolve the DNA strands. To trigger the HCR during RCA reaction on SiNP-P, ssDNA Th was used as the template and 4 $\mu$L H1 (2.25 mM) and 4 $\mu$L H2 (2.25 mM) were mixed with RCA reaction mixture.

### 5. Formation of a composite material comprising SiNP, CNT and DNA hydrogel

CNT-P was prepared by adding CNT (0.6 mg) and P2 (100 $\mu$M, 172 $\mu$L) into a NaCl solution (0.14 $\mu$M, 428 $\mu$L), and ultrasonication of the mixture in an ice bath for 90 min. After that, the unsuspended CNT were precipitated by centrifugation (40 min, 16000g). The suspension of CNT-P was collected and further purified by filtration until no free DNA could be detected.

The processes of synthesis of SiNP/CNT-DNA composite hydrogels SCx are similar to those of RCA-mediated synthesis of SiNP-DNA hydrogel S100. The difference is that both CNT-P and SiNP-P at different SiNP/CNT mass ratios were used in the one-pot reaction. Following by the same processes, a mixture of P1 primer-modified SiNP and P2 primer-modified CNT was used to synthesize nanoparticle/nanotube-primer-template complexes, which was used for the synthesis of the composite material SiNP/CNT-DNA hydrogel. Various composite materials that contained only SiNP or CNT (denoted as S100 or C100, respectively) or ternary composite materials containing both SiNP and CNT, denoted as SCx, where x gives the mass ratio of SiNP-P:CNT-P, were prepared as indicated in Table 2 below.

### 6. Cell culture

Human MCF7$_{eGFP}$ breast cancer cells stably transfected to express the EGF receptor fused to enhanced green fluorescent protein (eGFP-EGFR) were obtained from Max-Planck Institute for Molecular Physiology (Dortmund). The cells were cultured in MCF7$_{eGFP}$ medium, composed by EMEM, with addition of 1% penicillin/streptomycin, 10% FCS and 0.6% G418 disulfate salt solution at 37 °C in a 5% CO$_2$ environment. The cells were washed twice with DPBS (-/-) (without calcium and magnesium) and treated with trypsin by adding 500 $\mu$L 0.25% Trypsin solution in PBS-EDTA (PBS with 0.02% EDTA) for 3 minutes. The trypsin activity was blocked by adding 9.5 mL of fresh MCF7$_{eGFP}$ medium. The cell concentration of the resulting suspension was determined by hemocytometer analysis. MCF7 cells without transfection and HeLa cervical cancer cells from ATCC were also used in this research and cultivated without G418 disulfate salt solution in EMEM with 1% penicillin/streptomycin, 10% FCS. REF52 cells (a rat fibroblast line) were maintained in DMEM supplemented with 1% penicillin/streptomycin, 10% FCS, and 2 mM L-glutamine.

## 7. Bioimaging and drug delivery

Aptamers were inserted into the elongated DNA after RCA reaction by using the rationally designed DNA templates and primers. To this end, the long DNA chain-capped SiNP (S100apt) with aptamers were formed with periodic DNA containing multiple copies of aptamers. To evaluate the feasibility of S100apt for bioimaging application, HeLa cells which express PTK7 receptors were seeded with an initial density of $1.2 \times 10^4$ cells/well into a 4-well $\mu$-slide (Ibidi) and allowed to adhere for 12 h, followed by the addition of fresh medium that contained S100apt (400 $\mu$g/mL SiNP-P) for 6 h of incubation. Prior to fluorescence microscope imaging, the cells were washed with DPBS for three times, fixed by addition of a PFA solution (4%, in DPBS) for 10 min, permeabilized with a triton X-100 solution (0.1%, in DPBS) for 2 min, treated with CAS-Block for 20 min to suppress unspecific binding of the cell staining reagents, stained with Alexa Fluor 488 phalloidin solution (160 nM, in DPBS) for 20 min to visualize the actin filament, and ultimately counterstained with DAPI-solution (1.4 $\mu$M, in DPBS) for 10 min to locate the cell nucleus. DPBS was used to wash the cells for 2-3 times during each step.

The capability of $S100_{48h}$ for loading anthracycline drug doxorubicine (DOX) was investigated by stoichiometric quenching of DOX in DNA. Briefly, DOX (50 $\mu$M) was incubated with $S100_{48h}$ (0-75 $\mu$L) dispersed in 200 $\mu$L DPBS at room temperature for 24 h, followed by centrifugation at 10,000 rpm for 15 min. The free DOX in the supernatant was isolated and quantified by measuring the emission of DOX at an excitation of 480 nm. The DOX loading amount into $S100_{48h}$ was calculated by subtracting DOX amount determined in supernatant from initially used DOX amount. After that, $1.2 \times 10^4$ HeLa cells/well were seeded into a 4-well $\mu$-slide (Ibidi) and allowed to adhere for 12 h, followed by the addition of fresh medium that contained free DOX or $S100_{48h}$-DOX (5 $\mu$M DOX equivalent) for 2 h of incubation.

## 8. Controlled cell migration inside the SCx

Cell behavior inside the various SCx materials synthesized in a 25 $\mu$L RCA reaction was analyzed by confocal microscope (LSM 880, Carl Zeiss) after seeding cells on the SCx. To this end, SCx with different SiNP-P/CNT-P concentration ratios were prepared in 96-well plates with standard tissue culture (STC) cover glass as bottom (MoBiTec GmbH). After careful washing of the formed hydrogels with DPBS (3-5 times) and medium (3-5 times), $6 \times 10^3$ MCF7$_{eGFP}$ cells per well with 200 $\mu$L medium were seeded on the top of hydrogels and allowed to settle down for 2 h. The cell behavior was monitored by confocal fluorescence microscopy using both Z stack and time lapse modes. After cultivation of cells for 24 h, a cell proliferation assay was carried using the CCK-8 assay as described above.

Since the cells were restrained from vertical migration inside the hydrogel matrix within the SCx when the CNT concentration was higher than 160 $\mu$g/mL (e.g., as in SC25), it is possible to release them from the hydrogels. To this end, template T was firstly used to prepare the mixture of nanoparticle/nanotube-primer-template complexes. Then, SC25 containing BstEll restriction sites was synthesized by RCA reaction and used for restraining and controlled release of cells after digestion of hydrogel using BstEll-HF with a final concentration of 2 U/mL. The cell behavior was monitored by confocal fluorescence microscopy using both Z stack and time lapse modes.

## 9. Cell proliferation on the top of the condensed SCx

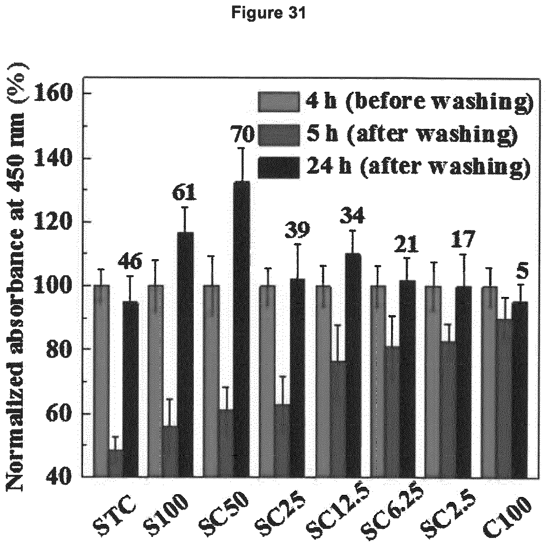

The various DNA-crosslinked SiNP and CNT composite materials were investigated for their ability to serve as coating that support cell spreading. To this end, various hydrogels of different SiNP/CNT concentration ratios were synthesized in 75 $\mu$L RCA reaction mixture in 96-plate wells with cover glass as bottom, purified by replacing the initial buffer with distilled water, and condensed by drying in vacuum (Eppendorf Concentrator plus, 20 mbar) to form the coatings. Afterwards, $6 \times 10^3$ MCF7$_{eGFP}$ or REF52 cells per well with 200 $\mu$L medium were seeded on the top of hydrogels and visualized by fluorescence microscopy. After cultivation of the cells for 48 h, a cell proliferation assay was carried using the aforementioned CCK-8 assay. To compare the adhesion strength of cells to different hydrogel surfaces, MCF7 or REF52 cells were incubated on hydrogel surfaces for 1 h and then washed with DPBS. The number of the remained cells was quantified by CCK-8 assay (see Figure 31).

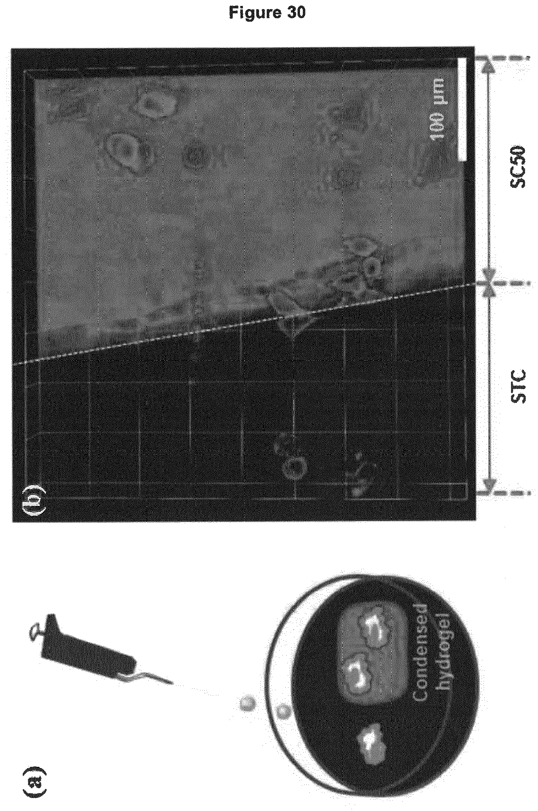

To compare the attraction of cells towards either the hydrogel surface or the STC surface, a sample of SC50 synthesized in 75 $\mu$L RCA reaction mixture was deposited and dried on petri dish with STC surface (Ibidi), thereby generating a patch of SC50 on the petri dish surface. Following, 1 mL medium containing $1.2 \times 10^4$ MCF7$_{eGFP}$ cells was transferred into a petri dish so that the hydrogel was fully covered by medium. After another 24 h of cultivation, adhered MCF7$_{eGFP}$ cells were visualized by fluorescence microscopy. Alternatively, for cell migration studies, 20 $\mu$L medium

containing $1.2\times10^4$ MCF7$_{eGFP}$ cells were transferred into the petri dish to form a droplet that was about 0.8 cm apart from the hydrogel. After 4 h incubation, the suspension cells were carefully removed by replacing the remaining medium with 1 mL fresh medium such that the entire surface of the petri dish was covered with medium. This enabled the adherent cells to migrate from the STC-surface to the SC50 surface where they proliferated (see Figure 30).

## 10. Cell release from SC25-coated microfluidic channels

Microfluidic channels ($\mu$-Slide I Luer) were purchased from Ibidi. The channel length, width, and height are 50 mm, 5 mm, and 400 $\mu$m, respectively. To prepare a thin SC25 film inside the microfluidic channel, 100 $\mu$L RCA reaction mixture was pipetted into the channel and then the inlet and outlet of the channel were tightly sealed. Following to incubation at 30 °C for 48 h, SC25-coated microchannel was carefully washed with distilled water and evaporated overnight in a sterile environment under normal pressure at room temperature. For fluidic cell experiments, the chip was connected with tubing to a peristaltic pump (IPC, Ismatec) and flushed with sterile DPBS for 30 min at a flow rate of 800 $\mu$L/min. Subsequently, a MCF7$_{eGFP}$ cell suspension ($5\times10^5$ cells/mL cell culture medium) was transfused through the microfluidic channel with a flow rate of 57.4 $\mu$L/min for 5 min. The flow was stopped for 2 h to allow the cells to settle and adhere to the SC25-coated surface. Then, medium without cells was transfused through the channel at a flow rate of 57.4 $\mu$L/min for 2.5 h. This process was monitored by fluorescence microscopy capturing images of the MCF7$_{eGFP}$ cells every 10 min. Following, the channel was transfused with the above specified medium containing BstEll-HF for 30 min. The enzyme solution was replaced by fresh cell culture medium and the flow was continued for another 2.5 h. The about 9 mL outflow volume were collected and the cells in the collected outflow were precipitated *via* centrifugation, resuspended in 200 $\mu$L fresh medium, and cultured in a 96-well plate with a cover glass bottom. After 24 h, the cells were examined by fluorescence microscopy.

## 11. Proliferation of mouse embryonic stem cells (mESCs)

To monitor the mESC proliferation on fresh composite materials, $5\times10^3$ cells were seeded on the freshly prepared SCx (S100, SC50, SC25, SC12.5, SC6.25, SC2.5, and C100, respectively) materials in the presence or absence of leukemia inhibitory factor (LIF). The relative cell numbers on each of the SCx were quantified by CCK-8 assay, as described above. All experiments were carried out in triplicate.

For control, glass substrate coated with poly-L-lysine (PLL), gelatin (PLL-G), Matrigel and Geltrex in the presence or absence of LIF were used. To investigate mESC growth in Matrigel or Geltrex, 2D and 3D cell cultivation of mESCs was performed. For 2D Matrigel or Geltrex cultures, 40 $\mu$L aliquots of cold Matrigel or Geltrex were diluted 10-fold with DPBS and immediately spread into wells of a glass bottom 96-well plate, where the gelation was completed after 2 h at 37 °C. Subsequently, 6000 mESCs were seeded on top of Matrigel- or Geltrex-coated surfaces. For 3D Matrigel or Geltrex culture, 40 $\mu$L aliquots of cold undiluted Matrigel or Geltrex were mixed with 6000 mESCs in 5 $\mu$L cell culture medium on ice. The resulting cold mixtures were immediately spread into microplate wells where gelation was completed for 30 min at 37 °C.

## 12. Immunofluorescence staining of mESCs on composite material

To investigate the mESCs pluripotency, $5\times10^3$ cells were seeded on the fresh SCx materials and allowed to grow for 4 days. For immunofluorescence staining of pluripotency markers, the cells were fixed with 4% PFA in DPBS for 30 min. After permeabilization with a triton X-100 solution (1%, in DPBS) for 1 h, cells were incubated with the primary antibodies (mAb rabbit anti-Nanog, 1: 200, Abcam; mAb IgG mouse anti-Oct4, 1: 200, Santa Cruz Biotechnology; pAb rabbit anti-Sox2, 1: 200, Abeam) for 12 h at 4 °C. Samples were washed with DPBS and subsequently incubated with the secondary antibodies (goat anti-mouse IgG Cy3, 1:200, Dianova; goat anti-rabbit IgG Alexa Fluor®488, 1:200, Life Technology) in 1% BSA in DPBS for 4 h at room temperature. The cell nuclei were counterstained with DAPI. For control, the cells grown on PLL and gelatine-covered PLL were used.

## 13. Immunofluorescence staining of germ layers

The formation of germ layers in the embryoid body (EB) was achieved by immunostaining of FoxA2, Brachyury and $\beta$ Tubulin III of mESC EB. Briefly, after cultivation on mESCs in S100 for 4 days in the absence of LIF, the formed spheroids were released *via* treatment with restriction endonuclease BstEll-HF for 2 h at 37 °C and allowed to grow in a U shaped plate for another 3 days. The EBs were then cultivated for 2 weeks on FN-coated coverslips, fixed, and permeabilized. Immunostaining was achieved with pAb IgG goat anti-FoxA2/Brachyury (R&D Systems) and pAb IgG rabbit anti-$\beta$ Tubulin III (Sigma) using secondary antibodies (Donkey anti-goat IgG Alexa Fluor®488/Alexa Fluor®647 and Donkey anti-rabbit IgG Alexa Fluor®568, Thermo Fisher Scientific).

14. Conductivity measurement

To evaluate the electrical conductivity of the composite materials, galvanostatic measurements were performed on interdigitated array (IDA) electrodes. Briefly, 50 μL of RCA reaction mixtures was prepared and then allowed to polymerize to form S100, SC12.5, and C100, respectively, as described above. Following, the buffer in the resultant hydrogels was exchanged with ultrapure water with a resistivity higher than 18.2 MΩ cm. Subsequently, the composite materials were placed on the surface of IDA (gold interdigitated electrodes made on transparent flexible plastic substrate with dimension for bands/gaps of 10 and 100 μm, respectively) and the current ranging from $1.0*10^{-8}$ to $5*10^{-5}$ A was applied.

15. Cultivation of bacteria on the composite material

The feasibility of the composite material for bacteria cultivation was exemplified by using monomeric red fluorescent protein (mRFP)-expressing E. coli and Shewanella strains carrying a kanamycin resistance. To this end, a lysogeny broth (LB) medium suspension of E. coli or Shewanella with an optical density of 0.1 at 600 nm was prepared. After dilution for 10 times using freshly prepared LB medium containing 50 μg/mL kanamycin and 10 mM arabinose, the suspensions of E. coli or Shewanella were transferred into S100-/SC12.5-coated 96-well glass bottom plates. The growth of bacteria was monitored by fluorescence microscopy at 37 °C for E. coli and 30 °C for Shewanella, respectively.

16. *In vitro* protein production in the composite material

Coupled transcription and translation kits (Rapid Translation System, RTS) were purchased from Biotechrabbit, and reactions were carried out by the procedures indicated by the manufacturer. Firstly, the PCR product of linear eGFP gene fragment (~6 kbp, 2 μg) was incorporated into the S100 or SC12.5 (25 μL) during RCA reaction, as described above. After purification of the protein-producing gel (P-gel) using 10% (v/v) reconstruction buffer, the S100/gene (S100 P-gel) or SC12.5/gene (SC12.5 P-gel) complex was used for *in vitro* protein expression. Unless stated otherwise, the reaction volume was kept at 50 μL. The protein expressions were conducted at 30 °C with 250 r.p.m. of shaking. As controls, the solution phase system (SPS, as specified by the RTS manufacturer) and a pure DNA hydrogel/gene (DNA P-gel) containing the same amounts of gene fragment were used for *in vitro* protein expression. Expression of the eGFP was monitored by fluorescence detection.

17. Data

17.1 RCA-based SiNP-DNA hydrogel

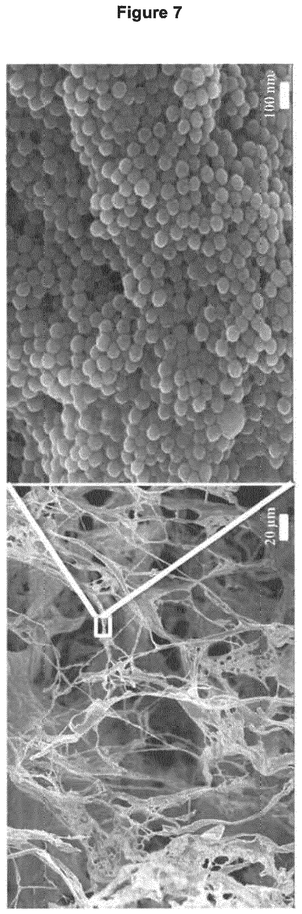

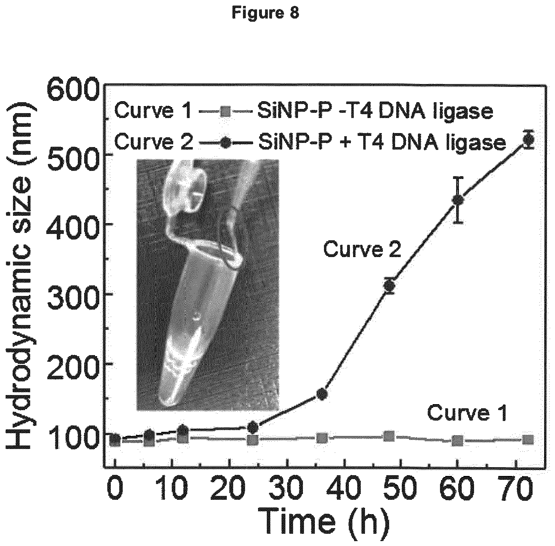

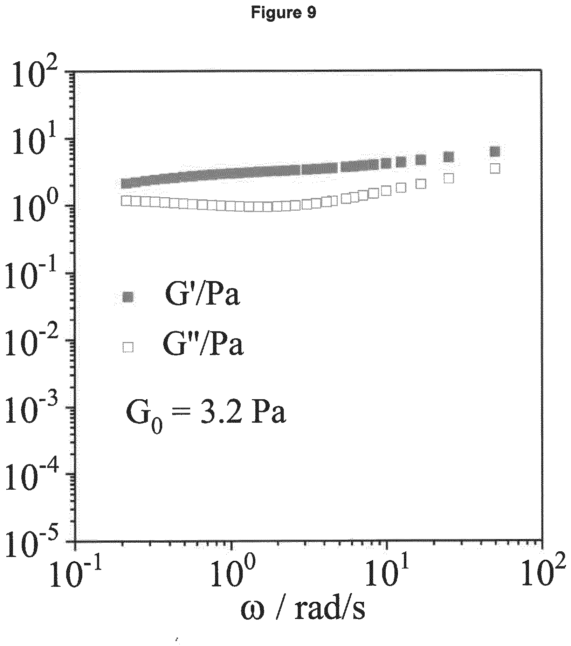

To monitor the gelation process, dynamic light scattering (DLS) was employed to record the hydrodynamic size of SiNP in the RCA reaction mixture (Figure 8). To simplify the description, we abbreviate SiNP-DNA hydrogel as S100xh, where x represents the RCA time. The particle-primer-template without T4 DNA ligase treatment displayed no obvious change in the hydrodynamic size. In sharp contrast, the hydrodynamic size of SiNP increased over time when the nicked end of template T on particle surface was ligated. The SiNP-DNA hydrogel (S100) formed after 48-hour incubation at 30 °C leading to a hydrogel that could be stretched like a rubber band (inset in Figure 8).
The structural feature of this hydrogel was characterized by scanning electron microscopy (SEM) (Figure 7). The dehydrated sample from RCA of primer on SiNP for 48 h showed a 3D crosslinked morphology with DNA linker filaments between particles. The DNA polymer which serves as a linker between two particles could be visualized in the magnified SEM image.
Multiple particle tracking (MPT) microrheology was then applied to quantitatively characterize the mechanical strength by introducing and tracking 0.5 μm or 1.0 μm polystyrene (PS) tracer particles in RCA reaction solution. In MPT experiments, the thermally driven motion of inert tracers that are distributed within a sample is monitored. The resulting particle trajectories are transformed into mean square displacement (MSD) traces and the viscoelastic moduli G' and G" can be extracted from the average MSD. The rheological data confirm that the $S100_{48h}$ (over the entire frequency range) reveals typical characteristics of hydrogels, as G' is constantly higher than G" and G' at the frequency of 10 rad $s^{-1}$ is termed as $G_0$ that was further calculated to be 3.2 Pa (Figure 9).

17.2 HCR-based SiNP-DNA hydrogel

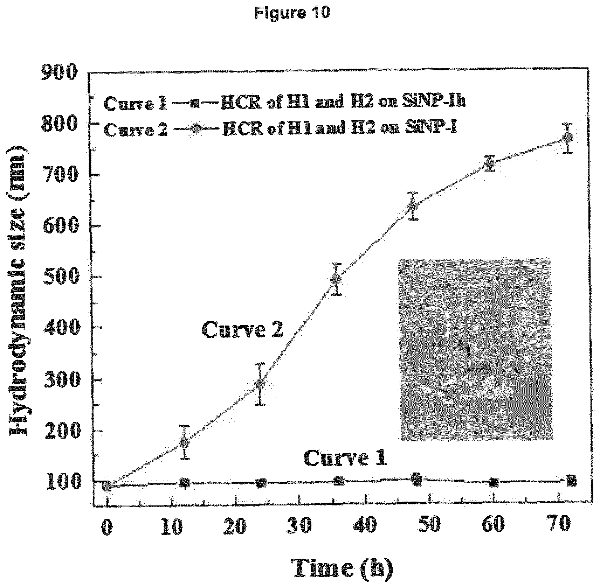

To monitor the gelation process, DLS was employed to record the hydrodynamic size of SiNP in the HCR reaction mixture (Figure 10). It is evident that SiNP-Ih without initiator I displays no obvious change in the hydrodynamic size. In sharp contrast, the hydrodynamic size of SiNP-1 increases over time when H1 and H2 were triggered by I on the particle. Notably, the SiNP-DNA hydrogel is solid after 48-hour incubation at room temperature (inset in Figure 10).

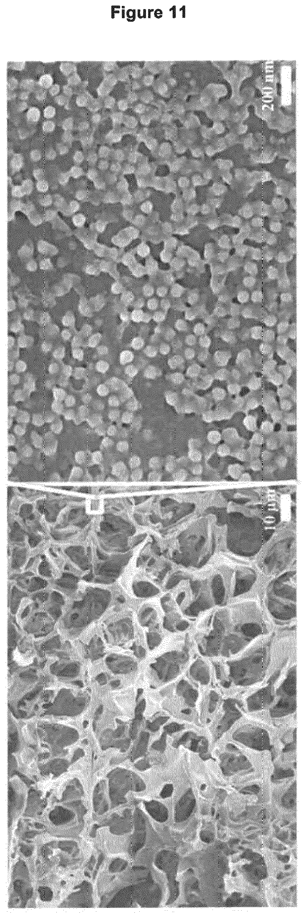

The structural feature of this HCR-reinforced hydrogel was characterized by scanning electron microscopy (SEM) (Figure 11). The dehydrated sample from HCR of H1 and H2 on SiNP-I for 48 h showed a 3D crosslinked morphology. The magnified SEM image shows DNA polymer covering on particle surface and crosslinked particles.

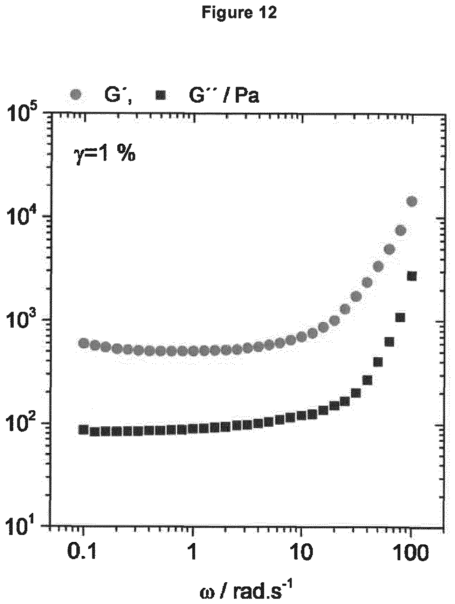

Bulk rheological tests were then applied to quantitatively characterize the mechanical strength of this hydrogel generated by HCR of H1 and H2 on SiNP-1. Rheology datum confirms that the SiNP-DNA hydrogel is a true hydrogel, as G' is constantly higher than G" in the frequency range of 0.05 and 100 rad s$^{-1}$ (see Figure 12).

17.3 RCA-mediated polymerization of SiNP-DNA hydrogel inside DOTAP-stabilized droplets

As shown in Figure 13a, Cy5-doped SiNP accumulated in the interior surface of the droplet like a shell. This occurs due to electrostatic interactions between the positively charged DOTAP membrane and negatively charged DNA-modified SiNP. After incubation of the RCA reaction mixture inside the droplet at 30 °C for 48 h, FRAP experiments were performed. It was found that the bleached central angel of Cy5-doped SiNP only slowly recovered (from 33.65° to 30.40°) within 2 h after photobleaching (Figure 13b,c), indicating that the fluidity of the SiNP shell was low. This indicates that the SiNP shell was in the gel phase due to RCA-induced polymerization.

17.4 Composite material comprising SiNP, CNT and DNA hydrogel

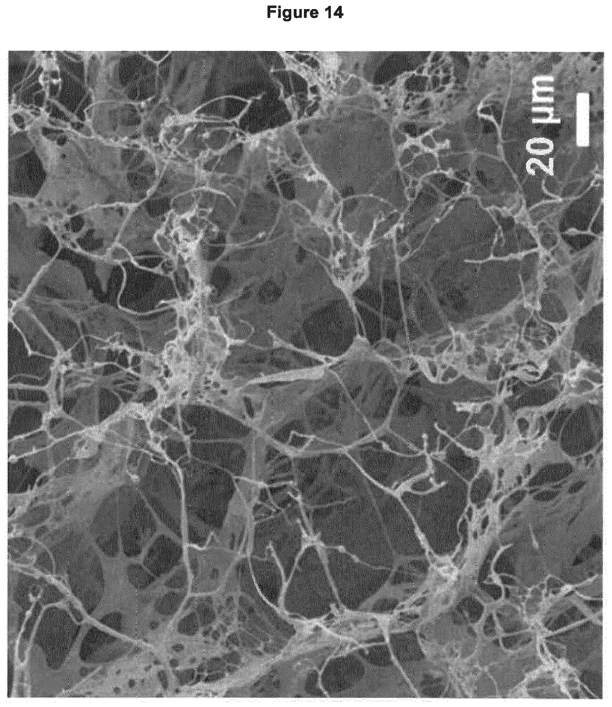

The structural feature of the composite material comprising SiNP/CNT-DNA hydrogel was characterized by SEM (Figure 14). The dehydrated sample obtained by RCA of primer-modified SiNP and CNT for 48 h showed the typical 3D crosslinked morphology.

17.5 Mechanical properties of the hydrogels

**Table 1:** MPT microrheology characterization of SiNP/CNT-DNA hydrogels synthesized *via* RCA reaction as compared to HCR-reinforced SiNP-DNA hydrogels.

| Entry | Name[a] | [SiNP-P][b,e] | [CNT-P][b,e] | [H1] or [H2] | $G_0$ (Pa)[c] |
|---|---|---|---|---|---|
| 1 | S100 | 4000 | 0 | 0 | 3.2 ± 0.4 |
| 2 | SC50 | 4000 | 80 | 0 | 4.8 ± 0.2 |
| 3 | SC25 | 4000 | 160 | 0 | 8.5 ± 0.7 |
| 4 | SC12.5 | 4000 | 320 | 0 | 14.1 ± 1.0 |
| 5 | S100-HCR[d] | 4000 | 0 | 120 µM H1, 120 µM H2 | 7.3 ± 0.7 |

[a] Acronyms in Entry 1-4 represent the mass ratio of SiNP-P:CNT-P subjected to RCA polymerization
[b] Given in µg/mL
[c] Determined by multiple particle tracking (MPT) after 48 h RCA reaction time. Note that the storage modulus (elastic modulus $G_0$) is substantially higher for ternary composite materials (e.g., SC12.5) than for binary composite material S100.
[d] S100-HCR represents the S100 reinforced by HCR method.
[e] DNA concentration may vary depending from the concentration and amount of nucleotides and the duration of RCA. This is well known for conventional DNA polymers. The maximum DNA concentration corresponds to the amount of nucleotides used in RCA or primers used in HCR.

Indicated in Table 2 are various composite materials that contained only SiNP or CNT (in the following denoted as S100 or C100, respectively) or ternary composite materials containing both SiNP and CNT, denoted as SCx, where x gives the mass ratio of SiNP-P:CNT-P. S100 hydrogels containing 4 mg/mL SiNP had excellent properties for cell experiments after 48 h of polymerization, whereas a maximum of 320 µg/mL CNT could be incorporated into pure C100 hydrogels. Higher amounts of CNT led to precipitation from the reaction mixture and hardly reproducible formation of brittle materials with no apparent viscosity. Hence, all ternary composites contained relative fractions of these maximal amounts. In the following, short term acronyms of the ternary materials SCx are as indicated in Table 2.

**Table 2:** Overview on binary and ternary SiNP/CNT-DNA hydrogel composite materials.

| Entry | Name[a,d] | [SiNP-P][b,d] | [CNT-P][b,d] | $G_0$ (Pa)[c] |
|---|---|---|---|---|
| 1 | S100 | 4000 | - | $3.2 \pm 0.4$ |
| 2 | SC50 | 4000 | 80 | $4.8 \pm 0.2$ |
| 3 | SC25 | 4000 | 160 | $8.5 \pm 0.7$ |
| 4 | SC12.5 | 4000 | 320 | $14.1 \pm 1.0$ |
| 5 | SC6.25 | 2000 | 320 | $10.3 \pm 1.0$ |
| 6 | SC2.5 | 800 | 320 | $3.1 \pm 0.2$ |
| 7 | C100 | - | 320 | $2.8 \pm 0.2$ |

[a] Acronyms represent the mass ratio of SiNP-P:CNT-P subjected to RCA polymerization

[b] Given in $\mu$g/mL

[c] Determined by multiple particle tracking (MPT) after 48 h RCA reaction time. Note that the storage modulus (elastic modulus $G_0$) is substantially higher for ternary composite materials (e.g., SC12.5) than for binary composite materials S100 or C100.

[d] DNA concentration may vary depending from the concentration and amount of nucleotides and the duration of RCA. This is well known for conventional DNA polymers. The maximum DNA concentration corresponds to the amount of nucleotides used in RCA.

Dynamic light scattering (DLS) was used to monitor the RCA-induced gelation process of S100 (SiNP-DNA hydrogel) (Figure 15d). It is clearly evident that the hydrodynamic size of SiNP-P (SiNP modified with ssDNA primers) increased over time when the linear template T was circularized and ligated on the particle surface. For control, SiNP-P without ligase treatment displayed negligible changes in the hydrodynamic size. Naked-eye-inspection clearly indicated that the materials revealed typical viscoelastic properties of hydrogels after reaction times of >12 h (Figure 2, right side).

To quantitatively characterize the mechanical properties of the composite materials, multiple particle tracking microrheology (MPT) is used. In this technique, the Brownian motion of polymer-embedded fluorescent tracer particles is quantitatively tracked by time-resolved microscopy. The computed trajectories are directly correlated with the rheological properties through the Generalized Stokes-Einstein Relation (Oelschlaeger, C., Bossler, F. & Willenbacher, N. Synthesis, structural and micromechanical properties of 3D hyaluronic acid-based cryogel scaffolds. Biomacromolecules 17, 580-589 (2016)). The particle trajectories are transformed into mean square displacement (MSD) traces that allow for calculation of the linear viscoelastic moduli, the shear-storage modulus (G') and the shear-loss modulus (G"). It is clearly evident from the trajectory maps of tracer particles in Figure 15e,f that the particles can freely diffuse in solutions of SiNP-P but are restrained by the network of polymerized S100. Calculation of the viscosity $\eta_{MPT}$ and plateau moduli of G' ($G_0$) yielded 1.0 mPa·s and 3.2 Pa, respectively, for S100 after 48 h of polymerization, and these values increased with increasing RCA time. Likewise, the viscoelastic properties of C100 gels depended on the CNT-P (CNT modified with P2 primers) concentration as indicated from MPT analyses. Notably, the binary nanocomposites differ in mesh size (0.2-0.5 and 0.5-1.0 $\mu$m, for S100 and C100, respectively), as estimated from MPT measurements using differently sized tracer particles experiments. The mechanical properties of the various ternary SCx materials were significantly changed in dependency of the mass ratio of SiNP-P:CNT-P (Tables 1 and 2). In fact, $G_0$ constantly increased from 4.8 Pa to 14.1 Pa with increasing amounts of CNT (SC50, SC25, and SC12.5), indicating that, indeed, the CNT content increases the composite material's mechanical stiffness. Likewise, increased concentrations of SiNP enhanced the mechanical stiffness of the ternary SC hydrogels (3.1 Pa for SC2.5, 10.3 Pa for SC6.25, and 14.1 Pa for SC12.5) when the concentration of CNT was constant. Importantly, the $G_0$ values of some SCx materials were substantially higher than those expected from totaling the values of pure S100 (3.2 Pa) and C100 (2.8 Pa). This result clearly demonstrates that the two differently shaped nanoparticles are synergistically determining the mechanical stiffness and viscoelastic properties of the composite materials.

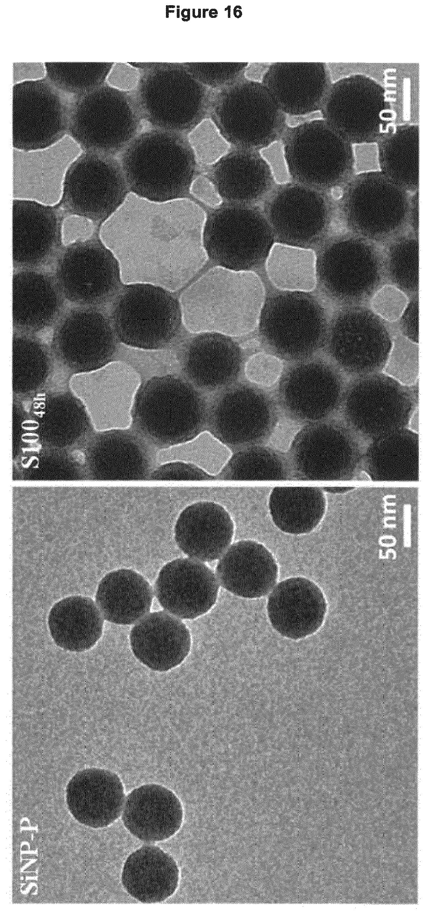

The structural features of the hydrogels were characterized by transmission electron microscopy (TEM, Figure 16) and scanning electron microscopy (SEM, Figure 7). S100 gel has an amorphous morphology with a distinctive hierarchical ultrastructure, which clearly reveals the DNA-coated particles that are connected by DNA filaments. Direct comparison of TEM/SEM images of SiNP-P and S100 (Figures 16 and 17) and analysis by scanning transmission electron microscopy (STEM) coupled with energy dispersive X-ray spectroscopy (EDS, Figure 15) confirmed the presence of a dense layer of polymerized DNA polymer that coats and crosslinks the particles in the composite material. Further, HCR-reinforced SiNP-DNA hydrogel displays a larger $G_0$ when compared with SiNP-DNA hydrogel S100 prepared by RCA.

Altogether, these results indicate that the mechanical properties of SiNP-DNA hydrogels can be enhanced by the formation of composite hydrogels or combination of HCR method.

17.6 Modular variations of the design

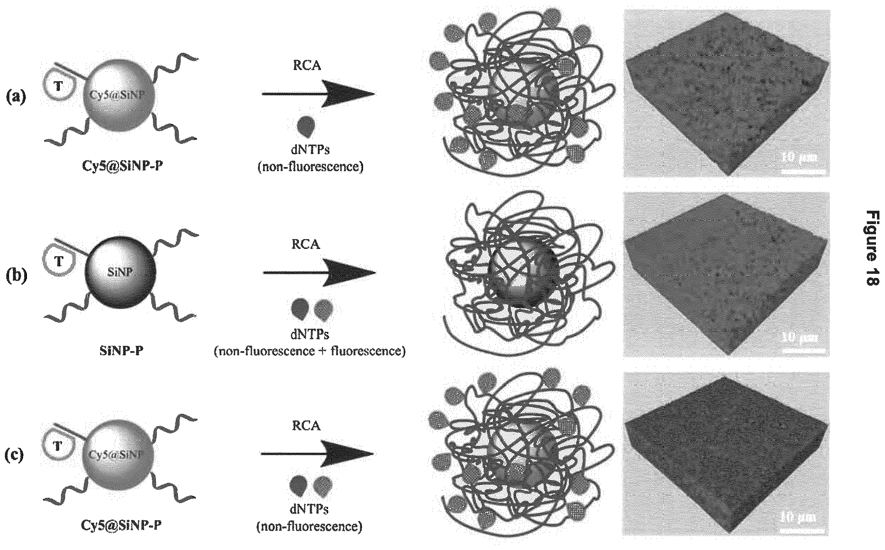

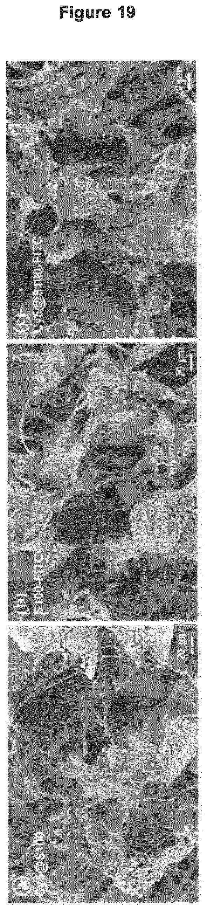

Several studies were carried out to exemplify the modularity of composite material design: Firstly, dye-modified SiNP can be conveniently introduced to enable fluorescence tracking of the materials. This feature is important, for instance, for studies of cell uptake and degradation of hydrogels. It is demonstrated in Figure 18 that fluorescent properties can be incorporated into the composite material by either using dye-encoded SiNP, obtained through co-hydrolysis of TEOS and Cy5 dye-conjugated silane (Leidner, A. et al. Biopebbles: DNA - Functionalized Core-Shell Silica Nanospheres for Cellular Uptake and Cell Guidance Studies. Adv. Funct. Mater. 0, DOI 10.1002/adfm.201707572 (2018)). Else, fluorescent properties can be generated by RCA-based incorporation of chemically modified deoxynucleotides bearing fluorescent dyes, such as fluorescein (FITC)-modified dUTP. The resulting materials revealed the expected optical properties (Figure 18) while their morphology remained unchanged (Figure 19).

Secondly, variations of the DNA backbone can be implemented. For example, the introduction of restriction sites in conventional DNA polymers enables the design of responsive materials that can be post-synthetically modified with enzymes (Lilienthal, S., Shpilt, Z., Wang, F., Orbach, R. & Willner, I. Programmed DNAzyme-Triggered Dissolution of DNA-Based Hydrogels: Means for Controlled Release of Biocatalysts and for the Activation of Enzyme Cascades. ACS Appl. Mater. Interfaces 7, 8923-8931 (2015)). To introduce restriction sites into the single-stranded backbone of the composite materials, stem-loop structures of appropriate sequence were encoded in the RCA template (T), thereby enabling efficient enzymatic degradation and concomitant gel-to-sol transition of the composite material (Figure 20). This feature is of great utility, for instance, to achieve controllable release of polymers or polymer-embedded cells, which is required for therapeutic applications and dynamic cell manipulations. To illustrate the applicability of the aforementioned strategies for studies on the trackable drug delivery to cells, a fluorescent S100 material was engineered that contained a structured backbone bearing the Sgc8 aptamer, which specifically binds to the PTK7 receptor (Shangguan, D. et al. Aptamers evolved from live cells as effective molecular probes for cancer study. Proc. Natl. Acad. Sci. U. S. A. 103, 11838-11843 (2006)), as well as double-stranded GG- or GC-rich drug association sites, which can be used for enrichment of intercalating drugs, such as anthracycline doxorubicin (DOX) (Zhu, G. et al. Self-Assembled Aptamer - Based Drug Carriers for Bispecific Cytotoxicity to Cancer Cells. Chem. - Asian J. 7, 1630-1636 (2012)). Studies with PTK7-positive HeLa cells showed that uptake of fluorescent S100 was dependent on the amount of aptamer units incorporated into the material (Figure 21). After loading these materials with DOX, their uptake was also clearly dependent on the amounts of aptamer and drug loading moieties incorporated into the DNA backbone (Figure 22). These findings indicate that the composite materials hold the potential for applications as vehicles for the cell-specific delivery of DNA-binding drugs into cancer cells.

17.7 Cytotoxicity Assay

Cell Count Kit-8 (CCK-8, sigma) cell viability assay was employed to evaluate the *in vitro* cytotoxicity of the SiNP/CNT-DNA hydrogels under standard manufacturer's instructions. In brief, MCF-7 cells at a density of $8 \times 10^3$ cells per well in 200 $\mu$L MCF7 medium were seeded into wells of a 96-well plate. After incubation at 37 °C and 5% $CO_2$ for 12 h, the adherent cells were incubated with 200 $\mu$L fresh medium containing PBS (control) and 75 $\mu$L SiNP-DNA hydrogel (S100), CNT-DNA hydrogel (C100), or SiNP/CNT-DNA hydrogel (SCx) at different SiNP/CNT mass ratios. After another 24 h incubation, 20 $\mu$L CCK-8 was added into each well, and the MCF7 cells were then incubated for another 4 h at 37 °C and 5% $CO_2$. Following, the absorbance of each well at 450 nm was recorded using a BioTek Synergy microplate reader. Cell viability was calculated according to equation (1):

$$\text{Cell viability} = \frac{A1-B}{A0-B} \qquad (1)$$

where A0 and A1 represent the OD values of CCK-8 in medium containing MCF-7 cells after treatment with PBS, S100, C100, and SCx, respectively. B represents the OD value of CCK-8 in medium. Average numbers and standard deviations (SD) of 5 parallels for each sample were reported.

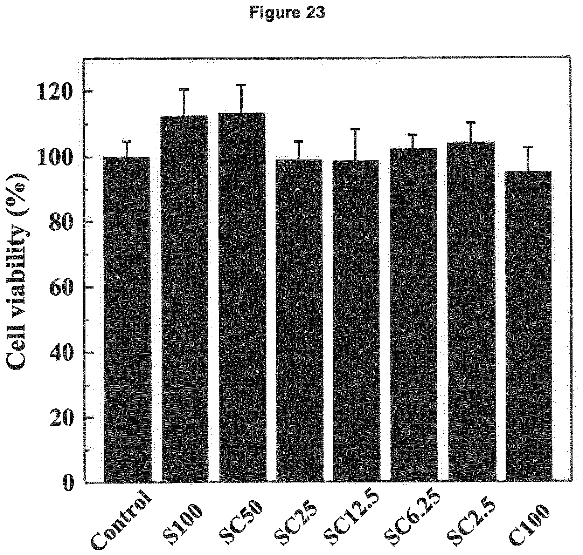

As shown in Figure 23, all types of hydrogels exhibit negligible cytotoxicity on cells as compared to PBS control. These results indicate that SiNP/CNT-DNA hydrogels possess excellent biocompatibility.

17.8 Cell adhesion, migration and proliferation on ternary SiNP/CNT DNA hydrogel composite materials

Further, the utility of ternary SCx composite materials for *in vitro* cell culture was determined. Using MCF7 breast cancer cells, as described above, it was established that none of the various hydrogels revealed any cytotoxicity (Figure 23). For an in-depth investigation of the composite materials, arrays of the various materials were prepared in 96 well glass bottom plates by adding 75 $\mu$L reaction mixtures for RCA synthesis of the materials directly inside the wells (Figure 24a). Polymerization for 48 h led to formation of a hydrogel layer of about 350 $\mu$m thickness. These freshly prepared materials were either used directly for cell culture experiments (route A in Figure 24a) or else subjected to drying for 4 h under low pressure at room temperature (route B in Figure 24a), which led to thin films of about 7 $\mu$m thickness (corresponding to an about 98% shrinkage upon drying). After seeding of MCF7 cells that constitutively express an eGFP-tagged epidermal growth factor receptor on their cell membrane (MCF7$_{eGFP}$) (Angelin, A. et al. Multiscale Origami Structures as Interface for Cells. Angew Chem Int Ed Engl 54, 15813-15817 (2015)) on top of these freshly prepared or dried hydrogels, cells were cultured for 24 h. Quantitative assessment of cell proliferation with the CCK-8 assay revealed distinctive material-dependent differences in the adhesion and proliferation of living cells (Figure 24b,c).

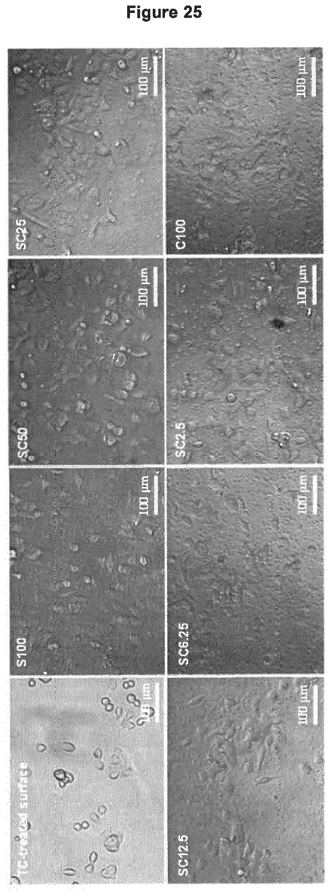

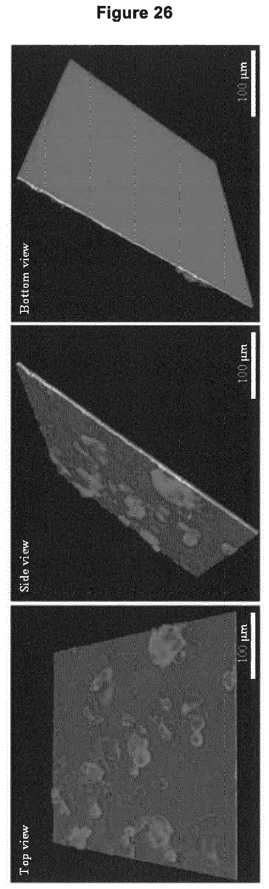

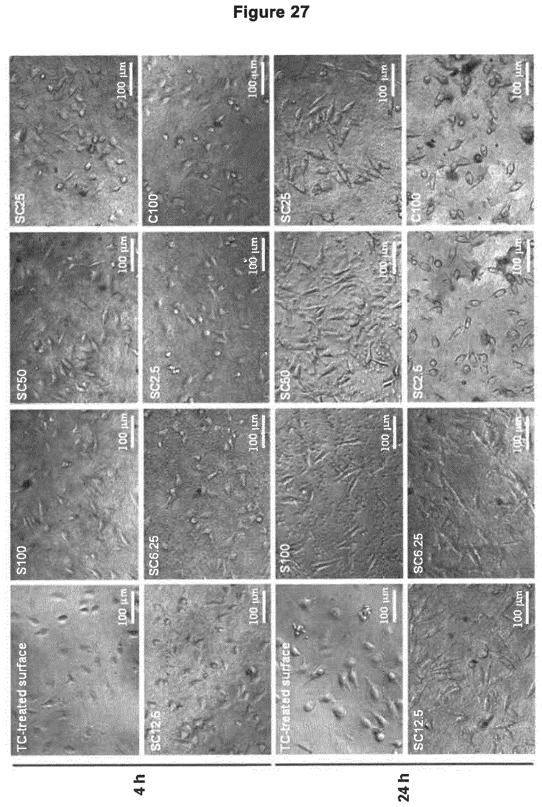

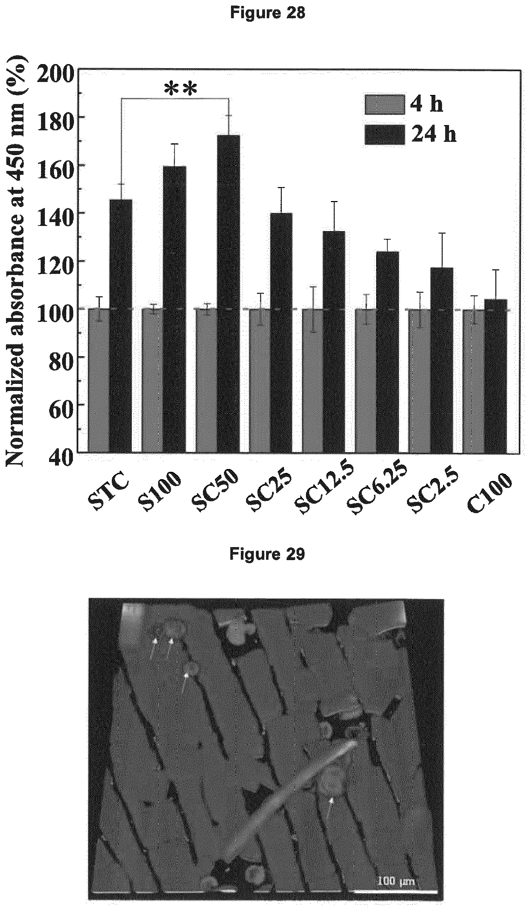

For the fresh hydrogels, proliferation of cells on S100 and SC50 was slightly lower than on the standard tissue culture (STC) surface lacking the hydrogel whereas the other composite thin films decreased proliferation upon increasing amounts of CNT (Figure 24b). The composition of dried hydrogels induced a similar trend, however, on a substantially different level: Proliferation of cells on S100 and SC50 was significantly higher than on STC, while the other composite thin films still induced proliferation, although with decreasing effectiveness at increasing amounts of CNT (Figure 24c). Microscopy inspection revealed that cells on dried films showed a more pronounced elongated fusiform and flattened morphology than cells adhered on STC surface (Figures 24e, Figure 25, Figure 26). The same, however, even more pronounced results were obtained with rat embryo fibroblast cells REF52 (Figures 27 and 28). Life cell imaging of MCF7$_{eGFP}$ also indicated that cells on SC50 are highly agile in terms of spreading, communication, motility, and proliferation. Control experiments with SiNP-P that were not subjected to RCA polymerization showed that the particles dried into thin layers of cracked particle blocks to which MCF7$_{eGFP}$ cells predominantly adhered at the blocks' edges in a round morphology (Figure 29). The high attractiveness of dried SC50 was also emphasized in experiments, where cells were allowed to adhere to either STC or SC50 surfaces (Figure 30) or to specifically migrate from STC to SC50 surfaces (Figure 24f,g).

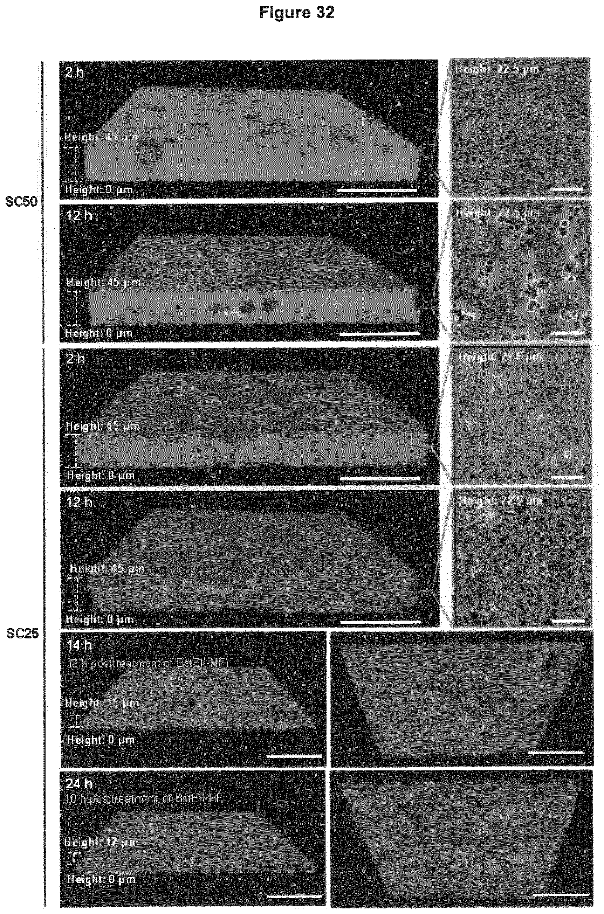

To shed light on cellular adhesion strength, MCF7 cells were allowed to adhere for 1 h on the various surfaces, and the samples were then washed thoroughly with buffer to remove weakly adhered cells. There, the adhesion strength steadily increased with increasing amounts of CNT in the composite materials (Figure 31). The observed preferences for distinct composite surfaces is attributed to a combination of nano-roughness, mechanical properties and anionic nature of the surfaces. Tumor cells are known for their preference to grow on rough surfaces. It is known from studies with standard cell culture substrates that highest cell motility and replication occurs at an intermediate adhesion strength, whereas strong adhesion impairs cell motility and proliferation. Therefore, it is concluded that the dried composite material films hold potential as tailorable coating for steering of cell adhesion, proliferation and motility, which is of substantial relevance for biomedical applications, such as medical implants. In addition, in-depth studies on the interaction of cells with the fresh composite material hydrogels were conducted, that led to a lower proliferation activity as compared to STC substratum (Figure 24b). It was observed that MCF7$_{eGFP}$ cells rapidly transmigrate into the matrix of hydrogels S100 and SC50 with an apparent velocity of about 2 $\mu$m/h (Figure 32). In contrast, the cells could not transmigrate but rather dug themselves into the upper layer of materials with higher contents of CNT (e.g., SC25, Figure 32). These observations are in line with the higher CNT concentration and mechanical stiffness of SC25, as compared to SC50 (Table 2).

Since washing did not remove the cells from SC25, it was concluded that this material should be suited for selective capture and enzyme-triggered release of surface-bound cells. Indeed, treatment of the SC25-entrapped cells with a restriction enzyme for 2 h led to a noticeable reduction of the gel's thickness from 45 $\mu$m to 15 $\mu$m along with the release of adhered cells (Figure 32). Importantly, the released cells were still alive and migrated deeper into the gel matrix to adhere to the underlying STC where they propagated to form small cell populations 10 h after of enzymatic treatment. These results confirm the utility of the composite materials of the present invention for biomedical applications.

17.9 Cell adhesion on SiNP/CNT-DNA hydrogel

SiNP/CNT-DNA hydrogel (75 $\mu$L) was deposited on a $\mu$-dish (Ø = 35 mm, standard tissue culture (STC) surface) and dried in vacuum overnight. Afterwards, $1.2\times10^4$ MCF7 cells stably transfected to express the EGF receptor fused to enhanced green fluorescent protein (MCF7$_{eGFP}$ cells) were seeded on the top of the dried hydrogel and incubated for another 24 h. The cell morphology was observed by confocal fluorescence microscope.

It was found that less cells settled down on the untreated $\mu$-dish surface while more cells chose to stay on the hydrogel surface and spread there (right part of Figure 30). These results indicate that the hydrogel of the present invention is more attractive for cells than commercially available culture dish surfaces and can support cell spreading.

17.10 Cell adhesion and release studies under flow conditions

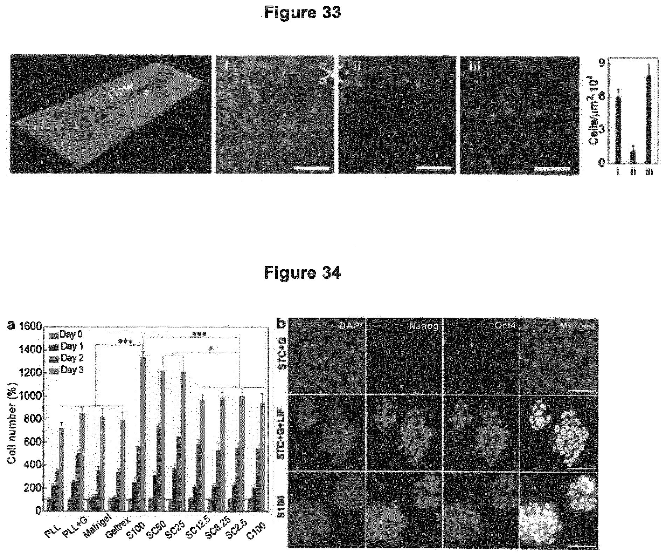

Using the microchannel, transfusion of the channel with a suspension of MCF7$_{eGFP}$ cells led to formation of surface-bound cell populations after 2 h. The SC25 coating was then broken by addition of BstEll-HF restriction enzyme (0.5 h) and the collected outflow of the channel was cultured for an additional 24 h under standard conditions in a petri dish. Fluorescence microscopy analysis clearly showed that the cells had not been harmed by the procedure but were capable of adhesion, spreading, and proliferation after release from the channel (Figure 33). These results underline the utility of the novel nanocomposite materials for biomedical research.

17.11 Expansion of stem cells

Quantification of growth rates by microscopic analysis and CCK-8 assays (Figure 34a) consistently showed that the mESCs proliferated faster on the composite materials than on the control surfaces, with SC100, SC50 and SC25 showing the best performance. The time-dependent consideration showed that mESC growth proceeds faster on more rigid surfaces in the beginning (propagation rates: SC25>SC50>S100 at day 1), whereas the growing 3D colonies expand better in the softer matrices with increased culture times (propagation rates: SC50>SC25>S100 or S100>SC50>SC25 at days 2 or 3, respectively). Importantly, the spheroid colonies grown on the composite materials maintained their pluripotency, as indicated by immunostaining of the pluripotency markers Nanog, Oct4, and Sox2, whereas LIF was required for the control surfaces (Figure 34b). These observations are consistent with previous studies on soft substrata for preserving stemness. Furthermore, comparative studies on the growth of mESC spheroids under 2D or 3D culture conditions indicated that growth in S100 led to spheroids that reveal the desired uniform, concentric, compact and raised shape to a much greater extent than those grown on standard substrates.

To further explore their applicability for stem cell research, we used composite materials in accordance with the present invention for the growth and isolation of spheroids (Figure 35). The enzymatic release of spheroids from S100 led to almost monodisperse cell bodies, which showed the desired shape to a much greater extent than those grown by established protocols based on Matrigel, Geltrex or pNIPAM substrates. The mESC spheroids grown in S100 for 4 days could be enzymatically released and further processed using standard methods for cultivation and differentiation of embryoid bodies (EBs) (Figure 35b). Cellular migration and outgrowth from the differentiating aggregates occurred in a similar way as observed for EBs prepared by the established hanging drop method. As determined by immunostaining, the resulting colonies exhibited the expected markers of the differentiated germ layers (Figure 35c).

17.12 Conductivity of the composite material

The results in Table 3 and Figure 36 clearly demonstrate that fresh composite materials (S100, SC12.5 and C100) are conductive materials. All composite materials under dry (dehydrated) state display much profound conductivity than those under fresh (hydrated) state and the utility of CNT facilitated their conductivity.

**Table 3:** The conductivity of the composite materials measured on 10 $\mu$m IDA.

| 10 $\mu$m distance | S100 (Sm$^{-1}$) | SC12.5 (Sm$^{-1}$) | C100 (Sm$^{-1}$) |
|---|---|---|---|
| fresh | $2.3*10^{-4}$ | $2.4*10^{-4}$ | $2.7*10^{-4}$ |
| dry | $2.3*10^{-4}$ | 0.29 | 0.22 |
| 100 $\mu$m distance | | | |
| Fresh | - | $4.7*10^{-4}$ | 0.016 |
| Dry | - | 0.0072 | 0.018 |

17.13 Cultivation of bacteria on the composite material

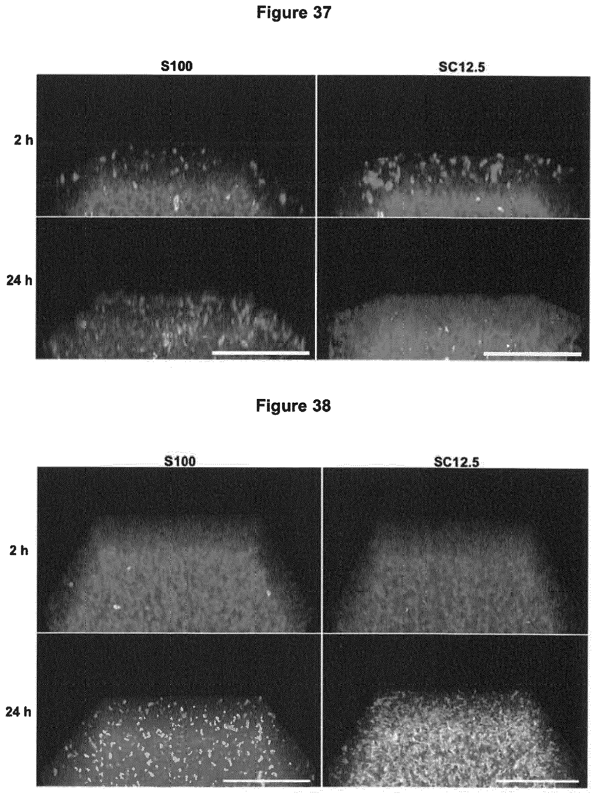

From microscopy inspection of the bacterial cultures, it is evident that E. coli (Figure 37) and Shewanella (Figure 38) cells grew into a three dimensional biofilm structure in the bulk composite materials. E. coli growth rate in S100 was higher than that in SC12.5, whereas Shewanella growth rate displayed the opposite behavior. Hence, the SiNP/CNT-DNA composite material can be designed as matrix for stabilizing and standardizing bacterial biofilms.

17.14 *In vitro* protein production in the composite material

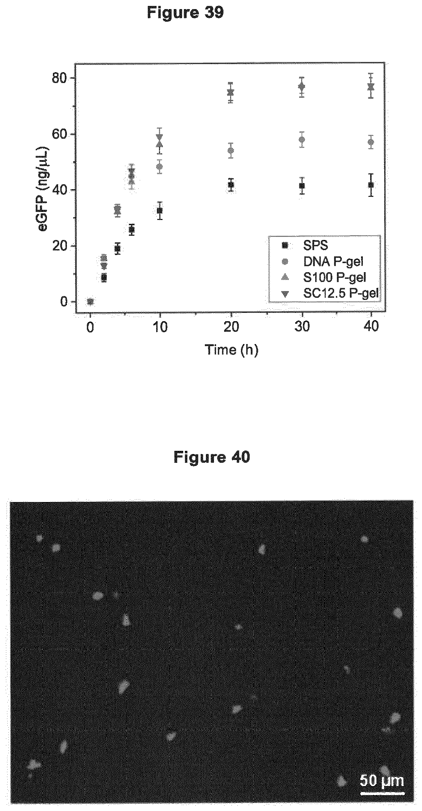

As shown in Figure 39, eGFP expression in all systems increased with reaction time and all P-gel systems greatly enhanced the protein production when compared with the standard SPS during the whole reaction time. S100 P-gel with additive SiNP was capable of producing more protein molecules than pure DNA P-gel after 10 h, while, after further interwaving of CNT, the stiffer SC12.5 P-gel did not suppress the protein production.

18. Microgels

18.1 Preparation of microscale SiNP/CNT-DNA hydrogel composite material

RCA reaction mixture (75 μL) for synthesis of SiNP/CNT-DNA composite microgel was prepared in the same way as described for the synthesis of SiNP/CNT-DNA hydrogel composite material. Herein, both SiNP-P and CNT-P were used with 4 mg/mL SiNP and 80 μg/mL CNT as the final concentrations, respectively. This mass ratio is the same as used for the SC50 composite material described above (Table 2). Subsequently, RCA reaction mixture was transferred into 600 μL mineral oil-surfactant mixture (4.5% (vol/vol) Span 80, 0.4% (vol/vol) Tween 80, 0.05% (vol/vol) Triton X-100), at the same time, stirred at a speed of 1,000 rpm for 30 min using a mechanical stirrer. The formed water-in-oil emulsion was incubated at 30 °C and stirred at a speed of 500 rpm. After another 48 h, microgels were isolated by precipitation in a large excess of isopropanol followed by centrifugation to remove the oil phase. The resultant microgels were thoroughly washed with isopropanol before being dispersed into 75 μL distilled water.

The morphology, size, and fluorescence property of the SiNP/CNT-DNA composite microgel were characterized by fluorescence microscopy (Figure 40). The microgels formed in an irregular shape and displayed strong Cy5 fluorescence. The typical diameter of the microgels was calculated to be about 10 μm in average.

18.2 Cellular uptake of microgels

200 μL REF52 cell suspension ($6 \times 10^3$ cells in REF52 medium) were placed into a 96-well plate. After incubation at 37 °C and 5% $CO_2$ for 12 h, the adherent cells were incubated with 200 μL fresh medium containing 10 μL of the aforementioned stock suspension of SiNP/CNT-DNA composite microgel. After an additional 6 h incubation, REF52 cells were washed with PBS, counterstained with Alexa Fluor 488 phalloidin and 4',6-diamidino-2-phenylindole (DAPI), and imaged by fluorescence microscope.

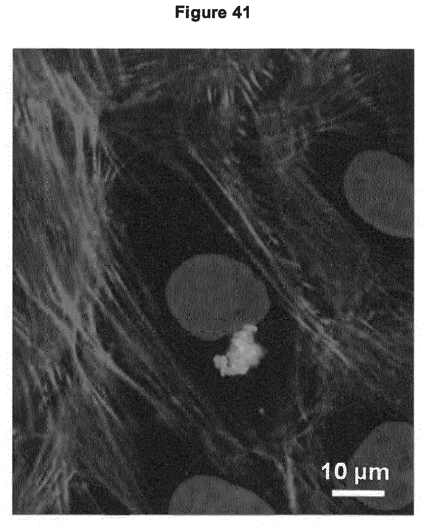

It was observed that SiNP/CNT-DNA composite microgels locate in the cytosolic compartment close to the cell nucleus, indicating the efficient cellular internalization of the microgels (Figure 41). Thus, the SiNP/CNT-DNA composite microgels can be used to realize delivery of cargo, such as drugs, proteins, or genes, to the nuclear compartment of eukaryotic cells.

18.3 Cellular uptake of microgel encoded with mKate gene fragments

A gene fragment encoding for the red fluorescent protein "mKate" was incorporated in the microgels. To prepare SiNP/CNT-DNA microgels containing mKate gene fragments, the mKate gene was amplified from plasmid (pcDNA-EXP40-mKate, 6217 base pairs) via PCR and then was mixed with RCA reaction mixture at the concentration of 15 ng/μL. After preparation and purification of the microgels as described above, REF52 cells ($6 \times 10^3$ cells per well with 200 μL REF52 medium) were seeded into a 96-well plate. After incubation at 37 °C and 5% $CO_2$ for 12 h, the adherent cells were incubated with 200 μL fresh medium containing 10 μL stock suspension of microgel. After an additional 12 h incubation (Day 1) or 6 days, REF52 cells were washed with PBS, counterstained with Alexa Fluor 488 phalloidin and 4',6-diamidino-2-phenylindole (DAPI), and imaged by fluorescence microscope.

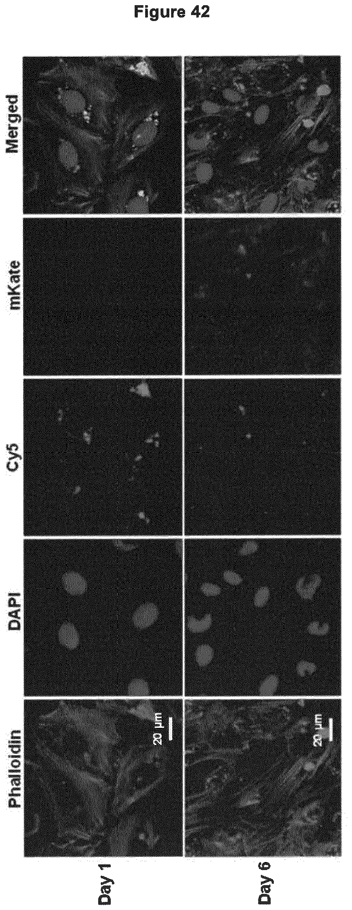

While REF52 cells did not express mKate protein after incubation for 12 h, the red fluorescence of mKate was clearly evident in the cytoplasma, indicating the capability of microgel for gene transfection (Figure 42). Thus, the SiNP/CNT-DNA microgels can be used as a carrier to deliver functional cargo into eukaryotic cells.

**List of Reference Numerals**

1    continuous phase inlet
2    reaction solution inlet
3    outlet

**Claims**

1. A composite material, comprising

   DNA hydrogel, and
   silica nanoparticles, wherein the silica nanoparticles are connected with the DNA hydrogel through DNA strands, wherein
   the content of the silica nanoparticles based on the total amount of the composite material is from 10 to 80 wt%,
   the content of the DNA hydrogel based on the total amount of the composite material is from 20 to 90 wt%,
   the silica nanoparticles have an average particle size in the range of from 20 nm to 2 $\mu$m,
   the composite material has a porous structure, wherein the pore size in the structure is more than 100 nm and less than 700 nm, and
   the DNA hydrogel is attached to the silica nanoparticles *via* a covalent bond.

2. The composite material according to claim 1, wherein the silica nanoparticles have an average particle size in the range of from 60 nm to 100 nm.

3. The composite material according to claim 1 or 2,
   further comprising carbon nanotubes embedded in the DNA hydrogel.

4. Use of the composite material according to any one of claims 1 to 3 for in vitro cargo delivery to a cell.

5. Use of the composite material according to any one of claims 1 to 3 as surface coating for *in vitro* cell culture.

6. Use of the composite material according to any one of claims 1 to 3 for in vitro enzyme-triggered cell release.

7. Use of the composite material according to any one of claims 1 to 3 for *in vitro* protein production.

8. Use of the composite material according to any one of claims 1 to 3 for applications in the fields of electronics, energy management, data processing, electrochemical or electrobiochemical syntheses, or biochemical fuel cells.

9. A microgel comprising the composite material according to any one of claims 1 to 3.

10. The microgel according to claim 9 having an average particle size of from 0.05 to 50 $\mu$m.

11. Use of the microgel according to claim 9 or 10 for the in vitro delivery of cargo to the
    cytosolic compartment of cells.

12. Use of the microgel according to claim 11 for in vitro gene transfection

13. A hollow spherical container, wherein the wall of the hollow spherical container comprises at least one layer containing the composite material according to any one of claims 1 to 3.

14. A microreactor comprising
    the hollow spherical container according to claim 13 and at least one cell in the space enclosed by the hollow spherical container.

15. Use of the microreactor according to claim 14 for high-throughput screening.

**Patentansprüche**

1. Verbundmaterial, umfassend

   DNA-Hydrogel, und
   Siliziumdioxid-Nanopartikel, wobei die Siliziumdioxid-Nanopartikel mit dem DNA-Hydrogel durch DNA-Stränge verbunden sind,
   wobei

der Gehalt der Siliziumdioxid-Nanopartikel, bezogen auf die Gesamtmenge des Verbundmaterials, 10 bis 80 Gew.-% beträgt,

der Gehalt des DNA-Hydrogels, bezogen auf die Gesamtmenge des Verbundmaterials, 20 bis 90 Gew.-% beträgt,

die Siliziumdioxid-Nanopartikel eine durchschnittliche Teilchengröße im Bereich von 20 nm bis 2 $\mu$m aufweisen,

das Verbundmaterial eine poröse Struktur aufweist, wobei die Porengröße in der Struktur mehr als 100 nm und weniger als 700 nm beträgt, und

das DNA-Hydrogel über eine kovalente Bindung an die Siliziumdioxid-Nanopartikel gebunden ist.

2. Verbundmaterial nach Anspruch 1, wobei die Siliziumdioxid-Nanopartikel eine durchschnittliche Teilchengröße im Bereich von 60 nm bis 100 nm aufweisen.

3. Verbundmaterial nach Anspruch 1 oder 2,
weiter umfassend in das DNA-Hydrogel eingebettete Kohlenstoff-Nanoröhrchen.

4. Verwendung des Verbundmaterials nach einem der Ansprüche 1 bis 3 zur Bereitstellung von Fracht an eine Zelle *in vitro.*

5. Verwendung des Verbundmaterials nach einem der Ansprüche 1 bis 3 als Oberflächenbeschichtung für *in vitro* Zellkultur.

6. Verwendung des Verbundmaterials nach einem der Ansprüche 1 bis 3 zur enzymatisch ausgelösten Zellfreisetzung *in vitro.*

7. Verwendung des Verbundmaterials nach einem der Ansprüche 1 bis 3 zur *in vitro* Proteinherstellung.

8. Verwendung des Verbundmaterials nach einem der Ansprüche 1 bis 3 für Anwendungen in den Bereichen Elektronik, Energiewirtschaft, Datenverarbeitung, elektrochemische oder elektrobiochemische Synthesen oder biochemische Brennstoffzellen.

9. Mikrogel, umfassend das Verbundmaterial nach einem der Ansprüche 1 bis 3.

10. Mikrogel nach Anspruch 9, das eine durchschnittliche Teilchengröße von 0,05 bis 50 $\mu$m aufweist.

11. Verwendung des Mikrogels nach Anspruch 9 oder 10 zur Bereitstellung von Fracht an das zytosolische Kompartiment von Zellen *in vitro.*

12. Verwendung des Mikrogels nach Anspruch 11 zur *in vitro* Gentransfektion.

13. Hohlkugelbehälter, wobei die Wand des Hohlkugelbehälters mindestens eine Schicht umfasst, welche das Verbundmaterial nach einem der Ansprüche 1 bis 3 enthält.

14. Mikroreaktor, umfassend
den Hohlkugelbehälter nach Anspruch 13 und mindestens eine Zelle in dem von dem Hohlkugelbehälter umschlossenen Raum.

15. Verwendung des Mikroreaktors nach Anspruch 14 zum Hochdurchsatz-Screening.

**Revendications**

1. Matériau composite, comprenant

de l'hydrogel d'ADN, et
des nanoparticules de silice, dans lequel les nanoparticules de silice sont connectées à l'hydrogel d'ADN par des brins d'ADN,
dans lequel
la teneur en les nanoparticules de silice basée sur la quantité totale du matériau composite va de 10 à 80 % en poids,
la teneur en l'hydrogel d'ADN basée sur la quantité totale du matériau composite va de 20 à 90 % en poids,
les nanoparticules de silice ont une taille particulaire moyenne dans la plage allant de 20 nm à 2 μm,
le matériau composite a une structure poreuse, dans lequel la taille de pore dans la structure est supérieure à 100 nm et inférieure à 700 nm, et
l'hydrogel d'ADN est relié aux nanoparticules de silice par le biais d'une liaison covalente.

2. Matériau composite selon la revendication 1, dans lequel les nanoparticules de silice ont une taille particulaire moyenne dans la plage allant de 60 nm à 100 nm.

3. Matériau composite selon la revendication 1 ou 2, comprenant en outre des nanotubes de carbone incorporés dans l'hydrogel d'ADN.

4. Utilisation du matériau composite selon l'une quelconque des revendications 1 à 3 pour la livraison de cargaison in vitro à une cellule.

5. Utilisation du matériau composite selon l'une quelconque des revendications 1 à 3 en tant qu'enrobage de surface pour la culture cellulaire in vitro.

6. Utilisation du matériau composite selon l'une quelconque des revendications 1 à 3 pour la libération cellulaire à déclenchement enzymatique in vitro.

7. Utilisation du matériau composite selon l'une quelconque des revendications 1 à 3 pour la production protéique in vitro.

8. Utilisation du matériau composite selon l'une quelconque des revendications 1 à 3 pour des applications dans les domaines de l'électronique, la gestion d'énergie, du traitement de données, des synthèses électrochimiques ou electrobiochimiques, ou des piles à combustible biochimiques.

9. Microgel comprenant le matériau composite selon l'une quelconque des revendications 1 à 3.

10. Microgel selon la revendication 9 ayant une taille particulaire moyenne allant de 0,05 à 50 μm.

11. Utilisation du microgel selon la revendication 9 ou 10 pour la livraison in vitro de cargaison au compartiment cytosolique de cellules.

12. Utilisation du microgel selon la revendication 11 pour la transfection génique in vitro.

13. Récipient sphérique creux, dans lequel la paroi du récipient sphérique creux comprend au moins une couche contenant le matériau composite selon l'une quelconque des revendications 1 à 3.

14. Microréacteur comprenant
le récipient sphérique creux selon la revendication 13 et au moins une cellule dans l'espace renfermé par le récipient sphérique creux.

15. Utilisation du microréacteur selon la revendication 14 pour le criblage à haut débit.

## Figure 1

# Figure 2

**Figure 3**

$\overline{30\ \mu m}$

**Figure 4**

## Figure 5

## Figure 6

## Figure 7

Figure 8

**Figure 9**

Figure 10

# Figure 11

**Figure 12**

## Figure 13

## Figure 14

**Figure 15**

## Figure 16

## Figure 17

# Figure 18

## Figure 19

## Figure 20

# Figure 21

# Figure 22

Figure 23

# Figure 24

## Figure 25

## Figure 26

## Figure 27

## Figure 28

## Figure 29

**Figure 30**

Figure 31

## Figure 32

## Figure 33

## Figure 34

## Figure 35

## Figure 36

## Figure 37

## Figure 38

## Figure 39

## Figure 40

Figure 41

10 µm

## Figure 42

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2005019369 A1

**Non-patent literature cited in the description**

- **BAOFEN YE et al.** *Nanoscale,* 2015, vol. 7 (17), 7565-7568
- **LI ZHOU et al.** *Chemical Communications,* 2014, vol. 50 (71), 10255-10257
- **ANATOLY ZINCHENKO et al.** *Nanomaterials,* 2015, vol. 5 (1), 270-283
- **NIKHITA D. MANSUKHANI et al.** *Macromolecular Rapid Communications,* 2017, vol. 39 (2), 1700587
- **ARGHYA PAUL et al.** *Biomaterials,* 2012, vol. 33 (30), 7655-7664
- **CHENG, E. ; XING, Y. ; CHEN, P. ; YANG, Y. ; SUN, Y. ; ZHOU, D. ; XU, L. ; FAN, Q. ; LIU, D.** A pH-triggered, fast-responding DNA hydrogel. *Angew. Chem., Int. Ed. Engl.,* 2009, vol. 121 (41), 7796-7799
- **XING, Y. ; CHENG, E. ; YANG, Y. ; CHEN, P. ; ZHANG, T. ; SUN, Y. ; YANG, Z. ; LIU, D.** Self-assembled DNA hydrogels with designable thermal and enzymatic responsiveness. *Adv. Mater.,* 2011, vol. 23 (9), 1117-1121
- **NÖLL, T. ; SCHÖNHERR, H. ; WESNER, D. ; SCHOPFERER, M. ; PAULULAT, T. ; NÖLL, G.** Construction of Three-Dimensional DNA Hydrogels from Linear Building Blocks. *Angew. Chem., Int. Ed. Engl.,* 2014, vol. 53 (32), 8328-8332
- **UM, S. H. ; LEE, J. B. ; PARK, N. ; KWON, S. Y. ; UMBACH, C. C. ; LUO, D.** Enzyme-catalysed assembly of DNA hydrogel. *Nat. Mater.,* 2006, vol. 5 (10), 797-801
- **LEE, J. B. ; PENG, S. ; YANG, D. ; ROH, Y. H. ; FUNABASHI, H. ; PARK, N. ; RICE, E. J. ; CHEN, L. ; LONG, R. ; WU, M.** A mechanical metamaterial made from a DNA hydrogel. *Nat. Nanotechnol.,* 2012, vol. 7 (12), 816-820
- **HUANG, Y. ; XU, W. ; LIU, G. ; TIAN, L.** A pure DNA hydrogel with stable catalytic ability produced by one-step rolling circle amplification. *Chem. Commun.,* 2017, vol. 53 (21), 3038-3041
- **HU, R. ; ZHANG, X. ; ZHAO, Z. ; ZHU, G. ; CHEN, T. ; FU, T. ; TAN, W.** DNA nanoflowers for multiplexed cellular imaging and traceable targeted drug delivery. *Angew. Chem., Int. Ed. Engl.,* 2014, vol. 126 (23), 5931-5936

- **ZHU, G. ; HU, R. ; ZHAO, Z. ; CHEN, Z. ; ZHANG, X. ; TAN, W.** Noncanonical self-assembly of multifunctional DNA nanoflowers for biomedical applications. *J. Am. Chem. Soc.,* 2013, vol. 135 (44), 16438-16445
- **LV, Y. ; HU, R. ; ZHU, G. ; ZHANG, X. ; MEI, L. ; LIU, Q. ; QIU, L. ; WU, C. ; TAN, W.** Preparation and biomedical applications of programmable and multifunctional DNA nanoflowers. *Nat. Protoc.,* 2015, vol. 10 (10), 1508-1524
- **SUN, W. ; JIANG, T. ; LU, Y. ; REIFF, M. ; MO, R. ; GU, Z.** Cocoon-like self-degradable DNA nanoclew for anticancer drug delivery. *J. Am. Chem. Soc.,* 2014, vol. 136 (42), 14722-14725
- **KIM, E. ; ZWI-DANTSIS, L. ; REZNIKOV, N. ; HANSEL, C. S. ; AGARWAL, S. ; STEVENS, M. M.** One-Pot Synthesis of Multiple Protein - Encapsulated DNA Flowers and Their Application in Intracellular Protein Delivery. *Adv. Mater.,* 2017, vol. 29, 1701086
- **WANG, J. ; CHAO, J. ; LIU, H. ; SU, S. ; WANG, L. ; HUANG, W. ; WILLNER, I. ; FAN, C.** Clamped Hybridization Chain Reactions for the Self - Assembly of Patterned DNA Hydrogels. *Angew. Chem., Int. Ed. Engl.,* 2017, vol. 56 (8), 2171-2175
- **LEIDNER, A. ; WEIGEL, S. ; BAUER, J. ; REIBER, J. ; ANGELIN, A. ; GRÖSCHE, M. ; SCHARNWEBER, T. ; NIEMEYER CHRISTOF, M.** Biopebbles: DNA-Functionalized Core-Shell Silica Nanospheres for Cellular Uptake and Cell Guidance Studies. *Adv. Funct. Mater.,* 2018
- **BARET, J.-C.** Surfactants in droplet-based microfluidics. *Lab Chip,* 2012, vol. 12 (3), 422-433
- **BAUER, W.-A. C. ; FISCHLECHNER, M. ; ABELL, C. ; HUCK, W. T.** Hydrophilic PDMS microchannels for high-throughput formation of oil-in-water microdroplets and water-in-oil-in-water double emulsions. *Lab Chip,* 2010, vol. 10 (14), 1814-1819
- **J. N. ZADEH ; C. D. STEENBERG ; J. S. BOIS ; B. R. WOLFE ; M. B. PIERCE ; A. R. KHAN ; R. M. DIRKS ; N. A. PIERCE.** *J. Comput. Chem.,* 2011, vol. 32, 170

- **D. SHANGGUAN ; Y. LI ; Z. TANG ; Z. C. CAO ; H. W. CHEN ; P. MALLIKARATCHY ; K. SEFAH ; C. J. YANG ; W. TAN.** *Proc. Natl. Acad. Sci. U. S. A.,* 2006, vol. 103, 11838
- **G. ZHU ; R. HU ; Z. ZHAO ; Z. CHEN ; X. ZHANG ; W. TAN.** *J. Am. Chem. Soc.,* 2013, vol. 135, 16438
- **OELSCHLAEGER, C. ; BOSSLER, F. ; WILLEN-BACHER, N.** Synthesis, structural and micromechanical properties of 3D hyaluronic acid-based cryogel scaffolds. *Biomacromolecules,* 2016, vol. 17, 580-589
- **LEIDNER, A. et al.** Biopebbles: DNA - Functionalized Core-Shell Silica Nanospheres for Cellular Uptake and Cell Guidance Studies. *Adv. Funct. Mater.,* 2018, vol. 0
- **LILIENTHAL, S. ; SHPILT, Z. ; WANG, F. ; OR-BACH, R. ; WILLNER, I.** Programmed DNAzyme-Triggered Dissolution of DNA-Based Hydrogels: Means for Controlled Release of Biocatalysts and for the Activation of Enzyme Cascades. *ACS Appl. Mater. Interfaces,* 2015, vol. 7, 8923-8931
- **SHANGGUAN, D. et al.** Aptamers evolved from live cells as effective molecular probes for cancer study. *Proc. Natl. Acad. Sci. U. S. A.,* 2006, vol. 103, 11838-11843
- **ZHU, G. et al.** Self-Assembled Aptamer - Based Drug Carriers for Bispecific Cytotoxicity to Cancer Cells. *Chem. - Asian J.,* 2012, vol. 7, 1630-1636
- **ANGELIN, A. et al.** Multiscale Origami Structures as Interface for Cells. *Angew Chem Int Ed Engl,* 2015, vol. 54, 15813-15817